# EUROPEAN PATENT SPECIFICATION

(11) **EP 0 445 868 B1**
(45) Date of publication and mention of the grant of the patent: **29.07.1998**
(21) Application number: 91200423.1
(22) Date of filing: 27.02.1991
(51) Int. Cl.: C12N 15/52, C12N 1/15, C12N 1/21, C12P 35/00, C12P 37/00, C07K 5/08

(54) **A method for influencing beta-lactam antibiotic production and for isolation of large quantities of ACV synthetase**
Verfahren zur Beeinflussung der Produktion von beta-lactam Antibiotika und zur Isolierung grosser Mengen von ACV-Synthetase
Méthode pour influencer la production des antibiotiques bêta-lactames et pour isoler des grandes quantités d'ACV-synthétase

(30) Priority: 28.02.1990 EP 90200475; 28.02.1990 EP 90200488; 02.07.1990 EP 90201768; 03.10.1990 EP 90202628
(43) Date of publication of application: 11.09.1991
(73) Proprietor: GIST-BROCADES N.V., NL-2600 MA Delft (NL)
(72) Inventor: Veenstra, Annemarie Eveline, NL-2151 XH Nieuw Vennep (NL); Martin, Juan Francisco, E-24004 Leon (ES); Garcia, Bruno Diez, San Sebastian (Guipuzcoa) (ES); Guttierez, Santiago, Leon (ES); Barredo, Jose Luis, Burgos (ES); Montenegro Prieto, Eduardo, E-24002 Leon (ES); Von Doehren, Hans, W-1000 Berlin 12 (DE); Palissa, Harriet, W-1000 Berlin 61 (DE); Van Liempt, Henk, W-1000 Berlin 61 (DE)
(74) Representative: Matulewicz, Emil Rudolf Antonius, Dr.

(56) References cited:
- EP-A- 0 280 051
- EP-A- 0 320 272
- EP-A- 0 357 119
- THE JOURNAL OF BIOLOGICAL CHEMISTRY, vol. 265, no. 27, September 1990, pages 16358-16365; B. DIEZ et al
- THE JOURNAL OF BIOLOGICAL CHEMISTRY, vol. 264, no. 7, March 1989, pages 3680-3684; H. VAN LIEMPT et al.: "Gamma-(L-alpha-aminoadipyl)-L-cysteinyl-D-valine synthetase from Aspergillus nidulans"

## Description

### INTRODUCTION

### Technical Field

This invention relates to methods and compositions to enhance the in vivo and in vitro production of fermentable or known and new secondary metabolites, particularly β-lactams and their biosynthetic intermediates using recombinant DNA techniques.

### Background

β-Lactam antibiotics are the largest family of secondary metabolites produced in nature by microorganisms. The most important classes of the β-lactam antibiotics both clinically and economically are the penicillins (penam) and cephalosporins (cephem). Their biosynthesis occurs via a complex pathway of enzymatic steps; the unravelling of this pathway has been the subject of many studies during the last few decades. The first two steps are the key steps both in the biosynthetic pathways of the penam and cephem classes of β-lactam antibiotics. After these two steps the biosynthetic pathways to the penicillins and cephalosporins diverge.

The first step in the biosynthesis of the penicillin, cephalosporin and cephamycin antibiotics is the condensation of the L-isomers of three amino acids, L-α-amino adipic acid (A), L-cysteine (C) and L-valine (V) into a tripeptide, δ-(L-α-aminoadipyl)-L-cysteinyl-D-valine or ACV. This step is catalyzed by δ-(L-α-aminoadipyl)-L-cysteinyl-D-valine synthetase or ACVS. In the second step, the tripeptide ACV is oxidatively cyclised by the action of the Isopenicillin N Synthase (hereinafter referred to as IPNS) or cyclase. The product of this reaction is Isopenicillin N; this compound contains the typical β-lactam and thiazolidine ring structures and possesses antibacterial activity. From Isopenicillin N the penicillins G or V are formed by exchange of the hydrophilic A side chain by a hydrophobic side chain. The side chains commonly used in industrial processes are either phenylacetic acid (PA), yielding penicillin G, or phenoxyacetic acid (POA), yielding penicillin V; this exchange reaction is catalyzed by the enzyme acyltransferase (hereinafter referred to as AT).

From Isopenicillin N the route to cephalosporin and the cephamycins proceeds via racemisation of the A side chain, forming penicillin N. This reaction is catalyzed by an enzyme named epimerase or racemase. The five-membered ring in penicillin N is expanded into a six-membered ring by the action of the enzyme deacetoxycephalosporin C synthase or expandase. The fungal enzyme has been shown also to catalyze the next reaction in the pathway, the hydroxylation of the methyl group at the 3'-position of the six-membered ring, forming deacetylcephalosporin C. In Streptomycetes this latter enzyme activity is encoded by a separate gene. From deacetylcephalosporin C, Cephalosporin C is formed by acetylation of the 3'-position. The cephamycins are formed from the same compound by several enzymatic steps.

In vivo synthesis of β-lactams and their precursors can be increased by increasing the activity of enzymes involved in β-lactam biosynthetic pathways. This can be achieved by either increasing the amount of enzyme present or by improving the specific activity of the enzyme. However, obtaining enzymes having the desired activity has typically been limited by the availability of spontaneous mutations in enzymes which are active at points in the pathway.
Currently, the cost effectiveness of in vitro synthesis of β-lactam precursors in cell-free extracts is poor in comparison to traditional fermentation processes. These in vitro processes are hampered by the limited amounts of enzyme present within the cell, and hence within the cell-free extract, and by the presence of inhibitory factors such as proteases and enzyme inhibitors. In addition, the use of fermentable or known β-lactam antibiotics (i.e. can be produced by fermentation of (β-lactam producing) non-recDNA microorganisms) is complicated by a) the development of resistance or tolerance to fermentable or known β-lactam antibiotics by bacterial species, and b) the limitations in therapeutic use, such as an allergy to penicillin. There is, therefore, substantial interest in the development of systems which allow for the efficient and cost-effective production of new β-lactam antibiotics (i.e. cannot be produced by fermentation of (β-lactam producing) non- recDNA microorganisms) and their precursors, both in vivo and in vitro.

### Relevant Literature

Van Liempt et al., (J. Biol. Chem. (1989), 264:3680-3684) have shown in Aspergillus nidulans that the ACV condensation is carried out by a large multifunctional enzyme, ACV synthetase. Similar results have been obtained for the Acremonium chrysogenum ACV synthetase by Banko et al., (J. Amer. Chem. Soc. (1987), 109:2858). The gene encoding ACV synthetase (pcbAB) has been located on the genome of A.nidulans (MacCabe et al., EMBO J. (1990), 8:279-287) and Penicillium chrysogenum (EP-A-320272; EP-A-357119; D. Smith et al., Bio/Technology (1990), 8:39-41). pcbAB is located just upstream of the gene encoding IPNS.

In vitro synthesis of the tripeptide ACV in cell-free extracts (Adlington et al., Biochem. J. (1983), 213:573; G. Banko et al., J. Am. Chem. Soc. (1987), 109:2858; Jensen and Westlake, Developments in Industrial Microbiology (1989), 30:113-119; EP-A-280051) has been used to study parameters of the ACV synthetase reaction and also to study the feasibility of commercial application of in vitro synthesis of β-lactam antibiotics; such as compared to traditional fermentation processes these processes are not commercially attractive (Jensen, supra). Several inhibitory compounds for the ACV synthetase reaction have been disclosed (Adlington and Banko, supra). It has also been established that the ACV synthetase has a rather narrow substrate specificity. Only a few amino acids can be substituted for the native α-amino adipic acid, cysteine and valine.

Amplification of antibiotic biosynthetic genes by increased copy number resulting in an increase in the production of an antibiotic has been described by Skatrud et al., Bio/technology (1989), 7:477-485 and EP-A-357119, supra). Increased cephalosporin production, using the cefEF gene (Skatrud et al., supra) or penicillin production, using the pcbC-penDE gene cluster (EP-A-357119) has been reported. Expression of β-lactam biosynthetic genes, other than the ACV synthetase gene, has been described in Streptomyces lividans (Chen et al., Bio/technology (1988), 6:1222-1224). Publications relating to enzymes included in β-lactam biosynthesis and the cloning of genes encoding these enzymes are as follows. The IPNS has been purified and the gene encoding this enzyme, pcbC, has been cloned from various organisms (reviewed in Miller and Ingolia, Molecular Microbiology 1989, 3:689-695; Martin and Liras, Advances in Biochemical Engineering/Biotechnology (1989), 39:153-187).

The enzyme acyltransferase has been purified (Alvarez et al., Antimicrob. Agents Chemother. 1987, 31:1675-1682; EP-A-336446) and the gene encoding this enzyme (penDE) has been cloned (EP-A-336446; EP-A-354624; Veenstra et al., In: C.L. Hershberger, S.W. Queener and G. Hegeman, eds: Genetics and Molecular Biology of Industrial Microorganisms, (1989), pp 262-269; Barredo et al., Gene 1989, 83:291-300). The pcbC and penDE genes are clustered in the genome of Penicillium chrysogenum (B. Diez et al., Mol. Gen. Genet. (1989), 218:572-576; Veenstra, supra).

The epimerase has been purified from Streptomyces clavuligerus (Usui and C-A Yu, Biochem. Biophys. Acta (1989), 999:78-85); the presence of this gene on a large DNA fragment was suggested in EP-A-233715. The enzyme expandase has been isolated both from Acremonium chrysogenum (Kupta et al., FEMS Microbiol. Letters 1983, 16:1-6; Dotzlaf and Yeh, J. Bacteriol. (1986), 16:1611-1618) and from Streptomyces clavuligerus (Rollins et al., Can. J. Microbiol. (1988), 34:1196-1202) and Streptomyces lactamdurans (Cortes et al., J. Gen. Microbiol. (1987), 133:3165-3174). The expandase genes have been cloned from both A.chrysogenum (cefEF, Samson et al., Bio/technology (1987), 5:1207-1214) and from Streptomyces clavuligerus (cefE, Kovacevic et al., J. Bacteriol. (1989), 171:754-760).

### SUMMARY OF THE INVENTION

Methods, and compositions for use therein, are provided for enhanced expression of β-lactam antibiotic biosynthetic genes, leading to increased production of β-lactam antibiotics and precursors thereto. The methods include the steps of stably transforming a host cell with an expression cassette containing at least one DNA sequence encoding an ACV synthetase (ACVS) or a biologically active mutant of ACV synthetase (ACVS') and isolating transformants which produce an enhanced amount of ACV synthetase. The expression cassette includes transcriptional and translational initiation regulatory regions, hereinafter defined as promoter, and transcriptional and translational termination regulatory regions, hereinafter defined as terminator, appropriate for the host cell. The promoter may also be heterologous to the open reading frame. The host cell may be a eukaryote or a prokaryote. Also included is the pcbAB gene from P.chrysogenum and A.chrysogenum, constructs and vectors comprising the pcbAB gene or a mutant thereof, and transformed cells comprising said pcbAB gene. The subject invention finds use particularly in improved production both in vivo and in vitro of β-lactam antibiotics and their precursors, particularly antibiotics of the penam and cephem classes.

### BRIEF DESCRIPTION OF THE DRAWINGS

Figure 1 shows a schematic representation of the distribution of β-lactam producing microorganisms.

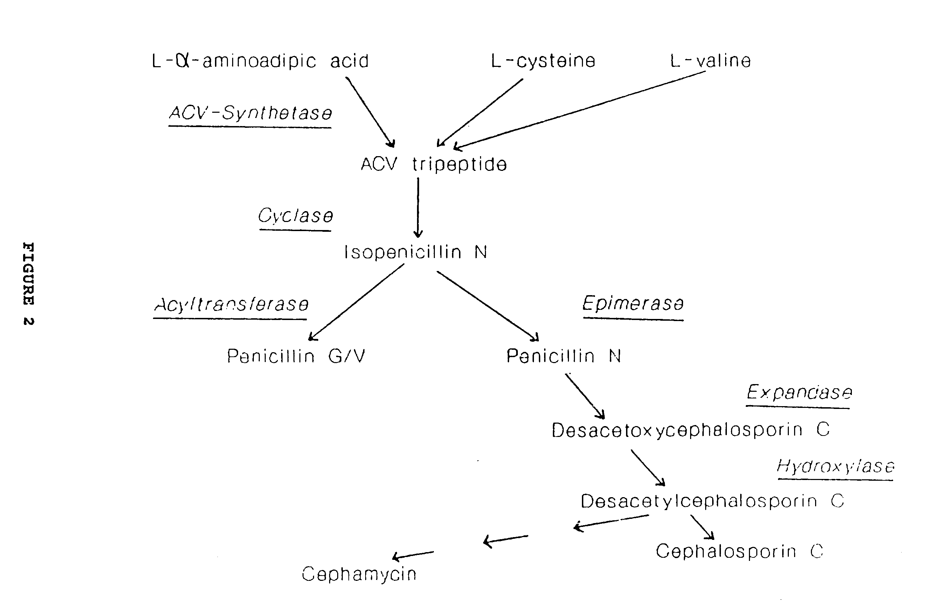

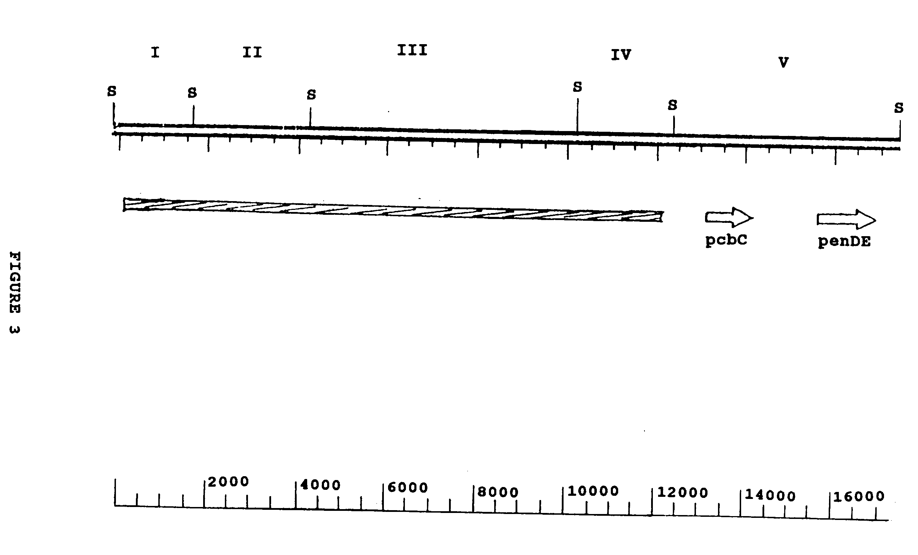

Figure 2 shows a schematic representation of the biosynthetic pathways of the penicillins, cephalosporins and cephamycins.

Figure 3 shows a restriction site and functional map of the chromosomal region containing the ACV synthetase gene in P.chrysogenum. Probes that have been used for Northern hybridizations are indicated. The region containing the pcbAB gene is shaded. S=SalI.

Figure 4 shows a detailed physical map of the region encoding the ACV synthetase gene in P.chrysogenum, including the sequencing strategy.

Figure 5 shows a schematic representation of the cluster of penicillin biosynthetic genes present in the genome of P.chrysogenum.

Figure 6 shows a restriction site and functional map of construct pPCV01.

Figure 7 shows a restriction site and functional map of construct pPCV02.

Figure 8 shows a restriction site and functional map of construct pPCV03.

Figure 9 shows a restriction site and functional map of the E.coli vector pMAtrp.

Figure 10 shows a restriction site and functional map of pMA-ACV synthetase.

Figure 11 shows a restriction site and functional map of pSLACV-01.

Figure 12A shows a restriction site and functional map of pSLACV-03A.

Figure 12B shows a restriction site and functional map of pSLACV-03B.

Figure 13 shows a scheme that shows the sequence homology between domains found within the ACVS protein and Gramicidin and Tyrocidin Synthetases.

Figure 14 shows a scheme that shows the sequence homology between the ACVS protein and the thioesterase domain of rat fatty acid synthetase.

Figure 15 shows a scheme listing the domains and sub-domains within the ACVS protein.

Figure 16 is a restriction site and functional map of the chromosomal region encoding the ACVS gene in A.chrysogenum. Arrows indicate the positions of the IPNS-(pcbC) and ACVS-(pcbAB) genes. The position of phages which have been isolated from a gene library and which contain part of the region are indicated.

Figure 17 is a restriction site and functional map of the chromosomal region encoding the ACVS gene in A.chrysogenum. Probes that have been used in Northern Blot Hybridizations, and the results obtained, are indicated.

Figure 18 shows the similarity between the deduced amino acid sequences from the ACVS genes from P.chrysogenum and A.chrysogenum.

### BRIEF DESCRIPTION OF THE SEQUENCE LISTINGS

Sequence listing 1 shows a nucleotide sequence and deduced amino acid sequence of the P.chrysogenum ACV synthetase gene.

Sequence listing 2 shows the nucleotide sequence and deduced amino acid sequence of the A.chrysogenum ACV synthetase gene.

### DESCRIPTION OF THE SPECIFIC EMBODIMENTS

In accordance with the subject invention, methods and compositions are provided which allow for increased, cost-effective production of β-lactam antibiotics and their precursors both in vivo and in vitro. The method includes the steps of transforming a host cell using an expression cassette which includes in the 5'-3' direction of transcription, a promoter, an open reading frame encoding an ACVS or ACVS', optionally having a signal sequence for secretion recognized by the host cell; and a terminator. The promoter and terminator are functional in the host cell and provide for efficient expression of ACVS or ACVS' without undesirable effects on the viability and proliferation of the host cell. Transcription and translation of the ACV synthetase DNA sequence will augment levels of ACV synthetase already present in the cell. Transcription and translation of the ACVS' DNA sequence will provide for expression of ACVS' in the host cell, which may have enhanced properties as compared to the native enzyme.

Optionally, the expression cassette may include a promoter which is not subject to repression by substances present in the growth medium. The expression systems may be used to prepare fermentable β-lactam antibiotics directly or they may be used to prepare cell-free extracts containing large quantities of ACVS or ACVS' for in vitro preparation of said antibiotics. Alternatively, the expression hosts may be used as a source of large quantities of enzyme which can be purified and used in in vitro systems.

Enhanced production of ACV synthetase is achieved by the introduction of extra copies of the gene encoding ACV synthetase (pcbAB gene) into a host cell by transformation: protoplasts are mixed with DNA constructs that contain at least one copy of the gene to be amplified linked to a selectable marker. By choosing the appropriate conditions, some protoplasts will take up the DNA construct which is thereafter stably maintained because the construct has become integrated into the host cell genome. Transformed cells can be selected from the background of so-called "non-transformed" cells by screening for expression of the selectable marker. Amplification of the pcbAB gene, which may be used to produce cephalosporin, penicillin, and cephamycin thus can be expected to result in an increase in intracellular enzyme activity, which in turn will be accompanied by an increase in production of the desired antibiotics. Since ACV synthetase forms part of the biosynthetic pathway of both the penam and the cephem classes of β-lactam antibiotics, increased production of this enzyme has several applications. Production of ACVS' is achieved by the introduction of at least one copy of a mutant gene encoding ACV synthetase (pcbAB' gene) into a host cell by transformation, as described hereinabove.

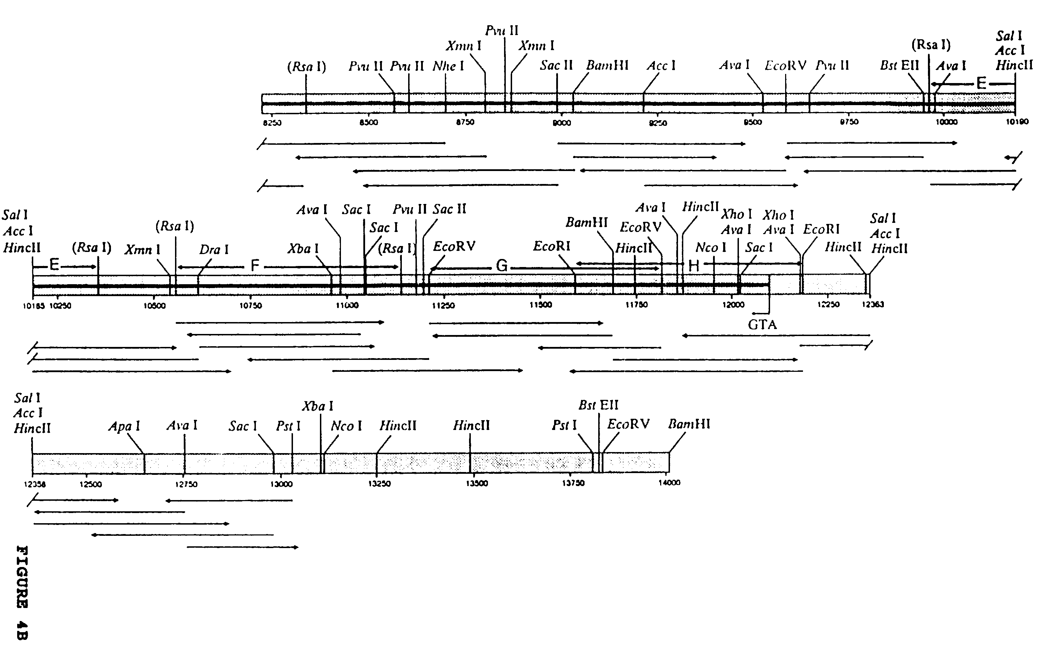

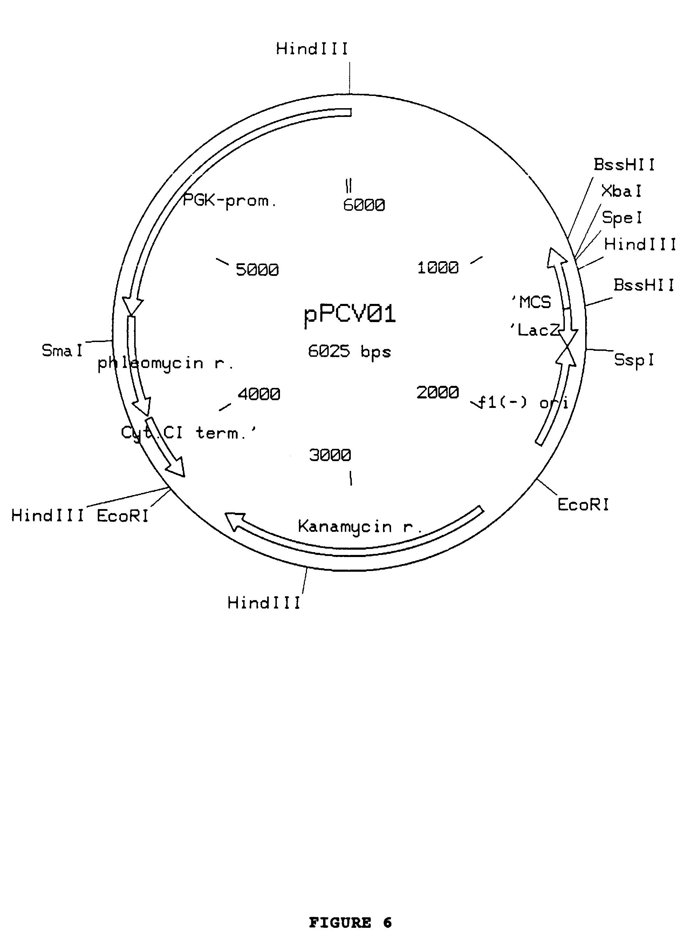

The existence of one enzyme indicates the presence of one gene encoding this enzyme, pcbAB. The localization of this gene in P.chrysogenum, just upstream of the gene encoding IPNS, is described in EP-A-357119 (non-prepublished). Similar results about the localization of the gene in P.chrysogenum are also described in EP-A-320272, and in Smith et al., cited above. These data still allow for the ACVS activity to be encoded in more than one gene, however. Based on the results obtained for A.nidulans (250 kDa enzyme: Van Liempt et al.) and P.chrysogenum (gene present on 8 x 10³ nucleotides DNA fragment: EP-A-320272) a gene with a size of at most 8 x 10³ nucleotides would be predicted. Surprisingly, the data presented in the present patent application indicate that the coding region of the P.chrysogenum pcbAB gene is 11,337 nucleotides long and encodes a protein of 413 kDa. In Figure 6B of EP-A-320272 a restriction map of the DNA comprising the asserted intact ACVS gene has been given. From the expression of the P. chrysogenum DNA fragment in the vector pCX3.2 it is concluded that this fragment contains one or more genes that are involved in the ACV synthetase production. Comparison of the restriction map (Figure 6B of EP-A-320272) with the one in this patent application (Figure 4) clearly demonstrates that this fragment of EP-A-320272 encodes an incomplete ACV synthetase lacking the last about 900 nucleotides (about 300 C-terminal amino acids). This is not recognized by the applicants of EP-A-320272. Besides that, a significant amount of ACV synthetase can now be achieved by using the manipulated pcbAB genes of the present invention.

Only now it can be envisaged that all catalytic activities required for tripeptide formation (i.e. activation of three amino acids, racemisation of valine, formation of dipeptide AC and tripeptide ACV, release of ACV) can indeed be present in one single polypeptide chain. ACVS hence is a multifunctional enzyme. Multifunctional enzymes are defined herein to be enzymes that consist of one single polypeptide chain and that carry within their structure the ability to perform more than one catalytic reaction. In contrast, in the case of the biosynthesis of Gramicidin S, a peptide antibiotic consisting of two identical units of five amino acids, at least two, and probably three polypeptide chains are involved (e.g. Von Döhren, 1982, In: Peptide Antibiotics, W. de Gruyter & Co., Berlin. pp 169-182; Krätzschmar et al., Journ. of Bacteriol. (1989) 171, 5422-5429).

In vitro synthesis of the ACV tripeptide has been described previously (Adlington and Banko, (supra). However, the only available systems up to now have been preparations comprising cell-free extracts of various organisms that possess ACV synthetase activity, followed by an in vitro reaction. The availability of an efficient expression system and the accompanying constructs, designed for high expression of the gene in suitable hosts is highly advantageous. It allows for the design of in vitro systems which do not have the disadvantages of a limited amount of enzyme present within the cell and hence within the cell-free extract, or the presence of an excess of negative factors such as proteases or inhibitors or other similar effects. Increased productivity thus is obtained as a result of the enzyme being present in large quantities in the cell-free extracts prepared from the transformed host organisms as compared to the organisms used by for example Adlington and Banko, supra. Alternatively, large quantities of purified enzyme can be obtained and subsequently incubated in systems devoid of negative components.

For preparation of ACV and secondary metabolites, or for preparation of ACV synthetase by recombinant methods, genes encoding ACV synthetase (the pcbAB gene) may be obtained from a variety of sources including Penicillium chrysogenum, Acremonium chrysogenum, Aspergillus nidulans, Flavobacterium or Streptomycetes. The structural genes may be isolated by various techniques. These include isolating mRNA from a host organism which codes for the polypeptide of interest, the mRNA reverse transcribed, the resulting single stranded (ss) DNA used as a template to prepare double stranded (ds) DNA and the dsDNA gene isolated.

Another technique is to isolate the chromosomal DNA from the source organism of interest and, using a probe, appropriately degenerate, comprising a region of the most conserved sequences in the gene of interest, identify sequences encoding ACV synthetase in the genome. The probe can be considerably shorter than the entire sequence, but should be at least 10, preferably at least 14, more preferably at least 20 nucleotides in length. Longer nucleotides are also useful, up to about 100 nucleotides of the gene of interest. Both DNA and RNA probes can be used.

In use, the probe is typically labeled in a detectable manner (for example with ³²P or biotinylated nucleotides) and are incubated with ss DNA or RNA from the organism in which the gene is being sought after separation and/or immobilization of the ss or ds DNA, typically using nitrocellulose paper or nylon membranes. Hybridization is detected by means of autoradiography. Hybridization techniques suitable for use with oligonucleotides are well known to those skilled in the art. Although probes are normally used with a detectable label that allows for easy identification, unlabeled oligonucleotides are also useful, both as precursors of labeled probes and for use in methods that provide for direct detection of DNA or DNA/RNA. Accordingly, the term "oligonucleotide" refers to both labeled and unlabeled forms.

The gene encoding the ACVS can be isolated from various organisms. For the gene encoding the IPNS, it has been shown (See for example, Ingolia and Queener, Medicinal Research Reviews (1989) 9:245-264) that genes isolated from different organisms show a high degree of homology, ranging from about 70% on the DNA level if two fungal or two Streptomyces genes are compared to 60% or more if a fungal and a Streptomycete gene are compared. Homologies on the protein level are 75% and 54%, respectively. Despite the differences on the DNA and protein level, all IPNS proteins catalyze the same reaction in a similar fashion. Therefore, ACVS sequences and enzymes can be identified that share a minimal homology of about 60% on the DNA level or 50% on the protein level.

Sequences that are at least substantially identical to the sequence given in sequence listing 1 or sequence listing 2 are of particular interest. By "substantially identical" is intended sequences which can include conservative mutations, where the sequence encodes the same amino acid sequence, but may have as many as 30% different nucleic acid bases, more usually not more than 10% different bases, or mutations which are non-conservative, where fewer than about 10%, more usually fewer than about 5%, and preferably not more than 1% of the encoded amino acids are substituted or deleted, and there are fewer than 5% of inserted amino acids, where the percentage is based on the number of naturally occurring amino acids in the native enzyme. The degree of change which is acceptable can be determined by assessing the enzymatic activity of the expression product of the isolated gene, where the expression product should retain the ability to catalyze the formation of ACV from its constituting amino acids.

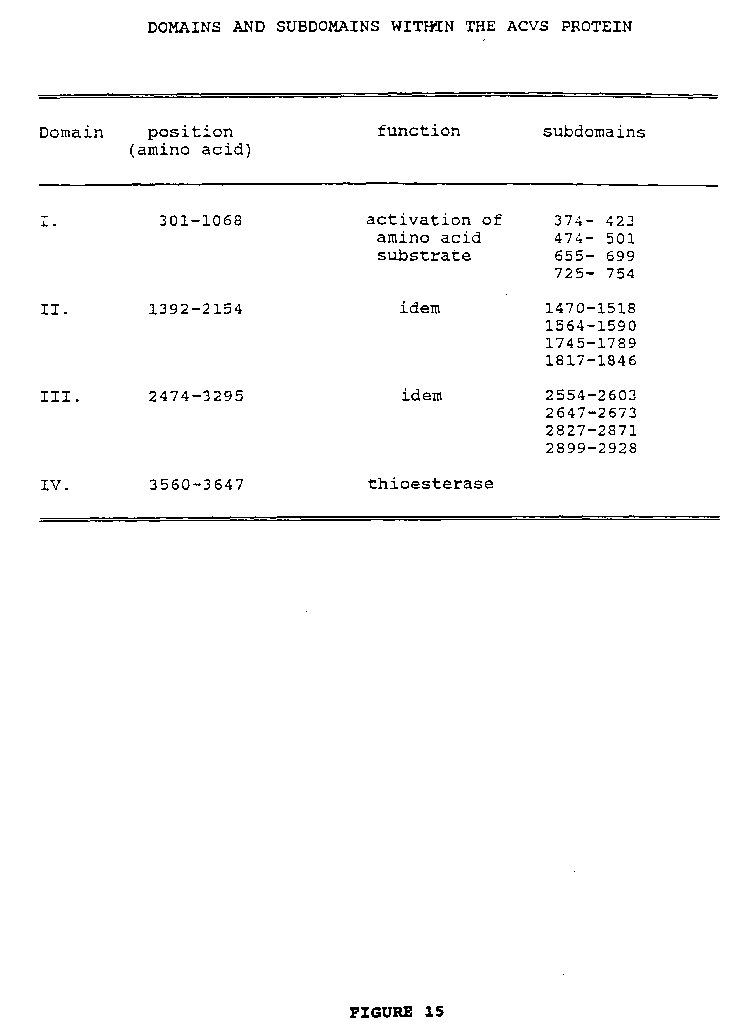

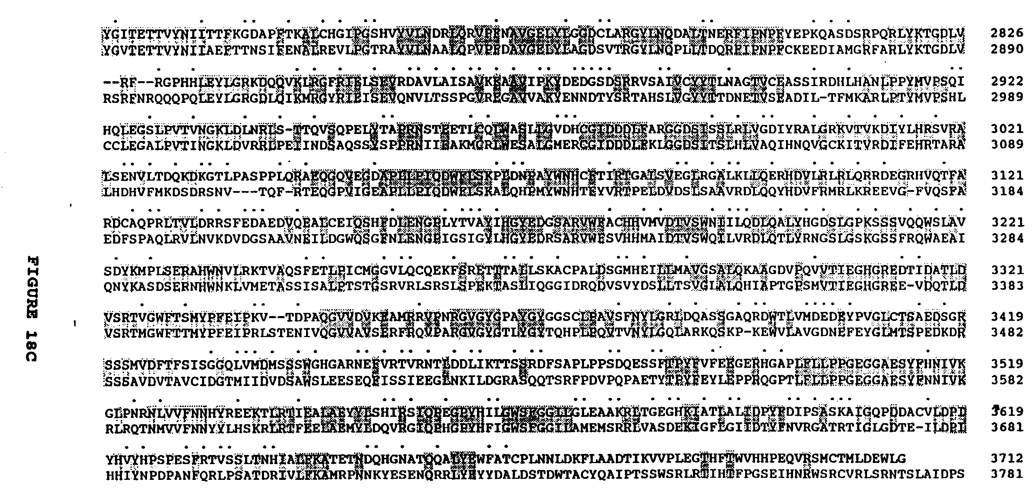

ACV synthetase genes can be isolated by using, for example, the sequence as given in sequence listing 1, or parts thereof, as a probe in heterologous hybridization experiments. These probes can be restriction fragments derived from the ACVS encoding DNA isolated from P.chrysogenum; such restriction fragments can easily be selected and isolated using the restriction map as given in Figure 4. Alternatively, synthetic oligonucleotide probes can be made based on the data given in sequence listing 1. In still another variation, oligonucleotides can be designed, based on the data in sequence listing 1, and used in a PCR reaction to generate a larger probe fragment. In this way, for example, the genes of Acremonium chrysogenum and Aspergillus nidulans can be readily isolated; also the genes from Streptomycetes can be isolated using less stringent hybridization conditions. The genes that are isolated in this fashion are also of interest. This procedure is exemplified by the isolation of the pcbAB gene from A.chrysogenum. The data obtained for this gene in their turn can be used for the gene-isolation procedure as described above. For example, using the data of the present invention, regions with a high homology can be identified, such as the domains of Figures 15 and 18, and selected for use as a highly specific probe in the isolation of pcbAB genes from more distantly related organisms.

Alternatively, the DNA sequences encoding the ACV synthetase can be synthesized using conventional techniques such as PCR (Polymerase Chain Reaction) or by synthesis of overlapping single strands which may be ligated together to define the desired coding sequences. The termini can be designed to provide restriction sites or one or both termini may be blunt-ended for ligation to complementary ends of an expression vector. For expression of the sequence an initial methionine is provided. Expression vectors are generally available and are amply described in the literature.

For preparation of ACVS' and modified secondary metabolites, or for preparation of ACVS' by recombinant methods, genes encoding ACVS' may be obtained from spontaneous pcbAB' mutants from a variety of sources including Penicillium chrysogenum, Acremonium chrysogenum, Aspergillus nidulans, or from Flavobacterium or the Streptomycetes. Also, sequences that encode biologically active mutant ACV synthetase can be derived from sequences that are at least substantially identical to the sequence of a fermentable or known ACV synthetase or from spontaneous mutant pcbAB alleles. Mutant sequences can be "derived" by a variety of genetic and recombinant DNA techniques, such as in vitro mutagenesis and homologous recombination. The gene of interest encodes biologically active mutant ACV synthetase or a biologically active portion of ACV synthetase or a mutant thereof. A spontaneous mutant gene or a wild type gene (from which a mutant structural gene subsequently can be derived) can be isolated by various techniques using host organisms which are ACV synthetase mutants or wild type. These techniques have been described hereinabove.

Once the ACV synthetase DNA is obtained, mutations can be introduced by a number of in vitro mutagenesis techniques, either random or site-directed. Precise changes to the amino acid sequence are obtained by site-directed mutagenesis techniques which use synthetic oligonucleotides complementary to the region to be modified, except for the desired nucleotide(s) change. Regions are precisely deleted by "loop-out" mutagenesis techniques using synthetic oligonucleotides. Precise insertions are obtained using synthetic oligonucleotides to generate appropriate restriction sites. A series of mutations localized to a region of the ACV synthetase-encoding DNA sequence on a plasmid are generated by in vitro mutagenesis techniques and subsequently identified by cloning and sequencing the isolated mutagenized plasmids.

Once specific mutations have been generated and isolated to individual plasmids, "cassette" mutagenesis is applied to generate a series of new mutants by combining mutations into the same plasmid using appropriate restriction sites. Alternatively, mutagenesis using two or more oligonucleotides directed to different regions of the gene yields mutants with the desired multiple mutations.

Mutant proteins are also generated by the insertion, addition, or substitution of coding sequences from other proteins to ACV synthetase coding sequences. The source proteins may be ACV synthetases from other strains or species. Such amino acid sequences introduced into ACV synthetase will impart desirable properties or characteristics which originally belonged to the source protein.

Desirable properties are useful properties for activities and functions which include and are not limited to protein stability, secretion, isolation, purification, increased enzymatic activity, resistance to inhibitors, proteases and denaturants, and solubility.

Although any region of the protein may be mutated, regions of the ACV synthetase protein which serve as candidates for mutagenesis are defined in order to minimize extensive screening of randomly generated mutants. The nucleotide and deduced amino acid sequences of ACV synthetase provided are a powerful tool with which to delineate such regions. Sequence homology comparisons, at both the DNA and amino acid level, identify protein regions with known function or structure.

Hydrophobicity profiles or related biophysical profiles, based on the amino acid sequence, with subsequent comparisons to profiles of other proteins may be used to identify additional protein regions of known structure and function. The finding of sequence or profile homologies indicates that the protein regions share a similar function, activity or enzymatic mechanism. Hydrophobicity or secondary structural profiles can indicate domain "linking" regions, "hinge" regions or "loops" which are candidates for restriction site insertions to generate domain "cassettes".

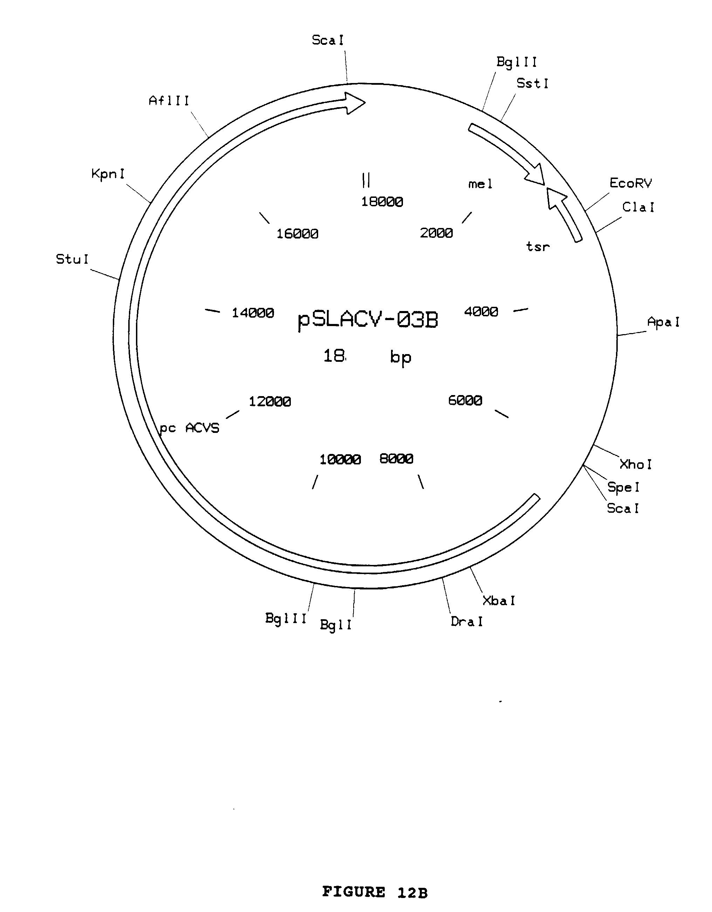

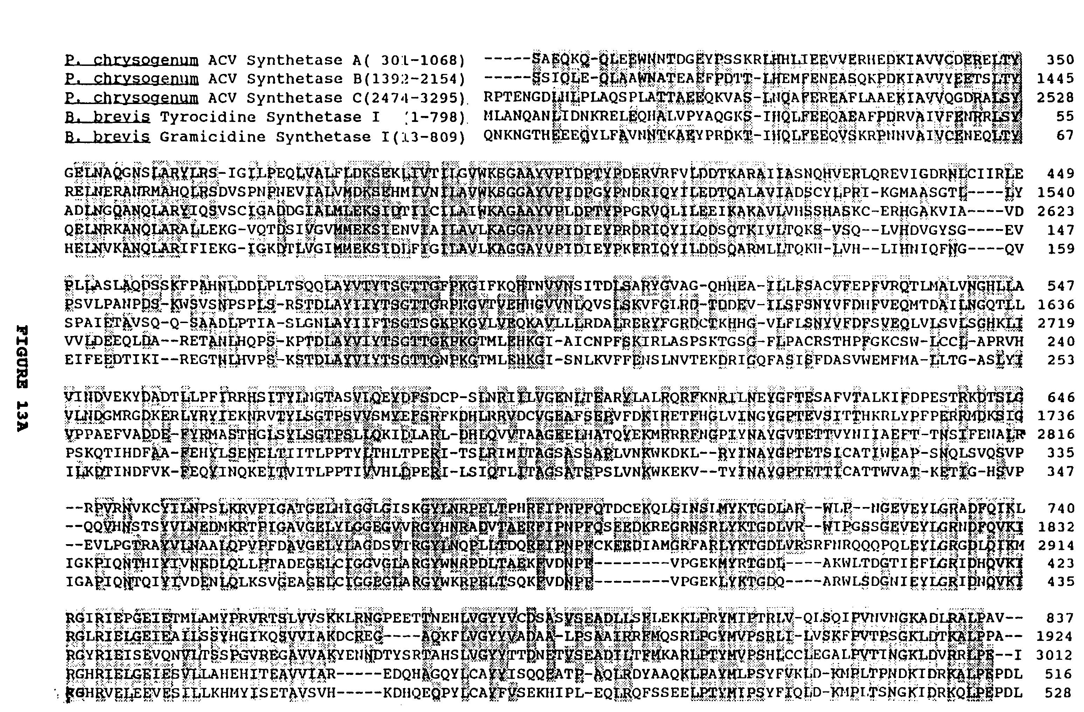

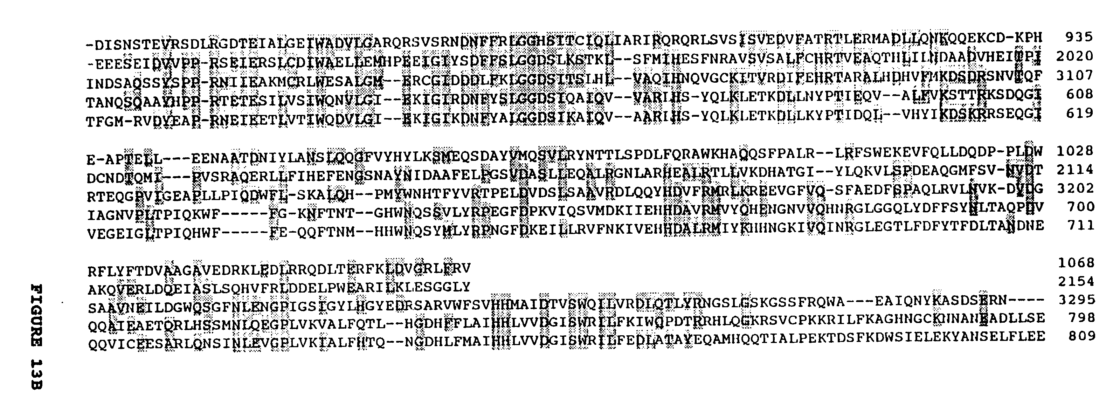

Regions for mutagenesis or replacement are also defined by correlation of a genetic map of ACV synthetase mutant alleles with a physical map, either at the restriction site or sequence level. Preferred regions for mutagenesis, either site-directed or via substitution include the functional domains and sub-domains of ACV synthetase as provided in Figures 13, 14, and 15.

Mutant proteins also include functional proteins formed by various combinations or quarternary assemblies of discrete polypeptides. The polypeptides are expressed individually, either from the same or different plasmids, in a host cell or isolated and recombined in vitro. The polypeptides contain one or more enzymatic activities. Preferably the polypeptides are from the domains of ACV synthetase as defined in Figures 13, 14, and 15. Polypeptides from regions of other proteins, as defined in the preceding sections, may also be combined with one or more ACV synthetase polypeptides in the same discrete fashion.

Once the desired DNA sequence has been obtained, it may be manipulated in a variety of ways to provide for expression. It is highly desirable that modifications of the nucleotide sequence, other than the modifications which result in the desired mutation(s), retain the three dimensional structure of the expression product, particularly that portion of the structure which may be responsible for the enzymatic activity of the resulting enzyme. Convenient restriction sites may be designed into the DNA sequence of interest; when possible the restriction site(s) leaves the amino acid sequence of the expression product unaltered. However, in some cases, incorporation of new restriction sites may yield an altered amino acid sequence.

Where the gene encoding ACVS or ACVS' is to be expressed in a host which recognizes the wild type promoter and terminator of the gene of interest, the entire gene with its wild type 5' and 3'-regulatory regions may be introduced into an appropriate expression vector. Where said gene is to be expressed in a host cell which does not recognize the naturally occurring wild type promoter and terminator, further manipulation may be required. Conveniently, a variety of 3'-transcriptional regulatory regions are known and may be inserted downstream from the stop codons. The non-coding 5'-region upstream from a structural gene may be removed by endonuclease restriction, Bal31 digestion or the like. Alternatively, where a convenient restriction site is present near the 5'-terminus of the structural gene, the structural gene may be restricted and an adaptor employed for linking the structural gene to the promoter region, where the adapter provides for the lost nucleotides of the structural gene.

The biosynthesis of β-lactam antibiotics in general, and of penicillin in particular, is subject to glucose repression (Martin and Liras, TIBS (1985), 3:39-44). This repression by glucose has been unequivocally established for the formation of the tripeptide by the ACV synthetase and for the activity of IPNS (Revilla et al., J. Bacteriol. (1986), 168:947-952). It is not known at which stage of expression repression by glucose is exerted; this can, for example, be at the transcriptional or at the translational level. If the former applies, constitutive expression of the pcbAB gene will result in an increase in enzyme activity followed by an increase in the production of ACV, and subsequently of the β-lactam antibiotic derived from it.

Increased expression of the gene in β-lactam producing bacterial or fungal hosts therefore can be obtained by changing the regulation of gene expression. Thus, the transcriptional regulatory region is preferably one which is not subject to repression by, for example, presence or absence of nutrients such as glucose, or expression products in the growth medium. The transcriptional regulatory region may additionally include regulatory sequences which terminate transcription and which provide sequences or structures which inhibit degradation of the mRNA.

Exemplary of changing the regulation of expression is modification of the pcbAB gene. The native sequence generally is replaced by a region which is functional in either the native or heterologous host and wherein expression is either inducible or constitutive. For example, the regulatory sequences can be changed by replacing the pcbAB promoter, which is strongly repressed by glucose, with a promoter which is insensitive to glucose or even is stimulated by it. In the latter situation, antibiotic will be produced during the early stages of the fermentation, when biomass is formed in high-glucose conditions. This modification may further increase the yield of the antibiotic during the fermentation. Expression of the gene may also be brought under control of other promoters, either promoters for which expression can be regulated in a different fashion or promoters that are expressed constitutively.

Illustrative promoters which find use in the subject invention, include, for prokaryotic cells, the lac, trp (Sommerville, Biotechnology and Genetic Engineering Reviews (1988), 6:1-41) or tac promoters of E.coli, or aph or tyrosine synthetase promoters of S.lividans. For filamentous fungi, illustrative promoters include the glyceraldehyde phosphate dehydrogenase (gapdh) promoter, the phosphoglycerate kinase (pgk) promoter, the nitrate reductase promoter and the like. A preferred embodiment of the present invention which is exemplified herein is the use of the pgk promoter of P.chrysogenum, which has been described in EP-A-354624.

In eukaryotic cells, a terminator provides for proper maturation of the mRNA transcript and is necessary for efficient expression. In general, it is preferable to use the native polyadenylation signal associated with the gene of interest. In both eukaryotic and prokaryotic systems, a terminator can also contain sequences or structures which increase the stability of the mRNA species and allow for higher expression. Several examples of prokaryotic sequences are known, for example the trp terminator, the gene 32 (T4) terminator, or synthetic terminators which are similar in sequence to gene 32. For eukaryotes, terminators can be used that are isolated from cloned genes. For yeast, the terminator of the CYC1 gene or the actin gene can be used for example. For filamentous fungi, the terminators isolated from for example the trpC gene, the pgk gene or the penDE gene are useful.

Where it is desired to isolate the ACVS or ACVS', secretion of the enzyme into the media or into the periplasmic space of the transformed microbial host can improve the efficiency of the isolation procedure. Secretion can be accomplished by using DNA expression cassettes as described herein, which further comprise a signal sequence (secretory leader) that is functional in the host cell. The signal sequence will be heterologous to the ACV synthetase gene and may be homologous or heterologous to the host cell, or may be a synthetic signal sequence. The signal sequence provides a peptide sequence that is in-frame with the enzyme sequence, and may be located 5' or 3' to the ACV synthetase sequence. The signal sequence can also be provided by joining, in-frame, an open reading frame of a protein that is secreted by the host cell and the open reading frame of the ACV synthetase. Illustrative secretory leaders include the secretory leaders of penicillinase, α-factor, immunoglobulin, T-cell receptors, outer membrane proteins, glucoamylase, fungal amylase and the like. By fusion in proper reading frame, the mature polypeptide may be secreted into the medium.

The promoter and terminator may be homologous (derived from the original host), or heterologous (derived from a foreign source or synthetic DNA sequences). The expression cassette thus may be wholly or partially derived from fermentable or known sources, and either wholly or partially derived from sources homologous to the host cell, or heterologous to the host cell. The various DNA constructs (DNA sequences, vectors, plasmids, expression cassettes) of the invention are isolated and/or purified, or synthesized and thus are not "naturally occurring."

The expression cassette may be included within a replication system for episomal maintenance in an appropriate cellular host or may be provided without a replication system, where it may become integrated into the host genome. Integration may be stimulated in yeast and bacteria by the inclusion of (parts of) ribosomal RNA genes or other yeast genes and subsequent linearization in these genes.

The DNA may be introduced into the host cell in accordance with known techniques, such as transformation of DNA, transfection by contacting the cells with a virus, micro-injection of the DNA into cells, biolistic transformation and the like. Both prokaryotic and eukaryotic hosts may be employed, which may include bacteria and fungi, particularly filamentous fungi. Prokaryotic cells include Escherichia coli, Flavobacterium and Streptomyces spp. Eukaryotic cells include filamentous fungi such as Penicillium chrysogenum, Acremonium chrysogenum, Aspergillus nidulans, niger and oryzae; and yeasts such as Saccharomyces cerevisiae, Kluyveromyces lactis. Preferred host cells include P.chrysogenum, A.chrysogenum and A.nidulans. Replacement of the native ACVS gene by the mutant gene may result in in vivo synthesis of β-lactam derivatives. The presence of a wild type gene in these organisms indicates that they allow for efficient expression of the wild type ACVS gene and hence they are inferred to also express the mutant gene without too many difficulties.

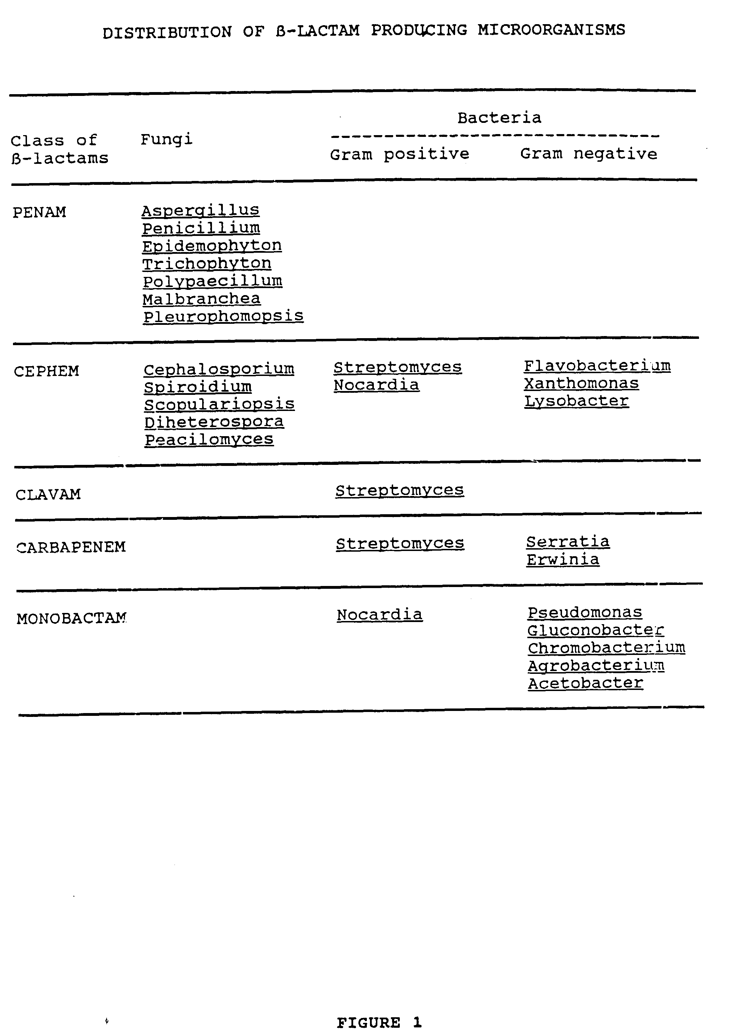

Other preferred hosts include the Streptomycetes. Several Streptomycetes also synthesize β-lactams (see Figure 1). They can be used for the same reasons as the above-mentioned fungi. On the other hand, a Streptomycete such as S.lividans is very amenable to genetic manipulation (D.A. Hopwood et al., (1985) Genetic Manipulation of Streptomyces: a Laboratory Manual, The John Innes Foundation, Norwich, U.K.). Moreover, it has been disclosed that β-lactam biosynthetic genes are actively expressed in S.lividans (Chen et al., supra). Therefore, S.lividans is a preferred host for expression of the gene, for isolation of the mutant protein, or to use for preparation of cell-free extracts. Expression of foreign genes in E.coli is very well known in the art. One disadvantage of this organism may be that the ACVS could be too large for production of active enzyme; inclusion bodies may be formed or (over)expression may be harmful to the cell. Yeasts, like S.cerevisiae or K.lactis are examples of frequently used hosts for expression of heterologous proteins.

Transformed host cells subsequently are grown under conditions that are suitable for the antibiotic production. These conditions have been amply described in the literature (Luengo et al., J. Gen. Microbiol. (1979), 115:207-211; Barredo et al., Antimicrob. Agents Chemother. (1988), 32: 1061-1067; Queener and Schwartz, In: Rose AH (ed) Secondary Products of Metabolism, Academic Press, London (1979): 35-122; Queener et al., In: Biotechnology of Industrial Antibiotics, EJ Vandamme (ed) Marcel Dekker Inc., New York, Basel (1984): 141-170). Generally, media for antibiotic production contain either a slow fermentable carbon source, like lactose, or are limited in the carbon source, e.g. glucose, in a so called fed-batch fermentation procedure. For the production of penicillin G or V the appropriate side chain precursor, as has been described hereinbefore, is added to the medium; for the production of cephalosporin the medium may be supplemented with DL methionin. Generally, Corn Steep Liquor or related compounds are supplied as a nitrogen source.

For prokaryotic hosts, the culture conditions are known in the art (e.g. T. Maniatis et al., Molecular Cloning, a Laborarory Manual, Cold Spring Harbor Laboratory (First edition, 1982 or second edition, 1989); and Hopwood (supra)); in this case it is advisable to maintain selective pressure by addition to the culture medium of the antibiotic that is used as a selective agent. The secondary metabolite is subsequently isolated from the cultured cells.

ACVS or ACVS' can be purified from the host cells that have been grown under these conditions or, alternatively, using the conditions that are most suited for the expression of the promoter used; preferably, cells are harvested early in the fermentation (2-4 days) for this purpose. For the isolation of ACVS or ACVS', the procedures as described in Van Liempt et al. (supra) or Banko et al. (supra) can be used. For isolation of ACVS or ACVS' from transformed E.coli, the cells are grown overnight in e.g. TY or LB medium (Maniatis et al., supra) and can be lysed by treatment with lysozyme. Protease inhibitors, such as PMSF or α-2 macroglobulin can be included in the buffers used, in order to avoid degradation of the ACVS or ACVS' to be purified.

Cell-free extracts can be prepared from the various cultures using the procedures as they are described in Adlington et al. (supra), Banko et al. (supra), Jensen et al. (supra), Zhang and Demain (Biochem. Biophys. Res. Comm. (1990) 196:1145-1152), Jhang et al. (FEMS Microbiol. Lett. (1989) 57:145-150). In general, cells are harvested, washed in a suitable buffer and disrupted in a French press, by grinding in liquid nitrogen or by sonication. The presence of glycerol (40-50%) as a stabilizer is crucial, both for isolation of large quantities of the enzyme and for isolation of an active cell-free extract.

The pcbAB genes of the present invention, and more particularly the DNA constructs derived therefrom, can be used to transform suitable host cells, in order to increase the fermentable or known ACV synthetase activity present in the host cells. Cells with an increased level of ACV synthetase find their use in the production of increased amounts of β-lactam antibiotics. This increased production can be the result of the increased ACV synthetase activity by itself, or in combination with an increased activity of other enzymes, known in the art. For example, host cells that have been transformed with the pcbAB gene can be retransformed with a construct containing other β-lactam-biosynthetic genes, such as the construct pGJ02 that has been described before (EP-A-357119). In this way the entire pathway leading to penicillin G or V formation can be amplified. Combined use of these two constructs can also confer the ability to synthesize penicillin to host organisms that by nature lack this ability.

Cells with increased ACV synthetase activity moreover find their use in the isolation of increased quantities of the ACV synthetase enzyme; the isolated enzyme can be used, for example, in in vitro reactions or for the further unraveling of the reaction mechanism.

Cells with increased ACV synthetase activity also find their use in the preparation of cell-free extracts with increased ACV synthetase activity, leading to improved yields -and hence an improved economy- of in vitro synthesis of β-lactam antibiotics and their precursors.

Amplification of the mutant pcbAB gene, particularly one with a phenotype of increased enzyme activity, will result in a further enhancement in intracellular enzyme activity, which in turn may be accompanied by an increase in productivity of the antibiotics of both the cephalosporin and penicillin pathways.

Cells with increased ACVS' activity also find their use in the isolation of ACVS' enzyme or in the preparation of cell-free extracts with ACVS' activity.

The following examples are offered by way of illustration and not by way of limitation.

### EXPERIMENTAL

### General Methods

In all examples described herein, the experimental techniques mentioned are known in the art: these have been described e.g. in Maniatis (supra).

### Deposits

The following organisms have been deposited with Centraal Bureau voor Schimmelcultures (CBS), Oosterstraat 1, 3742 SK Baarn, Netherlands:
E.coli WK6 containing plasmid pPCV01, Accession No. CBS 142.90, was deposited February 28, 1990;
Cosmid HM193, Accession No. CBS 179.89, was deposited on April 3, 1989 as a DNA sample (cosmid clone in E.coli unstable);
P.chrysogenum strain Wisconsin 54-1255 npe5 containing an ACV synthetase gene mutation, Accession No. CBS 178.89, was deposited on April 3, 1989.

### Example 1

### Characterization of the pcbAB gene from P.chrysogenum

### A. Isolation of the pcbAB gene

The isolation of DNA fragments encoding the ACV synthetase enzyme is described in EP-A-357119. Cosmid HM193 contains one such DNA fragment. Using parts of cosmid clone HM193 as a probe, cosmid clones containing similar or overlapping inserts have been isolated, using techniques known in the art. The presence of the pcbAB gene on the DNA fragments has been demonstrated in various ways. The DNA fragments were able to complement a mutation in the gene encoding ACV synthetase, which is present in the strain Wisconsin 54-1255 npe5, deposited as CBS 178.89. This is a mutant ACV synthetase-negative P.chrysogenum strain derived from Wis54-1255. Restoration of penicillin production by complementation was accompanied by the reappearance of in vitro ACV synthetase activity in cell-free extracts. Moreover, restoration of penicillin production was also accompanied by reappearance of a large protein on SDS-PAGE gels.

### B. Localization of the pcbAB aene

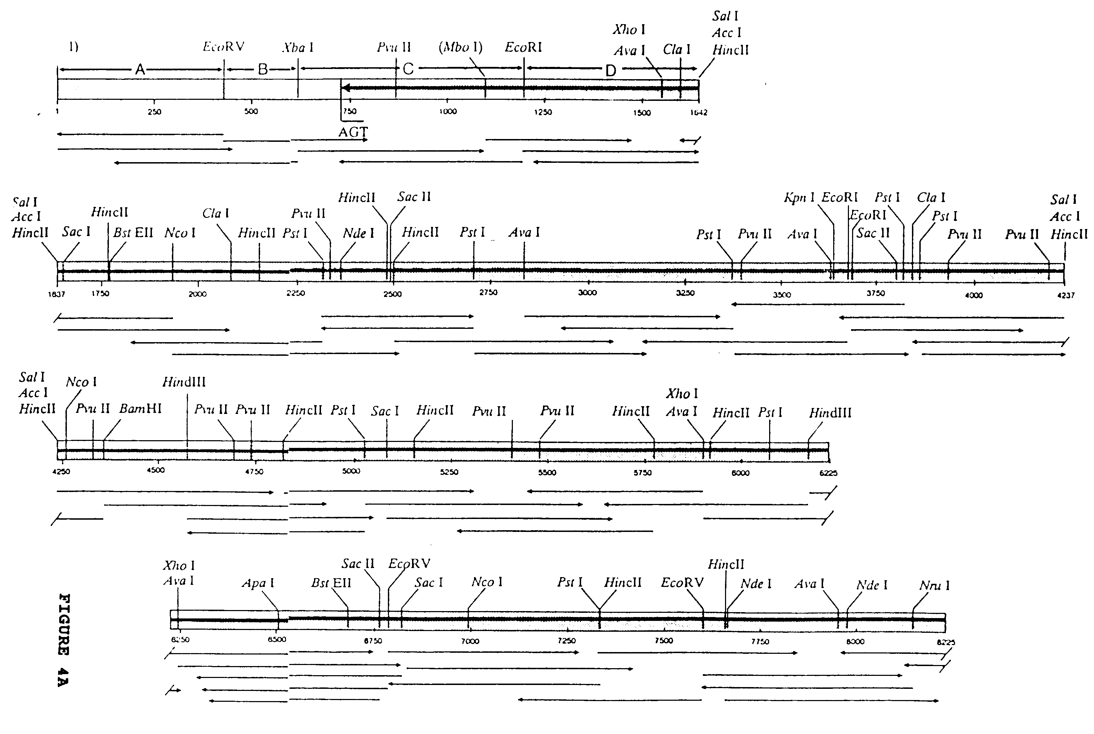

Northern hybridizations were performed using mRNA that was isolated from penicillin-producing cultures as described in EP-A-354624. The subcloned SalI fragments indicated in Figure 3 were used as probes. Using probes I, II, III and IV a large mRNA (≥ 10 x 10³ nucleotides) was detected in the Northern blot hybridizations. Probe V detected the mRNAs of the pcbC and penDE genes (about 1.5 x 10³ nucleotides). Using smaller probes (A-D and E-H in Figure 4), the positions of the putative 5'- and 3'-ends of the gene were located. The gene encoding the ACV synthetase is present on the chromosome of P.chrysogenum as indicated by the shaded region in the schematic of Figure 3.

### C. Polarity of the gene

The direction of transcription was determined by Northern hybridization of mRNA of P.chrysogenum using as a probe the following synthetic oligonucleotides that have been designed based on the nucleotide sequence surrounding the two HindIII sites located within the ACVS coding region (Figure 4).

AB1504 : 5'-CCC AGA CGC ACT TGA TCC TG-3'

AB1505 : 5'-GTC CCC GCT TGC GAC GAC TG-3'

AB1549 : 5'-CGG GAA TCA TCT GCG TAT C -3'

AB1550 : 5'-CGC GCT CAA AGG CCT GGT TC-3'

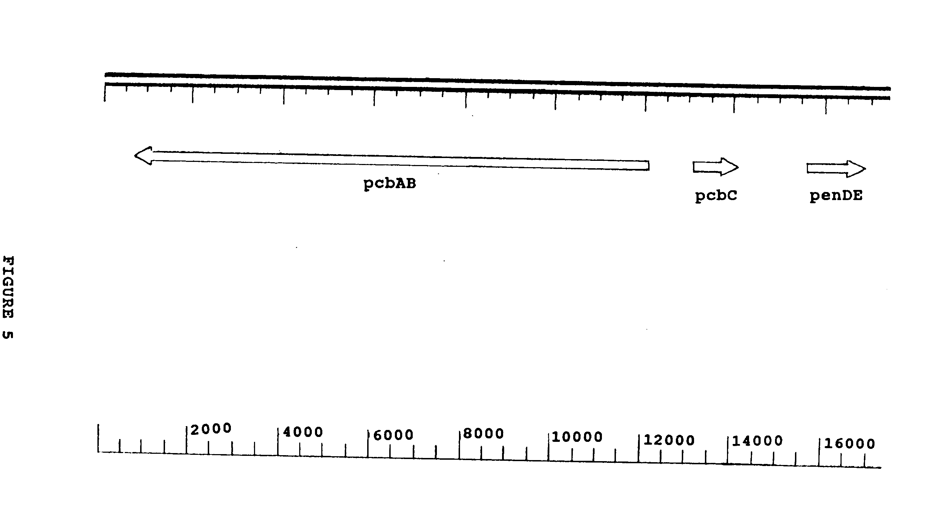

Only probes AB1549 and AB1550 hybridized to the ACV synthetase mRNA and the deduced direction of transcription is indicated in Figure 5. The direction of transcription of the ACV synthetase gene is in the opposite direction to the genes encoding IPNS and AT.

### D. Determination of the nucleotide sequence

The nucleotide sequence of a DNA fragment contained in cosmid HM193 containing the ACV synthetase gene was determined by the dideoxy method (Sanger et al., Proc. Natl. Acad. Sci. U.S.A. (1977) 74:5463-5467) using the Sequenase system 2.0 (U.S. Biochemicals, Cleveland, OH). Double sequencing reactions with dGTP and dITP were used in some clones to avoid errors (Barnes et al., (1983) Methods in Enzymology 101, 98-122). The sequencing strategy is given in Figure 4. The nucleotide sequence of the 12,364 nucleotide DNA fragment is given in sequence listing 1. In this sequence a long open reading frame (ORF) of 11,337 nucleotides was found from which a protein sequence of 3778 amino acids was deduced.

### E. Determination of partial protein sequence of ACV Synthetase

To further confirm that the gene cloned is indeed the gene encoding the P.chrysogenum ACV Synthetase, amino acid sequences have been obtained from ACV synthetase that has been purified from Aspergillus nidulans. The similarity between protein and nucleotide sequence data confirm the identity of the cloned gene.

ACV Synthetase was purified from A.nidulans, strain G-69, as has been described in Van Liempt et al., (supra).

The enzyme containing fractions from the DEAE column were applied to a Mono Q column on a FPLC apparatus (Pharmacia). ACV synthetase was eluted using a 0-300 mM NaCl gradient in Tris/HCl buffer (pH 7.5). The peak fraction, having a protein concentration of 0.5 mg/ml was shown to contain almost pure (over 90%) ACV synthetase; this was demonstrated by SDS-PAGE.

For digestion with protease, 1.25 ml (0.625 µg) of ACV synthetase from the Mono Q fraction was incubated with 62.5 µl (6.3 µg) of Subtilisin (Sigma) for 60 min. at room temperature. The reaction was terminated by the addition of trichloroacetic acid (10% final concentration). The precipitated protein was recovered by centrifugation, dissolved in approximately 200 µl of Laemmli sample buffer, and the mixture was neutralized by the addition of 4M Tris. The protein was dissolved and incubated for 5 min. at 95°C.

A slab gel of 140 x 170 x 1.5 mm of polyacrylamide (separating gel 5% T), with a 3% stacking gel (Laemmli) was allowed to polymerize overnight at 4°C. The digested protein was applied in several slots, and electrophoresis was carried out with 0.02% thioglycolic acid in the upper buffer compartment. After the Bromophenol Blue marker had migrated up to 70% of the gel length, the electrophoresis was terminated and the proteins were transferred electrophoretically onto a PVDF-membrane (Immobilon) in a semidry blotting apparatus (Sartorius). The transfer buffer was 25 mM Tris/HCl (pH 8.5), 0.5 mM dithioerythritol.

After transfer (approximately 2 hrs.) the membrane was washed with water, stained in 0.5% Coomassie R 250, 50% Methanol for 5 min., destained in 50% Methanol, 10% Acetic Acid, washed with water and air dried.

From the complex pattern of protein bands present on the membrane, the bands that were the most pronounced and least contaminated with other nearby bands were excised. The amino acid sequence of the peptides in the excised bands was determined using a gas-phase sequenator (Applied Biosystems model 470a). The following sequence was determined (in this notation amino acids separated by a slash indicates ambiguity of interpretation at this position while an amino acid in parentheses indicates uncertainty in the interpretation; Xxx indicates the presence of an unidentified amino acid):

band 3: Asn Ala Asn Val Tyr Leu Ala Asn Ser Leu Gln Gln Gly
Phe Val Tyr Gln Phe Leu Lys Asn Met Gly Asp/Arg Ser Glu/Trp
Ala Asp/Tyr Asp/Val Met Gln Xxx Val (Thr) (Asp/Arg) Tyr

band 9: Gln Ser Val Gln Xxx Ile Lys Ser Val Ala Lys Phe Asp
Leu Asn (Ala Thr) Ala Xxx (Glu) (Leu/Ser Asp/Gly Lys Ala)

band 12B: (Gln/Ser/Cys Gln Thr) Val Leu Gly Asp Ala Pro Leu
Leu Pro Ile Gln (Thr/Gln His/Gln Phe)

### F. Comparison of protein and nucleotide sequences

The amino acid sequences from Example 1E (A.nidulans) were compared with the deduced amino acid sequence from Example 1D (P.chrysogenum), which is shown in sequence listing 1. For this comparison the MicroGenie™ 6.0 program (Beckman) was used.

The following results have been obtained:

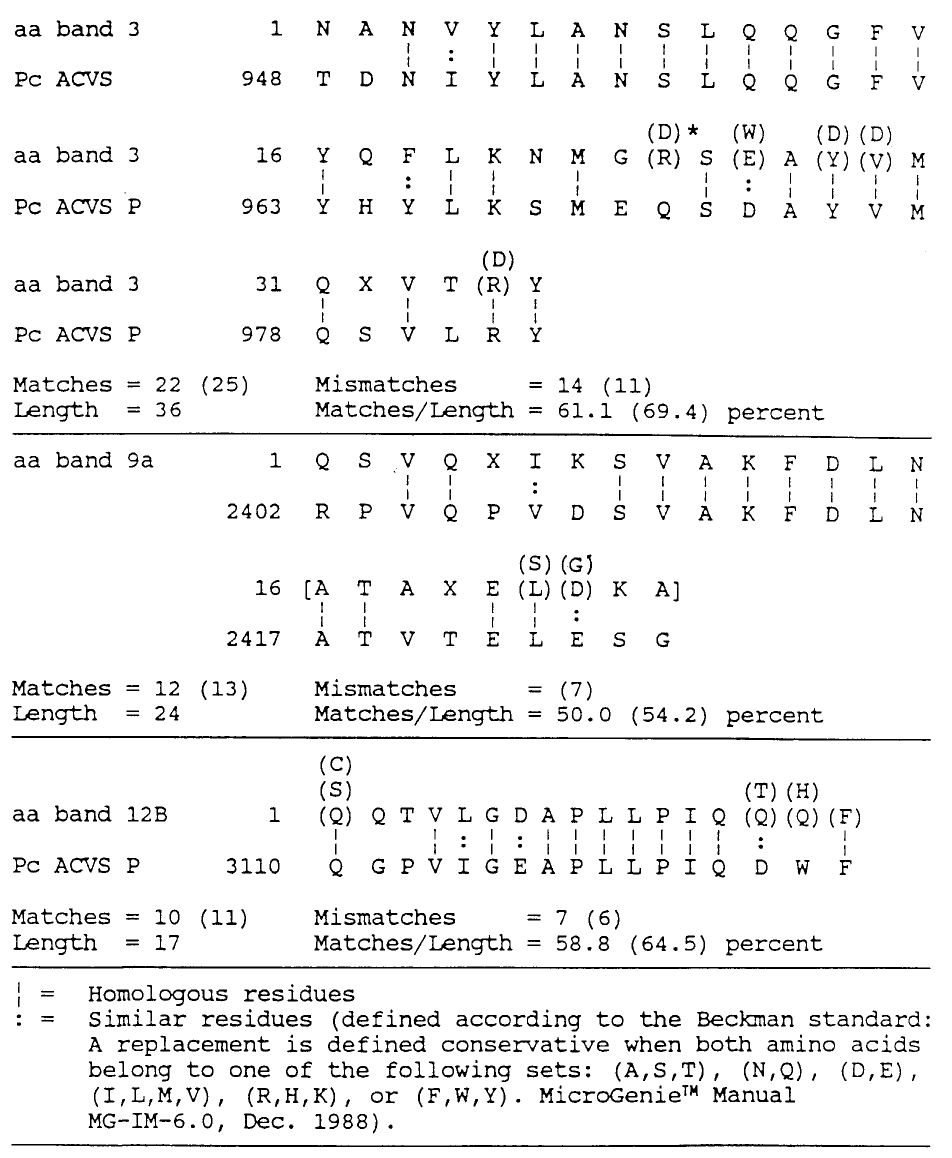

The degree of homology found between A.nidulans and P.chrysogenum varies between 50 and 61%. This is very similar to the degree of homology between the IPNS proteins derived from both organisms (e.g. G. Cohen et al., Trends in Biotechnology (1990), 8:105-111); hence the protein data confirm the conclusion that the gene cloned is indeed the P.chrysogenum ACV synthetase gene.

### G. Identification of distinct domains in the ACVS enzyme which are used to obtain ACVS' enzyme

From the nucleotide sequence of the pcbAB gene an amino acid sequence has been deduced, as indicated in sequence listing 1. Upon matrix comparison of this protein sequence with itself, three distinct regions of homology are found: these regions are defined herein as domains. These domains are located between amino acids 301 and 1068 (domain I), 1392 and 2154 (domain II) and between amino acids 2474 and 3295 (Domain III; Figures 13 and 15). Similar domains were found in the pcbAB gene of A.chrysogenum. Within these domains, several even more conserved elements can be distinguished. A summary is given in Figure 15. Since the ACVS enzyme synthesizes a tripeptide, which most probably requires the activation of three amino acids, a role of these three domains in the amino acid activation reactions seems likely. Therefore, the said domains are candidate regions for in vitro mutagenesis.

Comparison of the deduced amino acid sequence with the protein sequences known for other multifunctional enzymes reveals a significant homology with the Bacillus brevis tyrocidin synthetase I (hereinafter referred to as TYI; Weckermann et al., Nucleic Acids Research (1988), 16:11841) and gramicidin synthetase I (hereinafter referred to as GSI; Krätzschmar et al., (supra); compare Figure 13). Since both TYI and GSI are involved in activation and racemisation of the amino acid phenylalanine, this homology supports the notion that these conserved sequences may represent centers involved in ATP-mediated activation of amino acids. If the mechanism of peptide synthesis by ACVS or Gramicidin Synthetase is comparable, the expected order of the domains in the enzyme is in the same order as that of the amino acids in the tripeptide (Krätzschmar, supra).

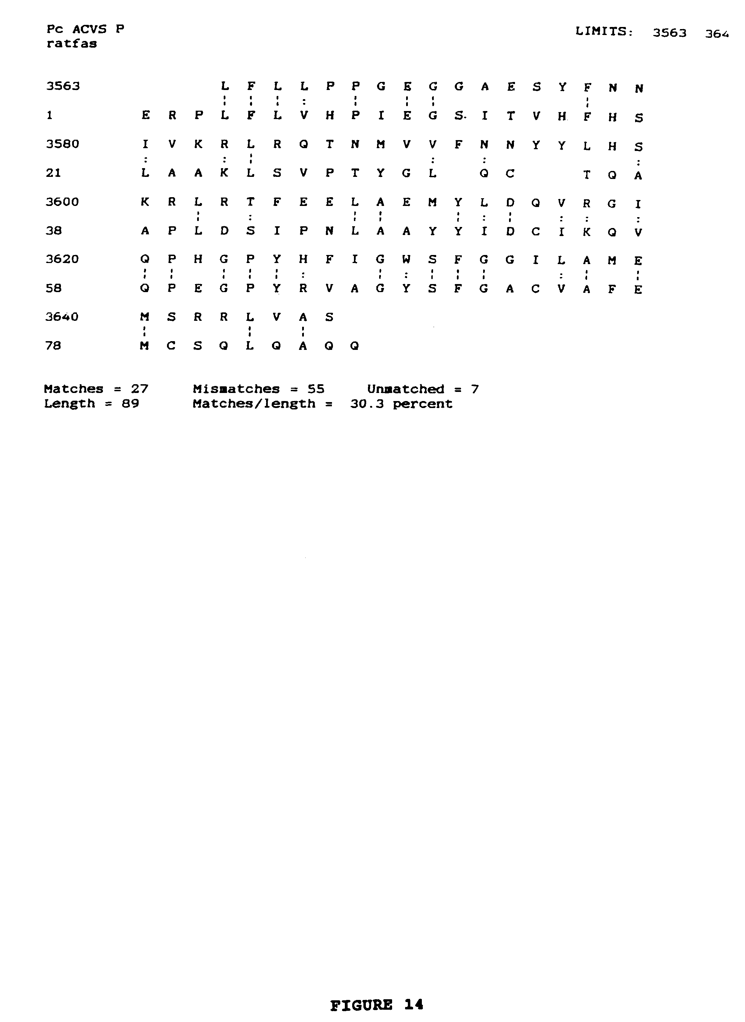

Upon comparison of the ACVS protein sequence with other known protein sequences of large enzymes, a significant homology is found with the Fatty Acid Synthetases (long chain) from rat and chicken (Figure 14; M. Schweizer et al., Nucleic Acids Research (1989) 17:567-586; Z. Yuan et al., PNAS (1988), 85:6328-6331). The homology is found between the COOH-terminal part of the ACVS protein (domain IV in Figure 15) and the thioesterase domain of the long chain Fatty Acid Synthetase proteins. Even the active site of the thioesterase, viz. G.X.S.X.G. (e.g. Krätzschmar, supra), is present in the ACVS protein sequence. A similar homology has been described for a subunit of gramicidin Synthetase; in this case the homology is found between the grsT subunit and the type II fatty acid synthetases. The demonstration of the said homology suggests that ACVS most probably also contains within its structure the ability to release the tripeptide, once it has been formed and bound to the enzyme by thioester bond formation, by the action of its thioesterase domain. This activity may form another target for in vitro mutagenesis, e.g. by increasing the efficiency of the release of the tripeptide, in case this part of the reaction is the rate determining step. However, in experiments designed to change substrate specificity, this domain preferably remains unchanged.

The functional domains of the ACVS protein being identified, it now is possible to construct modified peptide synthetases by the exchange of functional domains. This can be achieved e.g. by interchanging the three domains that have been identified within the ACVS protein. An alternative way is the exchange of ACVS specific domains with domains from other proteins known to have a similar function. Selected domains can be isolated by restriction enzyme digestion of clones containing the genes encoding the said proteins or domains thereof. However, suitable restriction sites at useful positions are seldom encountered. Therefore, the Polymerase Chain Reaction offers a good alternative for the isolation of suitable DNA fragments. In general, DNA fragments containing functional domains are prepared as follows: for each domain two oligonucleotides are designed, one starting at the N-terminal end of the selected domain, in the direction of the C-terminal end. The other oligonucleotide is derived from the C-terminal end of the domain and is designed in the opposite direction; consequently this oligonucleotide is derived from the other DNA strand. At the 5'-end of each oligonucleotide a suitable restriction site can be included in the oligonucleotide, in order to facilitate ligation of the domains after amplification. The DNA fragments between the two oligonucleotides are amplified using the polymerase chain reaction, thereby following the protocols known in the art (described e.g. in 'PCR-Technology', supra). Preferably, Taq DNA polymerase is used for the amplification reaction.

### Example 2

### Expression of the pcbAB gene from P.chrysogenum in P.chrysogenum

### Synthesis of pPCV01:

This vector was derived from pBluescript II KS M13(+) (Stratagene, La Jolla, CA) and contains the phleomycin resistance gene under control of the P.chrysogenum pgk promoter. It moreover contains a synthetic multiple cloning site, including a unique SpeI site. The P.chrysogenum pgk gene has been isolated from a genomic cosmid library (EP-A-357119) using the corresponding gene of Saccharomyces cerevisiae (Dobson et al., Nucleic Acid Research (1982) 10:2625-2637) as a probe (Van Solingen et al., Nucleic Acid Research (1988) 16:11823). The sequence of part of the promoter is disclosed in EP-A-354624. The promoter and a small part of the coding region can be isolated as a 1.5 kb HindIII fragment.

### Synthesis of PCV02:

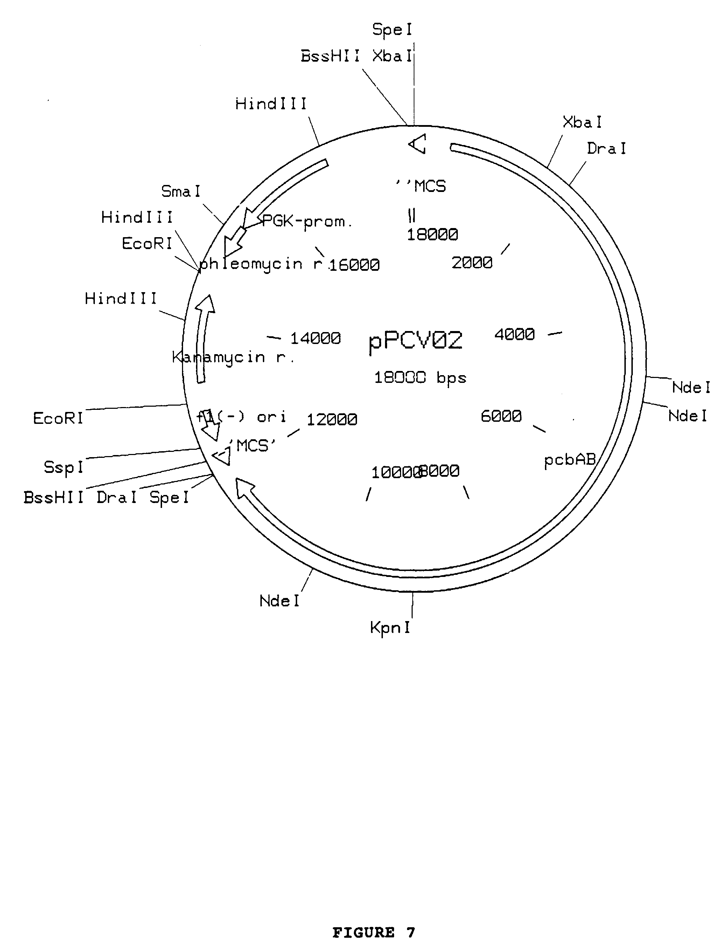

The pcbAB gene was isolated from cosmid clone HM193 as a 1.2 x 10⁴ nucleotide SpeI fragment and was subcloned into the vector pPCV01, using the unique SpeI site. Conditions used were as disclosed in Maniatis et al. (supra). The resulting construct was named pPCV02 (Figure 7). The orientation of the gene in the vector has been determined by digestion with restriction enzymes.

### Transformation

The plasmid pPCV02 has been transformed into P.chrysogenum Wis54-1255 npe5 (CBS 178.89) using the procedure described in EP-A-260762. Strain npe5 is a non-producing mutant of Wis45-1255; the npe phenotype is caused by the absence of ACV synthetase activity. Transformants have been selected for resistance against 30 µg/ml of phleomycin. Isolated transformants have been tested in a bioassay, as described in EP-A-354624, for a restoration of penicillin production. In a representative experiment penicillin production has been restored in 80% (8 out 10) of the pPCV02-transformants analysed; in transformants having received the vector pPCV01, without the ACV synthetase insert, a restoration of the penicillin production has not been demonstrated (0 out of 26).

The construct pPCV02 is also transformed into wild type P.chrysogenum. Selected transformants are assayed for an increased ACV synthetase activity, using cell-free extracts as described in EP-A-357119, or for an increased penicillin productivity, using shake flask experiments as have been described in EP-A-354624.

### Example 3

### Expression of the pcbAB aene from P.chrysogenum under control of the P.chrysogenum pgk promoter

### Transcription of the pcbAB gene is subiect to glucose repression

mRNA preparations, isolated from Penicillium cultures grown on either glucose- or lactose-containing media (EP-A-354624) are transferred to Genescreen-plus® (NEN/DuPont) and hybridized with the 1.5 x 103 nucleotide HindIII fragment, which is internal to the pcbAB gene (Figure 4). In glucose-grown cultures, no pcbAB mRNA is detected, while in the lactose grown cultures, a large mRNA (≥ 10 x 10³ nucleotides) is detected.

### Construction of pPCV03

The region surrounding the ATG start codon of the pcbAB gene is isolated as a 1.7 x 10³ nucleotide SalI-DraI fragment. The vector pTZ18R (US Biochemical Corporation, Cleveland, OH) is digested with SalI and SmaI restriction enzymes. The digested vector and the 1.7 x 10³ fragment are ligated. A construct containing the pTZ18R vector bearing the 1.7 x 10³ fragment insert is isolated. Into this construct, the P.chrysogenum pgk promoter is ligated as a 1.5 x 10³ nucleotides HindIII fragment (EP-A-354624). A construct containing the pgk promoter in the desired orientation (same polarity as the pcbAB fragment) is isolated. From this construct single stranded DNA is isolated by superinfection with the helper phage M13K07, (U.S. Biochemical Corporation, Cleveland, OH) using techniques known in the art or as prescribed by the supplier of the pTZ cloning vector. By in vitro mutagenesis using a synthetic oligonucleotide having the following sequence: 5'-TGG CTT CAG TTG AGT CAT ATG GGT AGT TAA TGG TAT-3', a DNA fragment containing the mature pgk region and the region upstream of the pcbAB ATG is looped out. This mutagenesis introduces an NdeI site at the position of the ATG (underlined in the oligonucleotide sequence). The construct is named pTZpgk::acvsl. This construct is partially digested with HindIII and XbaI and the promoter-gene fusion is isolated on a DNA fragment of 2.9 x 10³ nucleotides as described by Maniatis et al., (supra). This fragment is ligated with the 16.3 kb DNA fragment containing the pcbAB gene, isolated after partial XbaI digestion of pPCV02, the remaining HindIII-XbaI sticky ends are filled in with T4 DNA polymerase and the construct is circularized by the addition of ligase.

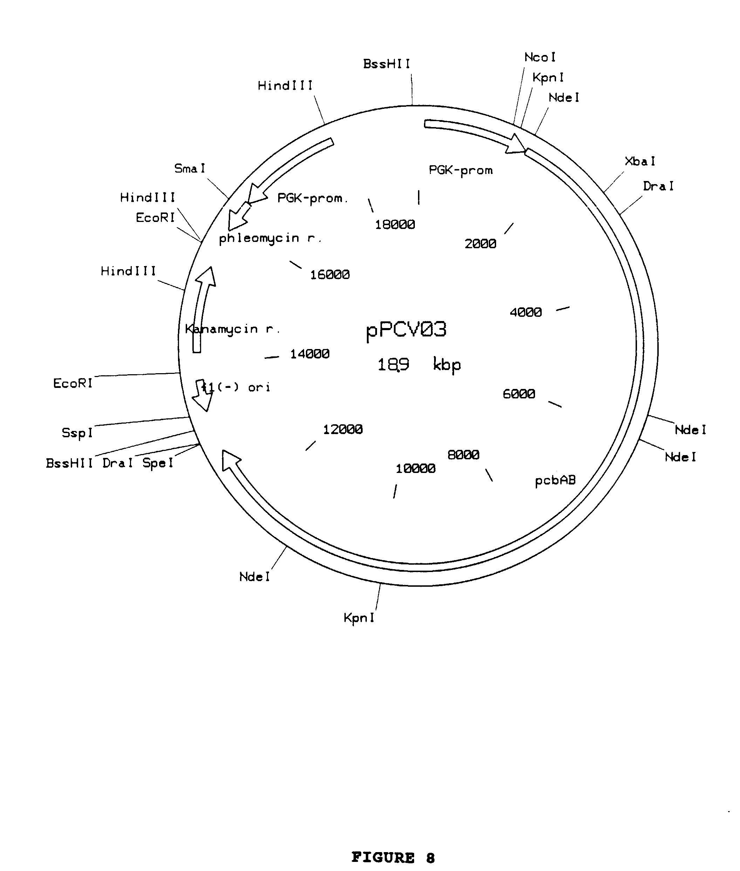

The resulting construct, pPCV03 (Figure 8), is isolated as follows. The ligation mixture is transformed into E.coli HB101 (ATCC 33694) using standard techniques. Plasmid DNA is isolated from several transformants and analysed by restriction enzyme-digestion and agarose gel electrophoresis. Transformants containing the correct DNA constructs are grown on large scale (500 ml) and plasmid DNA is isolated using methods as disclosed in Maniatis et al., (supra) and transformed into P.chrysogenum. Expression of the pcbAB gene, enzyme activity and penicillin production of transformants is analyzed after growth in shake flasks on both glucose- and lactose-containing media; the data obtained are compared with those obtained for transformants containing pPCV02. In contrast to transformants containing pPCV02, transformants containing pPCV03 express the pcbAB gene in glucose-containing media: both an ACV synthetase-specific mRNA and ACV synthetase enzyme activity are detected.

### Example 4

### Expression of the pcbAB gene in E.coli

For efficient production of ACV synthetase enzyme in E.coli it is necessary to place the pcbAB gene under control of a promoter which allows efficient gene expression in E.coli. Examples of such efficient promoters are the trp promoter, the lac promoter and the tac promoter. In this example the trp promoter is described, but it will be obvious to those skilled in the art that the experiments can be easily repeated with the lac and tac promoters, leading to similar results. If desired, a runaway replicon can be included in the construct; this will allow for controlled amplification of the plasmid copy number after a temperature shift.

### Synthesis of pMA-ACVS:

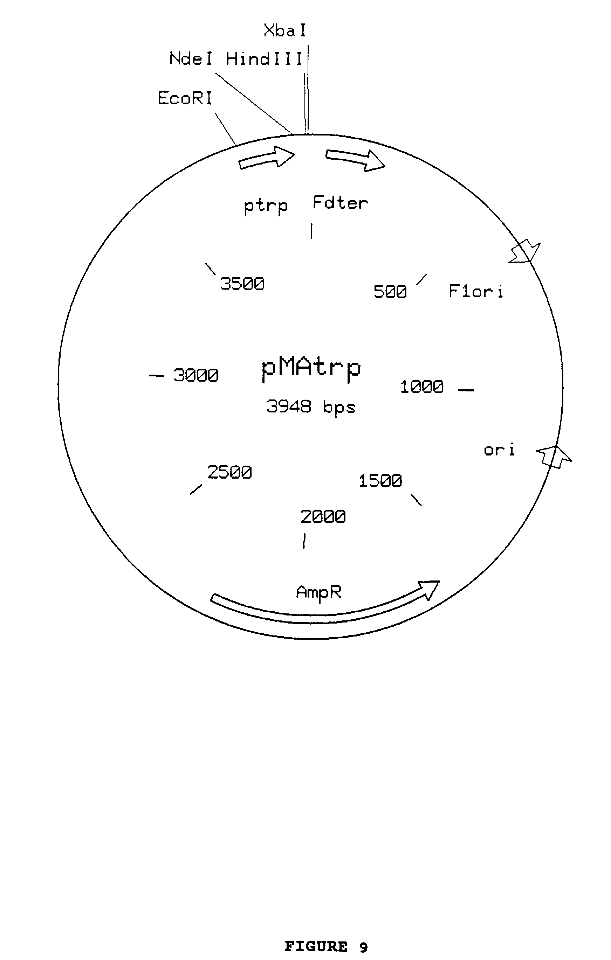

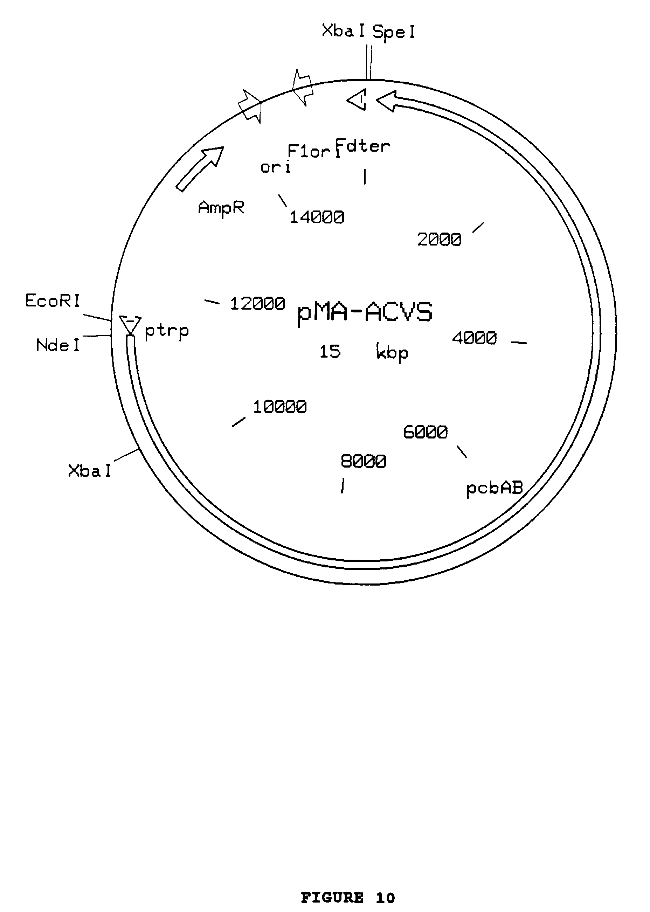

pMAtrp (Figure 9), having the trp promoter region between -113 and the ATG transcription start flanked by an NdeI site at the position of the ATG (Sommerville, supra), is digested with SmaI and NdeI. From pPCV03 a 1.5 x 10³ nucleotide NdeI-DraI fragment, containing the 5'-part of the pcbAB gene, is isolated and ligated into the SmaI, NdeI digested pMAtrp. Into the XbaI sites of the resulting construct, the 3'-part of the pcbAB gene is inserted as a 1.0 x 10⁴ nucleotide XbaI fragment isolated from HM193 or pPCV02. Constructs are selected for the correct orientation of the inserted XbaI fragment; the construct containing the fragment in the correct orientation is named pMA-ACV synthetase (Figure 10). Orientation is determined by digestion with various restriction enzymes.

pMA-ACV synthetase is isolated and used to transform a suitable E.coli host, such as, for example E.coli HB101, E.coli C600 or E.coli JM101. Transformants are analyzed by determination of ACV synthetase activity in cell-free extracts, by electrophoresis of cell-free extracts in 5% polyacrylamide gels (SDS-PAGE) and by immunoblotting, using a polyclonal antiserum which had been raised against purified ACV synthetase.

### Example 5

### Expression of the pcbAB gene from P.chrysogenum in Streptomyces lividans

For expression in a Streptomyces host, several options are available. S.lividans is a preferred host because of the ease of transformation of this host, as compared to several other Streptomycetes.

### Expression of the P.chrysogenum ACV synthetase gene under control of the aph promoter

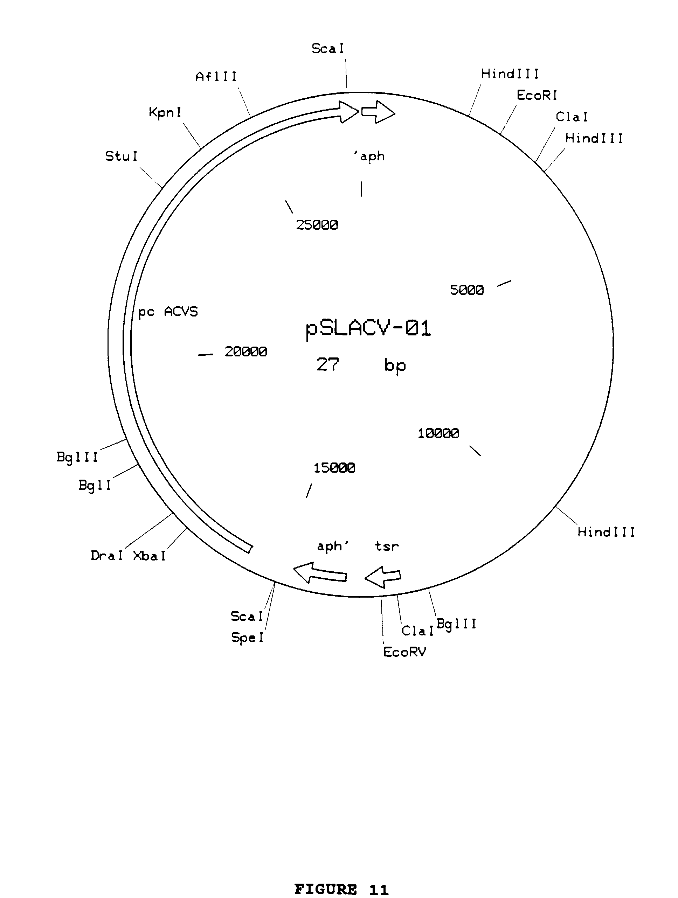

The P.chrysogenum ACV synthetase gene is isolated as the 1.2 x 10⁴ nucleotide SpeI fragment described in Example 2. The sticky ends of this fragment are made blunt by treatment with T4 DNA polymerase using the procedures known in the art. The vector pIJ61 has been described by C.J. Thompson et al., (1982) Gene 20, 51-62); reviewed in. Hopwood et al. (supra); the vector can be obtained from D.A. Hopwood. The vector pIJ61 was digested with BamHI, and the ends were made blunt using T4 DNA polymerase. Subsequently, the blunt-ended 1.2 x 10⁴ nucleotide SpeI fragment is inserted into the blunt-ended BamHI site via ligation, and the mixture is used to transform S.lividans 66, by the method of Hopwood et al. (D.A. Hopwood et al., 1985, supra). Transformants are selected for resistance to thiostreptone (50 µg/ml) and are subsequently analyzed for the orientation of the pcbAB gene within the aph gene. A construct having the pcbAB gene in the same orientation as the aph gene is named pSLACV-01 (Figure 11).

Selected transformants containing the plasmid pSLACV-01 were cultured as described in Chen et al., (supra). Cell-free extracts are prepared and analyzed by SDS-PAGE or immunoblotting for the presence of a large (>250 kDa) protein; the ACV synthetase activity in the extracts is also determined using the procedure as described by Van Liempt (supra).

### Expression of the P.chrysogenum pcbAB aene in S.lividans under control of the tyrosinase promoter

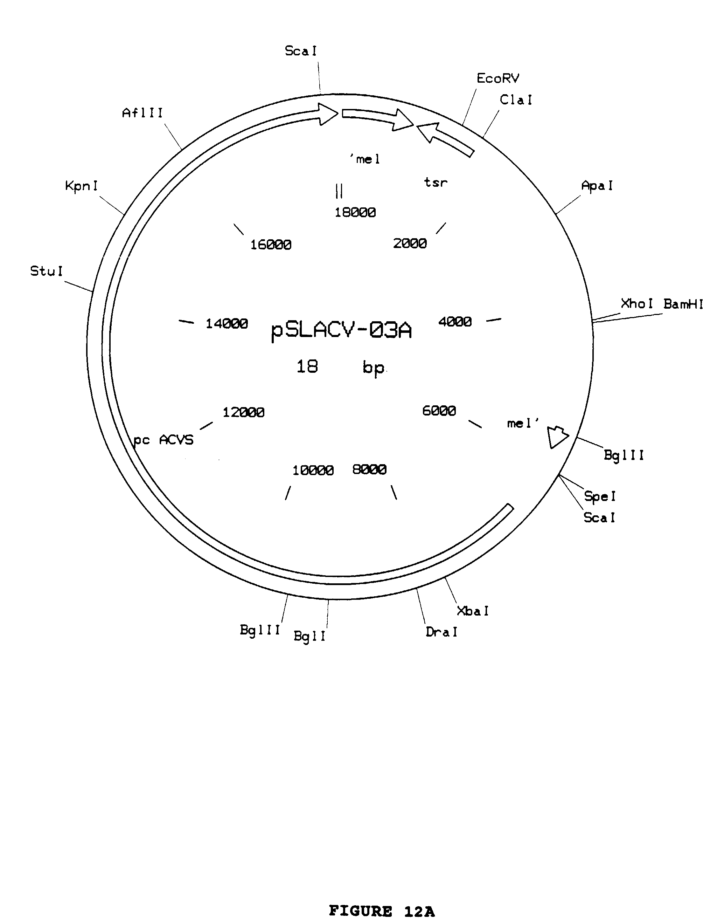

The vector pIJ702 is digested with either BglII or SstI. pIJ702 is described by E. Katz et al., Journ. Gen. Microbiol. (1983) 129:2703-2714; reviewed in: D.A. Hopwood et al., supra; obtainable from D.A. Hopwood. BglII digested pIJ702 is made blunt-ended by treatment with T4 DNA polymerase; SstI digested pIJ702 is treated with Mung bean nuclease in order to obtain blunt ends. The blunt-ended SpeI fragment containing the P.chrysogenum pcbAB gene as described hereinabove is inserted into both blunt-ended vectors via ligation. Thiostreptone resistant transformants are screened for a melanin-negative phenotype by application of a soft agar overlay containing tyrosine (0.1 mM) as described in Hopwood et al. (supra). Melanin-negative transformants contain an interrupted tyrosinase gene (by insertion of the ACV synthetase gene) and their colonies remain colorless upon addition of tyrosine, while wild type colonies turn brown. Analysis by restriction enzyme digestion and agarose gel electrophoresis for the correct orientation of the inserts yielded plasmids pSLACV-03A (SstI site; Figure 12A) and pSLACV-03B (BglII site; Figure 12B).

Analysis of transformants is as described hereinabove. Induction of the tyrosinase promoter is established by the addition of methionine (10 mM) to the culture medium.

### Example 6

### Isolation and characterization of the pcbAB gene from A.chrysogenum

### Isolation of the A.chrysogenum pcbAB gene

A gene library of A.chrysogenum C10 (ATCC 48272) has been constructed in the lambda cloning vector EMBL3. To this end, A.chrysogenum DNA has been partially digested with Sau3A and fragments ranging in size from 13 to 17 x 10³ nucleotides have been isolated by sucrose gradient ultracentrifugation. The vector EMBL3 has been digested with BamHI and arms have been separated and purified by sucrose gradient centrifugation. About 0.4 µg of vector arms have been ligated with 0.5 µg of partially digested, purified A.chrysogenum DNA; the ligation mixture has been packaged in vitro using the lambda phage packaging system of Amersham (Buckinghamshire, UK), following the procedure provided by the supplier. The packaged mixture has been used to infect E.coli Q-359 (ATCC 47019); about 70,000 plaques have been obtained.

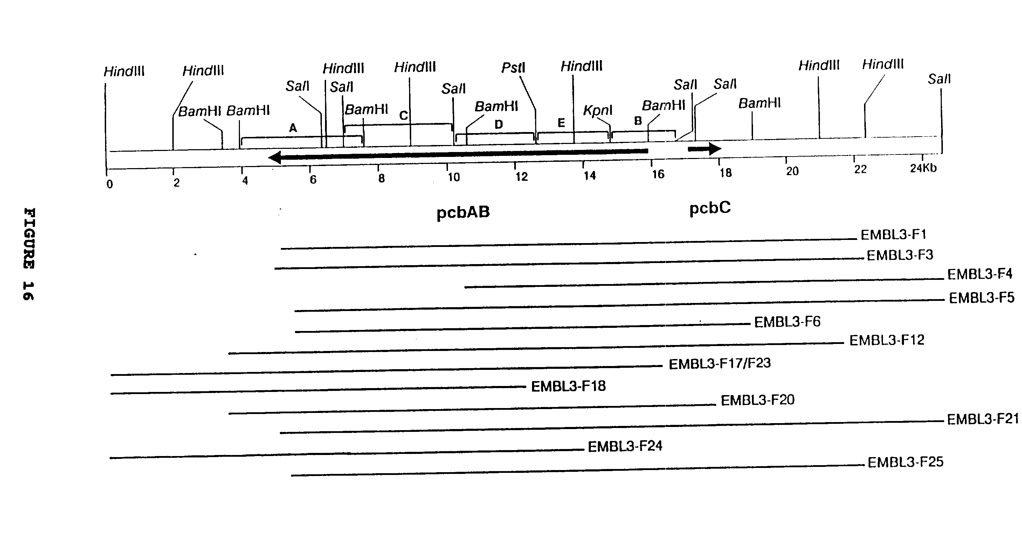

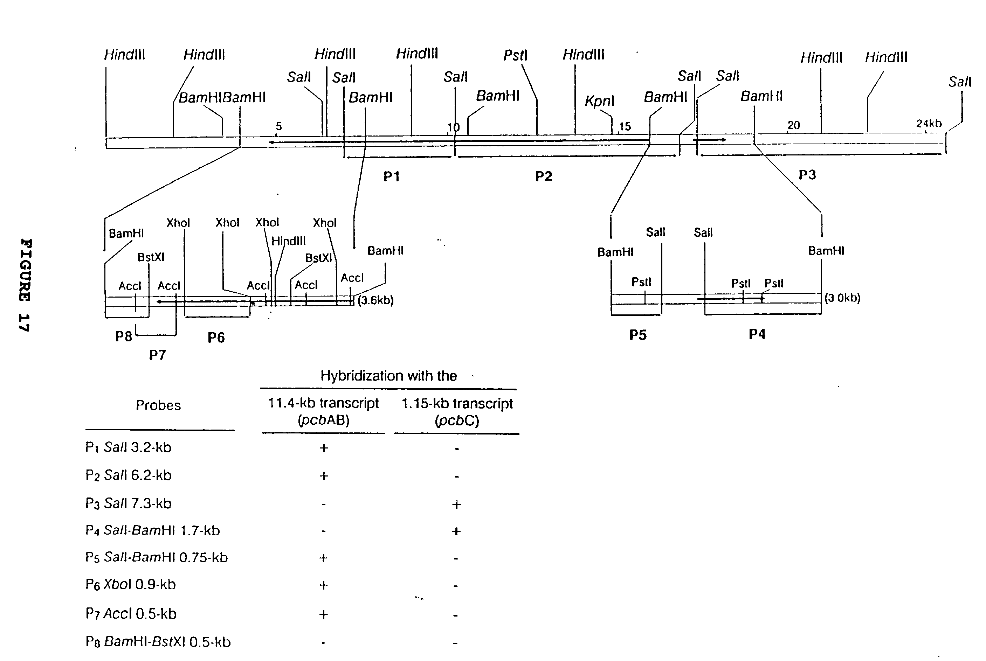

In order to isolate phages containing the A.chrysogenum pcbAB gene, the gene library first was screened using as a probe the isolated pcbC gene from P.chrysogenum, namely a 1 x 10³ nucleotides NcoI fragment, carrying the promoterless gene (Barredo et al., (1989)b, Mol. Gen. Genet. 216:91-98); conditions have been described in Barredo et al., 1989b (supra). Five phages, showing a positive hybridization signal have been purified and a restriction map has been constructed. The position of these phages (F1, F3, F4, F5 and F6) relative to a restriction map of the genomic region is given in Figure 16.

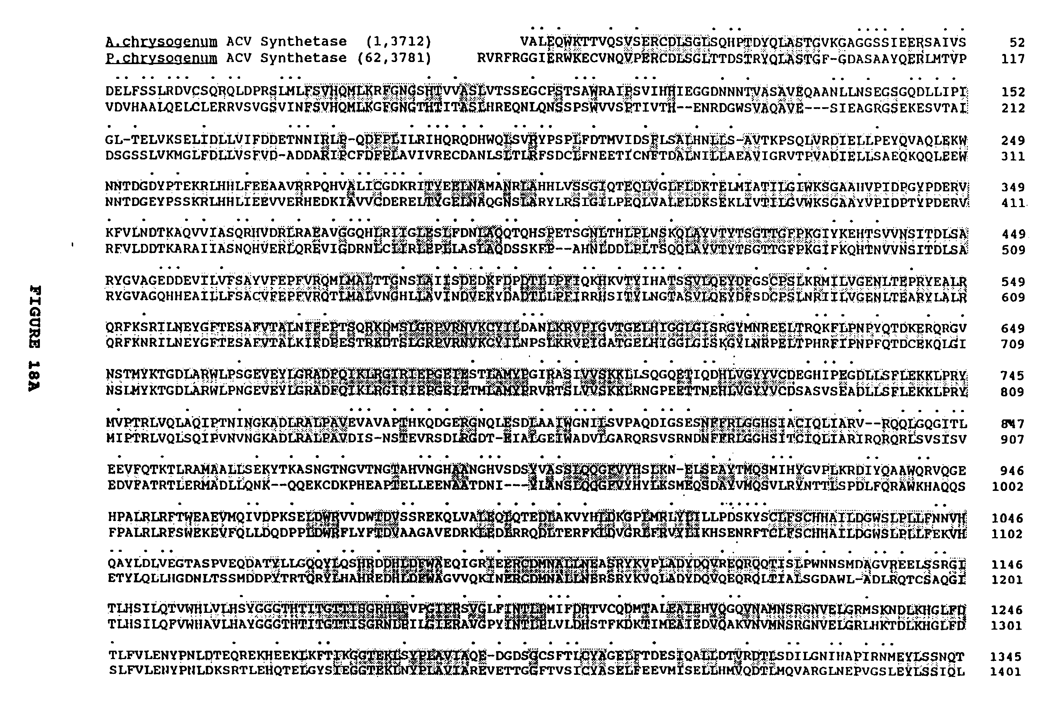

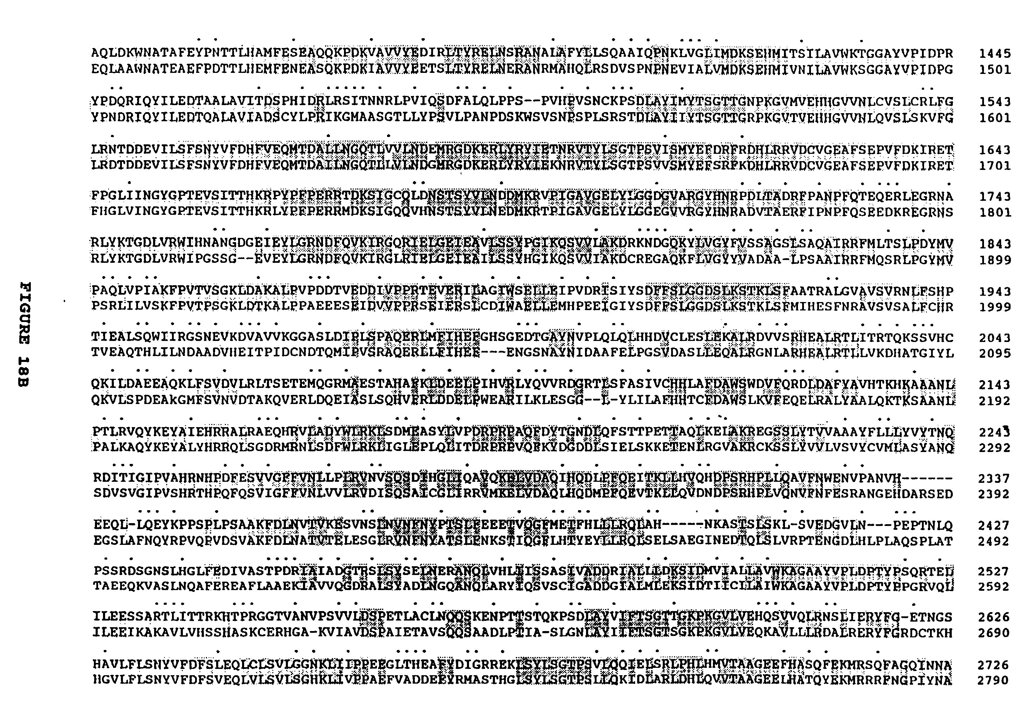

To identify the presence of another gene on the phages, the isolated DNA has also been hybridized with a 6.0 x 10³ nucleotides SalI fragment (III in Figure 3) derived from the P.chrysogenum pcbAB gene as it is present in, for example, HM193. All five phages showed a strong hybridization signal with this latter probe. This finding indicates that in A.chrysogenum the pcbC and pcbAB genes are linked, as they are in P.chrysogenum and as indicated in Figure 16. To isolate phages with an insert containing more upstream sequences, as compared to the isolated phages (which extend a maximal 11.5 x 10³ nucleotides upstream from the 5'-end of the pcbC gene) the library has been rescreened using as a probe a 0.9 x 10³ nucleotides Xho fragment (probe P6 in Figure 17) isolated from, for example, phages F1, F3, F5 or F6. This rescreening has yielded another seventeen phages. Eight of these phages have been further purified and characterized; the position of the phages F12, F17, F18, F20, F21, F23, F24 and F25 relative to a restriction map of the genomic region is given in Figure 16.

### Localization of the pcbAB gene of A.chrysogenum.

Northern blot hybridizations have been performed using mRNA that has been isolated from A.chrysogenum following the procedure described in Barredo et al., 1989b (supra). As probes, the fragments P1-P8 as indicated in Figure 17 have been used. Probes P3 and P4 hybridize to a transcript of about 1.15 x 10³ nucleotides; this transcript is derived from the pcbC gene and encodes the IPNS enzyme. Probes P1, P2, P5, P6 and P7 hybridize to a large mRNA, size >11 x 10³ nucleotides; this indicates the presence of a large gene in the region covered by these probes. Moreover, the ends of the large transcript have been more accurately mapped by the results using the small probes P5-P8: absence of hybridization using probe P8 indicates that the distal end of the putative pcbAB gene is located within the 0.5 x 10³ nucleotides AccI fragment preceding P8 (namely in region P7 in Figure 17) while the hybridization patterns of probes P4 and P5 indicate that the proximal end of the putative pcbAB gene has to be present outside region P4, most probably within region P5. The orientation of the gene has been established by homology with the P.chrysogenum gene; it is transcribed in the opposite direction to the pcbC gene (Figure 16), which also occurs in P.chrysogenum.

### Determination of the nucleotide sequence of the pcbAB gene from A.chrysogenum.

The nucleotide sequence of the region encoding the putative pcbAB gene of A.chrysogenum has been determined. Five subclones have been constructed in pBluescript KS(+) (Stratagene, LaJolla, U.S.A.), starting from phage F12 (Figure 16). The subclones contain the following fragments: A (3.6 x 10³ nucleotides BamHI), B (1.7 x 10³ nucleotides SalI-KpnI), C (3.2 x 10³ nucleotides SalI), D (2.4 x 10³ nucleotides SalI-PstI) and E (2.0 x 10³ nucleotides PstI-KpnI). Fragment A has been cloned in both orientations in pBluescript KS(+) which had been digested with BamHI; subsequently the fragment has been subcloned into 23 smaller, overlapping fragments, which have been sequenced using the dideoxy chain termination method (Sanger et al., Proc. Natl. Acad. Sci. (U.S.A.) (1977) 74:5463-5467), using either Sequenase (U.S. Biochemicals, Cleveland, OH) or Taq polymerase (Promega, Madison, Wisc). The sticky ends of fragment B have been made blunt, using standard techniques, and the blunt end fragment has been cloned into EcoRV digested pBluescript KS(+). Subsequently, the fragment has been subcloned into fourteen overlapping smaller fragments. These fragments have been sequenced using the dideoxy chain termination method.

Fragments C, D and E have been sequenced by generating sets of ordered deletion mutants using the "erase-a-base" system (Promega, Madison, Wisc). To this end, fragment C has been subcloned into SalI-digested pBluescript KS(+) in both orientations; the clones have been opened by digestion with BstXI and XbaI to obtain appropriate ends to generate the deletions. Fragment D has been subcloned in both orientations into EcoRV-digested pBluescript KS(+), after filling in the sticky ends of the fragment. For the generation of sets of deletion mutants, the clones have been opened by digestion with PstI and EcoRI. Fragment E (after filling in the sticky ends) has been subcloned into EcoRV digested pBluescript KS(+) in both orientations; clones have been opened for generation of sets of deletion mutants by digestion with EcoRI and PstI.

The digested clones of the fragments C, D and E have been treated with exonuclease III, followed by deletion of the remaining sticky ends with exonuclease S1. The gaps that have been introduced by both enzymes have been repaired with Klenow DNA polymerase, followed by ligation of the linear molecules and transformation into E.coli. For all treatments, the conditions recommended by the supplier of the "erase-a-base" system have been used. The resulting fragments have been sequenced using the dideoxy chain termination method. Clones overlapping the junctions of the five fragments have also been sequenced; the entire region has been sequenced in both strands. The nucleotide sequence of a 11.8 x 10³ nucleotides DNA fragment is shown in sequence listing 2. In this sequence a long open reading frame (ORF) of 11,139 bp has been demonstrated. From this ORF a protein sequence of 3712 amino acids has been deduced (sequence listing 2).

### Similarity with the P.chrysogenum pcbAB sequence

The nucleotide sequence of the A.chrysogenum pcbAB gene has been compared with the nucleotide sequence that has been determined for the P.chrysogenum pcbAB gene (Example 1; sequence listing 1). A 62.9% homology has been determined at the nucleotide level. At the protein level the similarity is 54.9% (based on the deduced amino acid sequences); a comparison between the deduced amino acid sequences for the P.chrysogenum and A.chrysogenum ACVS is given in Figure 18.

The ACV synthetase enzyme activities reside on a single gene in P.chrysogenum and A.chrysogenum. Elevated activity of these enzymes can be obtained in host cells using the recombinant compositions of the instant invention. Heterologous expression of ACV synthetase can provide a means of using more efficient and robust hosts for the commercial production of ACV synthetase and its secondary metabolites. Finally, the availability of large amounts of the ACV synthetase-enzyme, for example, by expression of the gene in a suitable host, will allow for better prospects for commercial application of in vitro synthesis of β-lactam antibiotics and their precursors.

All publications and patent applications cited in this specification are herein incorporated by reference as if each individual publication or patent application were specifically and individually indicated to be incorporated by reference.

### Sequence Listing No. 1

SEQ. No.: 1
SEQUENCE TYPE : Nucleotide with corresponding protein
SEQUENCE LENGTH: 12364 base pairs
STRANDEDNESS: single
TOPOLOGY: linear
MOLECULE TYPE: genomic
ORGANISM: Penicillium chrysogenum
FEATURES: from 264 to 11600 bp peptide
PROPERTIES: gene encoding ACV Synthetase

GTCGACAGTT GACAGAGCCA ATGGCATCGG ATCTGCCCTA GACCGTGCTA GACGAAAGTC 60
TCCATCTTGT CTGCGGGCAG TTCTTCAGTC GCCCAGATTC TCGATGGAGA TTGGCCAGGT 120
CAGCCATATA TACCCTGCAA TGGCAGACCA ATGCAGCAGG CCCAGTATAA GGAATTCCCC 180
TCGAGCTTGT CTGTGATTGC GTTTTTTCTA ACACTTGTTG TTGCATCCGA TCCGTCGCTA 240
CCAATTATTG GTCATTGACA GAC ATG ACT CAA CTG AAG CCA CCG AAC GGA ACC 293

Met Thr Gln Leu Lys Pro Pro Asn Gly Thr
1               5                   10

ACG CCG ATA GGC TTC TCG GCC ACT ACA TCC CTG AAC GCC AGT GGG AGC 341
Thr Pro Ile Gly Phe Ser Ala Thr Thr Ser Leu Asn Ala Ser Gly Ser
                15                  20                  25

TCG AGT GTG AAA AAT GGG ACC ATC AAA CCC AGC AAT GGC ATC TTC AAG 389
Ser Ser Val Lys Asn Gly Thr Ile Lys Pro Ser Asn Gly Ile Phe Lys
            30                  35                  40

CCC AGC ACT AGG GAC ACC ATG GAC CCT TGC AGT GGG AAT GCG GCC GAT 437
Pro Ser Thr Arg Asp Thr Met Asp Pro Cys Ser Gly Asn Ala Ala Asp
        45                  50                  55

GGC AGT ATC CGC GTC CGT TTC CGT GGA GGA ATC GAA CGG TGG AAG GAG 485
Gly Ser Ile Arg Val Arg Phe Arg Gly Gly Ile Glu Arg Trp Lys Glu
    60                  65                  70

TGC GTC AAC CAG GTC CCC GAG CGC TGC GAC CTG AGT GGT CTG ACA ACC 533
Cys Val Asn Gln Val Pro Glu Arg Cys Asp Leu Ser Gly Leu Thr Thr
75                  80                  85                  90

GAC TCC ACG CGA TAT CAG CTC GCA TCG ACT GGG TTC GGT GAC GCG AGC 581
Asp Ser Thr Arg Tyr Gln Leu Ala Ser Thr Gly Phe Gly Asp Ala Ser
                95                  100                 105

GCT GCG TAC CAG GAG CGC TTG ATG ACG GTC CCT GTT GAC GTA CAT GCC 629
Ala Ala Tyr Gln Glu Arg Leu Met Thr Val Pro Val Asp Val His Ala
            110                 115                 120

GCG CTC CAA GAG CTG TGC CTA GAA CGC CGT GTG AGC GTG GGA TCC GTC 677
Ala Leu Gln Glu Leu Cys Leu Glu Arg Arg Val Ser Val Gly Ser Val
        125                 130                 135

ATT AAT TTC TCC GTG CAC CAG ATG CTG AAA GGG TTT GGA AAT GGC ACA 725
Ile Asn Phe Ser Val His Gln Met Leu Lys Gly Phe Gly Asn Gly Thr
    140                 145                 150

CAC ACT ATC ACC GCC TCT CTG CAC CGT GAG CAG AAT TTG CAG AAT TCT 773
His Thr Ile Thr Ala Ser Leu His Arg Glu Gln Asn Leu Gln Asn Ser
155                 160                 165                 170

TCG CCA TCC TGG GTA GTC TCC CCC ACA ATC GTC ACC CAT GAG AAC AGA 821
Ser Pro Ser Trp Val Val Ser Pro Thr Ile Val Thr His Glu Asn Arg
                175                 180                 185

GAC GGA TGG TCC GTC GCG CAG GCG GTC GAG AGT ATC GAA GCG GCG CGC 869
Asp Gly Trp Ser Val Ala Gln Ala Val Glu Ser Ile Glu Ala Ala Arg
            190                 195                 200

GGT TCC GAG AAG GAG TCA GTG ACT GCG ATT GAC TCC GCG TCA AGT CTC 917
Gly Ser Glu Lys Glu Ser Val Thr Ala Ile Asp Ser Ala Ser Ser Leu
        205                 210                 215

GTG AAA ATG GGG TTA TTT GAC TTA CTC GTC AGC TTT GTC GAT GCA GAC 965
Val Lys Met Gly Leu Phe Asp Leu Leu Val Ser Phe Val Asp Ala Asp
    220                 225                 230

GAT GCT CGT ATT CCA TGT TTC GAC TTT CCC CTC GCA GTG ATA GTG CGT 1013
Asp Ala Arg Ile Pro Cys Phe Asp Phe Pro Leu Ala Val Ile Val Arg
235                 240                 245                 250

GAG TGT GAT GCC AAC CTC TCG CTG ACT CTG CGT TTC TCC GAC TGT CTC 1061
Glu Cys Asp Ala Asn Leu Ser Leu Thr Leu Arg Phe Ser Asp Cys Leu
                255                 260                 265

TTC AAC GAG GAG ACG ATA TGC AAT TTT ACC GAT GCC CTA AAC ATC TTG 1109
Phe Asn Glu Glu Thr Ile Cys Asn Phe Thr Asp Ala Leu Asn Ile Leu
            270                 275                 280

CTC GCC GAA GCA GTG ATA GGA AGA GTG ACC CCG GTT GCC GAT ATC GAA 1157
Leu Ala Glu Ala Val Ile Gly Arg Val Thr Pro Val Ala Asp Ile Glu
        285                 290                 295

CTA CTA TCC GCG GAG CAG AAG CAG CAG CTG GAA GAG TGG AAC AAC ACG 1205
Leu Leu Ser Ala Glu Gln Lys Gln Gln Leu Glu Glu Trp Asn Asn Thr
    300                 305                 310

GAT GGC GAG TAC CCT TCA TCA AAG CGA CTG CAC CAT CTC ATT GAA GAG 1253
Asp Gly Glu Tyr Pro Ser Ser Lys Arg Leu His His Leu Ile Glu Glu
315                 320                 325                 330

GTG GTT GAA CGG CAT GAA GAC AAA ATA GCC GTT GTC TGC GAC GAG CGA 1301
Val Val Glu Arg His Glu Asp Lys Ile Ala Val Val Cys Asp Glu Arg
                335                 340                 345

GAG CTC ACT TAC GGC GAG CTC AAT GCC CAA GGC AAC AGC CTC GCA CGC 1349
Glu Leu Thr Tyr Gly Glu Leu Asn Ala Gln Gly Asn Ser Leu Ala Arg
            350                 355                 360

TAT CTC CGT TCC ATT GGT ATC CTG CCC GAG CAG CTA GTC GCA TTG TTT 1397
Tyr Leu Arg Ser Ile Gly Ile Leu Pro Glu Gln Leu Val Ala Leu Phe
        365                 370                 375

CTA GAT AAG AGC GAG AAG CTC ATT GTT ACC ATC CTC GGC GTG TGG AAA 1445
Leu Asp Lys Ser Glu Lys Leu Ile Val Thr Ile Leu Gly Val Trp Lys
    380                 385                 390

TCC GGC GCC GCC TAC GTG CCC ATC GAC CCG ACT TAT CCG GAT GAG CGA 1493
Ser Gly Ala Ala Tyr Val Pro Ile Asp Pro Thr Tyr Pro Asp Glu Arg
395                 400                 405                 410

GTG CGC TTC GTG CTG GAT GAC ACC AAG GCA CGG GCC ATC ATC GCC AGT 1541
Val Arg Phe Val Leu Asp Asp Thr Lys Ala Arg Ala Ile Ile Ala Ser
                415                 420                 425

AAT CAA CAT GTG GAG AGG CTC CAG CGA GAG GTC ATC GGC GAT AGA AAC 1589
Asn Gln His Val Glu Arg Leu Gln Arg Glu Val Ile Gly Asp Arg Asn
            430                 435                 440

CTA TGC ATT ATC CGT CTG GAG CCC TTG TTG GCC TCC CTT GCT CAG GAT 1637
Leu Cys Ile Ile Arg Leu Glu Pro Leu Leu Ala Ser Leu Ala Gln Asp
        445                 450                 455

TCC TCA AAA TTC CCC GCG CAT AAC TTG GAC GAC CTA CCC CTC ACA AGC 1685
Ser Ser Lys Phe Pro Ala His Asn Leu Asp Asp Leu Pro Leu Thr Ser
    460                 465                 470

CAG CAG CTC GCC TAT GTG ACT TAC ACC TCT GGG ACC ACT GGT TTC CCA 1733
Gln Gln Leu Ala Tyr Val Thr Tyr Thr Ser Gly Thr Thr Gly Phe Pro
475                 480                 485                 490

AAG GGC ATA TTT AAA CAA CAC ACC AAT GTG GTG AAC AGT ATT ACC GAC 1781
Lys Gly Ile Phe Lys Gln His Thr Asn Val Val Asn Ser Ile Thr Asp
                495                 500                 505

CTG TCT GCA AGG TAC GGG GTG GCC GGG CAG CAC CAC GAA GCC ATT CTG 1829
Leu Ser Ala Arg Tyr Gly Val Ala Gly Gln His His Glu Ala Ile Leu
            510                 515                 520

CTT TTC TCG GCC TGC GTG TTC GAG CCG TTC GTT CGA CAG ACG CTC ATG 1877
Leu Phe Ser Ala Cys Val Phe Glu Pro Phe Val Arg Gln Thr Leu Met
        525                 530                 535

GCA CTC GTG AAT GGC CAT CTC CTC GCA GTT ATC AAT GAC GTG GAA AAA 1925
Ala Leu Val Asn Gly His Leu Leu Ala Val Ile Asn Asp Val Glu Lys
    540                 545                 550

TAT GAT GCC GAT ACG CTC CTG CCG TTC ATA CGC AGA CAC AGC ATC ACC 1973
Tyr Asp Ala Asp Thr Leu Leu Pro Phe Ile Arg Arg His Ser Ile Thr
555                 560                 565                 570

TAC CTC AAT GGT ACT GCC TCT GTC TTG CAA GAG TAC GAC TTT TCC GAC 2021
Tyr Leu Asn Gly Thr Ala Ser Val Leu Gln Glu Tyr Asp Phe Ser Asp
                575                 580                 585

TGC CCA TCA CTG AAT CGG ATA ATC CTG GTG GGT GAG AAC CTG ACA GAA 2069
Cys Pro Ser Leu Asn Arg Ile Ile Leu Val Gly Glu Asn Leu Thr Glu
            590                 595                 600

GCC CGG TAT CTG GCG CTG CGC CAG CGG TTC AAG AAT CGC ATC CTC AAC 2117
Ala Arg Tyr Leu Ala Leu Arg Gln Arg Phe Lys Asn Arg Ile Leu Asn
        605                 610                 615

GAG TAT GGT TTT ACC GAG TCA GCC TTT GTA ACG GCC CTC AAG ATT TTC 2165
Glu Tyr Gly Phe Thr Glu Ser Ala Phe Val Thr Ala Leu Lys Ile Phe
    620                 625                 630

GAC CCG GAG TCG ACC CGT AAG GAC ACG AGT CTG GGG AGA CCG GTG CGC 2213
Asp Pro Glu Ser Thr Arg Lys Asp Thr Ser Leu Gly Arg Pro Val Arg
635                 640                 645                 650

AAC GTC AAG TGC TAC ATC CTC AAT CCA TCC CTT AAA CGT GTC CCG ATT 2261
Asn Val Lys Cys Tyr Ile Leu Asn Pro Ser Leu Lys Arg Val Pro Ile
                655                 660                 665

GGA GCT ACG GGT GAG TTG CAT ATC GGA GGG TTG GGC ATT TCC AAG GGA 2309
Gly Ala Thr Gly Glu Leu His Ile Gly Gly Leu Gly Ile Ser Lys Gly
            670                 675                 680

TAC CTC AAC CGC CCC GAA CTC ACG CCG CAC CGC TTC ATT CCC AAC CCC 2357
Tyr Leu Asn Arg Pro Glu Leu Thr Pro His Arg Phe Ile Pro Asn Pro
        685                 690                 695

TTC CAA ACG GAT TGC GAG AAG CAG CTC GGG ATC AAC AGC TTG ATG TAC 2405
Phe Gln Thr Asp Cys Glu Lys Gln Leu Gly Ile Asn Ser Leu Met Tyr
    700                 705                 710

AAG ACC GGT GAC CTG GCC CGC TGG CTT CCG AAC GGC GAG GTT GAG TAT 2453
Lys Thr Gly Asp Leu Ala Arg Trp Leu Pro Asn Gly Glu Val Glu Tyr
715                 720                 725                 730

CTC GGA CGC GCA GAT TTC CAG ATC AAA CTG CGA GGT ATT CGA ATT GAA 2501
Leu Gly Arg Ala Asp Phe Gln Ile Lys Leu Arg Gly Ile Arg Ile Glu
                735                 740                 745

CCT GGT GAA ATT GAG ACG ATG CTG GCT ATG TAC CCT AGG GTC CGG ACC 2549
Pro Gly Glu Ile Glu Thr Met Leu Ala Met Tyr Pro Arg Val Arg Thr
            750                 755                 760

AGT TTA GTG GTG TCC AAA AAG CTC CGC AAC GGT CCA GAG GAA ACT ACC 2597
Ser Leu Val Val Ser Lys Lys Leu Arg Asn Gly Pro Glu Glu Thr Thr
        765                 770                 775

AAC GAG CAC CTC GTG GGT TAT TAT GTT TGT GAT AGC GCC TCA GTG TCC 2645
Asn Glu His Leu Val Gly Tyr Tyr Val Cys Asp Ser Ala Ser Val Ser
    780                 785                 790

GAG GCA GAC CTG CTG TCA TTT TTA GAG AAG AAA CTG CCT CGA TAC ATG 2693
Glu Ala Asp Leu Leu Ser Phe Leu Glu Lys Lys Leu Pro Arg Tyr Met
795                 800                 805                 810

ATT CCC ACG CGG TTG GTA CAG CTG TCG CAG ATC CCA GTG AAT GTG AAC 2741
Ile Pro Thr Arg Leu Val Gln Leu Ser Gln Ile Pro Val Asn Val Asn
                815                 820                 825

GGG AAG GCG GAC CTA CGC GCC TTG CCG GCC GTC GAT ATC TCC AAT TCC 2789
Gly Lys Ala Asp Leu Arg Ala Leu Pro Ala Val Asp Ile Ser Asn Ser
            830                 835                 840

ACG GAG GTG CGT TCC GAC CTT CGA GGC GAT ACG GAA ATC GCC CTC GGG 2837
Thr Glu Val Arg Ser Asp Leu Arg Gly Asp Thr Glu Ile Ala Leu Gly
        845                 850                 855

GAA ATC TGG GCC GAC GTG TTG GGA GCC CGC CAG AGA TCC GTC TCT CGC 2885
Glu Ile Trp Ala Asp Val Leu Gly Ala Arg Gln Arg Ser Val Ser Arg
    860                 865                 870

AAC GAC AAC TTC TTC CGC CTA GGA GGG CAC AGC ATC ACC TGC ATC CAA 2933
Asn Asp Asn Phe Phe Arg Leu Gly Gly His Ser Ile Thr Cys Ile Gln
875                 880                 885                 890

CTG ATC GCT CGC ATC CGA CAA CGA CAA CGA CTC TCG GTC AGC ATC TCC 2981
Leu Ile Ala Arg Ile Arg Gln Arg Gln Arg Leu Ser Val Ser Ile Ser
                895                 900                 905

GTC GAA GAT GTT TTT GCA ACA AGG ACA CTT GAG CGC ATG GCA GAC CTT 3029
Val Glu Asp Val Phe Ala Thr Arg Thr Leu Glu Arg Met Ala Asp Leu
            910                 915                 920

CTA CAG AAC AAG CAG CAG GAG AAA TGC GAC AAA CCC CAT GAG GCG CCG 3077
Leu Gln Asn Lys Gln Gln Glu Lys Cys Asp Lys Pro His Glu Ala Pro
        925                 930                 935

ACA GAG CTG CTT GAG GAG AAT GCA GCA ACG GAC AAT ATC TAT CTG GCA 3125
Thr Glu Leu Leu Glu Glu Asn Ala Ala Thr Asp Asn Ile Tyr Leu Ala
    940                 945                 950

AAC AGT CTT CAG CAG GGC TTC GTC TAC CAT TAC CTC AAG AGC ATG GAA 3173
Asn Ser Leu Gln Gln Gly Phe Val Tyr His Tyr Leu Lys Ser Met Glu
955                 960                 965                 970

CAA TCC GAC GCC TAT GTA ATG CAG TCC GTT CTT CGG TAC AAC ACC ACA 3221
Gln Ser Asp Ala Tyr Val Met Gln Ser Val Leu Arg Tyr Asn Thr Thr
                975                 980                 985

TTG TCT CCA GAT CTG TTT CAG AGA GCC TGG AAG CAT GCA CAG CAG TCC 3269
Leu Ser Pro Asp Leu Phe Gln Arg Ala Trp Lys His Ala Gln Gln Ser
            990                 995                 1000

TTT CCA GCG CTG CGG CTG CGG TTC TCA TGG GAA AAG GAG GTT TTC CAA 3317
Phe Pro Ala Leu Arg Leu Arg Phe Ser Trp Glu Lys Glu Val Phe Gln
        1005                1010                1015

CTG CTC GAT CAG GAT CCA CCA TTG GAC TGG CGT TTC CTC TAC TTC ACC 3365
Leu Leu Asp Gln Asp Pro Pro Leu Asp Trp Arg Phe Leu Tyr Phe Thr
    1020                1025                1030

GAC GTT GCC GCG GGT GCT GTC GAG GAC CGG AAA TTG GAA GAC TTG CGG 3413
Asp Val Ala Ala Gly Ala Val Glu Asp Arg Lys Leu Glu Asp Leu Arg
1035                1040                1045                1050

CGC CAA GAC CTT ACG GAG AGA TTC AAG CTG GAT GTT GGC AGA CTG TTC 3461
Arg Gln Asp Leu Thr Glu Arg Phe Lys Leu Asp Val Gly Arg Leu Phe
                1055                1060                1065

CGC GTC TAT CTG ATT AAA CAC AGC GAG AAT CGC TTC ACG TGT CTT TTC 3509
Arg Val Tyr Leu Ile Lys His Ser Glu Asn Arg Phe Thr Cys Leu Phe
            1070                1075                1080

AGC TGC CAG CAT GCA ATC CTC GAT GGT TGG AGT CTG CCA CTC TTG TTC 3557
Ser Cys Gln His Ala Ile Leu Asp Gly Trp Ser Leu Pro Leu Leu Phe
        1085                1090                1095

GAA AAG GTT CAC GAG ACC TAC CTG CAA CTG CTG CAT GGG GAC AAT CTC 3605
Glu Lys Val His Glu Thr Tyr Leu Gln Leu Leu His Gly Asp Asn Leu
    1100                1105                1110

ACT TCG TCC ATG GAT GAC CCT TAC ACT CGC ACC CAG CGG TAT CTC CAC 3653
Thr Ser Ser Met Asp Asp Pro Tyr Thr Arg Thr Gln Arg Tyr Leu His
1115                1120                1125                1130

GCT CAC CGT GAG GAT CAC CTC GAC TTT TGG GCC GGT GTG GTT CAA AAG 3701
Ala His Arg Glu Asp His Leu Asp Phe Trp Ala Gly Val Val Gln Lys
                1135                1140                1145

ATC AAC GAA CGG TGT GAT ATG AAC GCC TTG TTG AAC GAG CGC AGT CGT 3749
Ile Asn Glu Arg Cys Asp Met Asn Ala Leu Leu Asn Glu Arg Ser Arg
            1150                1155                1160

TAC AAA GTC CAG CTG GCA GAC TAT GAC CAG GTG CAG GAG CAG CGA CAC 3797
Tyr Lys Val Gln Leu Ala Asp Tyr Asp Gln Val Gln Glu Gln Arg His
        1165                1170                1175

GTG ACA ATT GCT CTC TCT GGA GAC GCA TGG CTA GCA GAC CTT CGT CAG 3845
Val Thr Ile Ala Leu Ser Gly Asp Ala Trp Leu Ala Asp Leu Arg Gln
    1180                1185                1190

ACC TGC TCC GCC CAG GGT ATT ACC TTA CAT TCG ATT CTC CAA TTT GTT 3893
Thr Cys Ser Ala Gln Gly Ile Thr Leu His Ser Ile Leu Gln Phe Val
1195                1200                1205                1210

TGG CAC GCC GTG CTG CAC GCT TAT GGC GGT GGC ACC CAC ACC ATA ACC 3941
Trp His Ala Val Leu His Ala Tyr Gly Gly Gly Thr His Thr Ile Thr
                1215                1220                1225

GGC ACG ACC ATT TCT GGA AGG AAC CTG CCC ATC TTG GGA ATT GAA CGA 3989
Gly Thr Thr Ile Ser Gly Arg Asn Leu Pro Ile Leu Gly Ile Glu Arg
            1230                1235                1240

GCA GTT GGT CCG TAT ATC AAC ACT CTA CCG CTG GTA CTC GAT CAT TCG 4037
Ala Val Gly Pro Tyr Ile Asn Thr Leu Pro Leu Val Leu Asp His Ser
        1245                1250                1255

ACG TTC AAG GAT AAG ACA ATC ATG GAG GCC ATC GAG GAT GTG CAG GCC 4085
Thr Phe Lys Asp Lys Thr Ile Met Glu Ala Ile Glu Asp Val Gln Ala
    1260                1265                1270

AAG GTA AAC GTC ATG AAC AGC CGG GGC AAT GTG GAA CTG GGC CGT TTG 4133
Lys Val Asn Val Met Asn Ser Arg Gly Asn Val Glu Leu Gly Arg Leu
1275                1280                1285                1290

CAC AAA ACC GAC TTA AAG CAC GGA TTA TTC GAT TCT TTA TTC GTG CTT 4181
His Lys Thr Asp Leu Lys His Gly Leu Phe Asp Ser Leu Phe Val Leu
                1295                1300                1305

GAA AAC TAC CCG AAT TTG GAC AAA TCG CGA ACA CTT GAG CAC CAG ACT 4229
Glu Asn Tyr Pro Asn Leu Asp Lys Ser Arg Thr Leu Glu His Gln Thr
            1310                1315                1320

GAA CTG GGG TAT TCG ATT GAA GGC GGC ACT GAG AAG CTG AAT TAT CCA 4277
Glu Leu Gly Tyr Ser Ile Glu Gly Gly Thr Glu Lys Leu Asn Tyr Pro
        1325                1330                1335

CTG GCT GTC ATC GCG CGC GAA GTC GAG ACG ACT GGC GGA TTC ACA GTA 4325
Leu Ala Val Ile Ala Arg Glu Val Glu Thr Thr Gly Gly Phe Thr Val
    1340                1345                1350

TCC ATC TGC TAC GCC AGT GAG CTA TTT GAG GAG GTT ATG ATC TCC GAG 4373
Ser Ile Cys Tyr Ala Ser Glu Leu Phe Glu Glu Val Met Ile Ser Glu
1355                1360                1365                1370

CTT CTT CAT ATG GTC CAG GAC ACA CTG ATG CAG GTT GCC CGA GGT TTG 4421
Leu Leu His Met Val Gln Asp Thr Leu Met Gln Val Ala Arg Gly Leu
                1375                1380                1385

AAT GAA CCC GTC GGC AGC CTG GAG TAT CTC TCA TCT ATC CAA TTG GAG 4469
Asn Glu Pro Val Gly Ser Leu Glu Tyr Leu Ser Ser Ile Gln Leu Glu
            1390                1395                1400

CAA CTC GCC GCG TGG AAT GCC ACG GAA GCT GAG TTT CCC GAT ACC ACG 4517
Gln Leu Ala Ala Trp Asn Ala Thr Glu Ala Glu Phe Pro Asp Thr Thr
        1405                1410                1415

CTT CAT GAG ATG TTT GAA AAC GAA GCG AGC CAG AAG CCG GAC AAG ATA 4565
Leu His Glu Met Phe Glu Asn Glu Ala Ser Gln Lys Pro Asp Lys Ile
    1420                1425                1430

GCA GTG GTC TAT GAG GAG ACG TCC TTG ACT TAC CGC GAG TTG AAT GAG 4613
Ala Val Val Tyr Glu Glu Thr Ser Leu Thr Tyr Arg Glu Leu Asn Glu
1435 1140 1445 1450

CGG GCG AAC CGT ATG GCA CAT CAG CTA AGG TCC GAC GTC AGC CCC AAC 4661
Arg Ala Asn Arg Met Ala His Gln Leu Arg Ser Asp Val Ser Pro Asn
                1455                1460                1465

CCC AAC GAG GTC ATT GCG CTG GTG ATG GAC AAG AGC GAG CAT ATG ATC 4709
Pro Asn Glu Val Ile Ala Leu Val Met Asp Lys Ser Glu His Met Ile
            1470                1475                1480

GTC AAC ATT CTG GCC GTA TGG AAG AGC GGC GGT GCC TAT GTC CCC ATT 4757
Val Asn Ile Leu Ala Val Trp Lys Ser Gly Gly Ala Tyr Val Pro Ile
        1485                1490                1495

GAC CCT GGA TAT CCT AAC GAC CGC ATT CAA TAT ATC CTA GAG GAC ACA 4805
Asp Pro Gly Tyr Pro Asn Asp Arg Ile Gln Tyr Ile Leu Glu Asp Thr
    1500                1505                1510

CAA GCC CTC GCA GTC ATC GCG GAC TCC TGC TAT CTG CCT CGC ATC AAG 4853
Gln Ala Leu Ala Val Ile Ala Asp Ser Cys Tyr Leu Pro Arg Ile Lys
1515                1520                1525                1530

GGA ATG GCT GCC TCC GGC ACG CTT CTT TAT CCC TCT GTC TTG CCT GCC 4901
Gly Met Ala Ala Ser Gly Thr Leu Leu Tyr Pro Ser Val Leu Pro Ala
                1535                1540                1545

AAT CCG GAT TCC AAG TGG AGC GTA TCG AAC CCT TCA CCG TTG AGT CGG 4949
Asn Pro Asp Ser Lys Trp Ser Val Ser Asn Pro Ser Pro Leu Ser Arg
            1550                1555                1560

AGC ACG GAC TTA GCT TAT ATC ATC TAT ACC TCT GGA ACG ACA GGT CGG 4997
Ser Thr Asp Leu Ala Tyr Ile Ile Tyr Thr Ser Gly Thr Thr Gly Arg
        1565                1570                1575

CCC AAG GGC GTC ACG GTA GAG CAT CAT GGA GTG GTC AAC CTG CAG GTG 5045
Pro Lys Gly Val Thr Val Glu His His Gly Val Val Asn Leu Gln Val
    1580                1585                1590

TCG CTA TCC AAA GTA TTC GGA CTA CGG GAT ACG GAC GAC GAG GTA ATT 5093
Ser Leu Ser Lys Val Phe Gly Leu Arg Asp Thr Asp Asp Glu Val Ile
1595                1600                1605                1610

CTC TCC TTT TCC AAC TAT GTG TTC GAC CAT TTC GTG GAG CAG ATG ACC 5141
Leu Ser Phe Ser Asn Tyr Val Phe Asp His Phe Val Glu Gln Met Thr
                1615                1620                1625

GAC GCC ATT CTC AAT GGC CAA ACC CTC CTG GTC CTC AAC GAT GGA ATG 5189
Asp Ala Ile Leu Asn Gly Gln Thr Leu Leu Val Leu Asn Asp Gly Met
            1630                1635                1640

CGC GGG GAC AAA GAG CGA CTC TAC AGA TAC ATT GAG AAG AAC CGA GTG 5237
Arg Gly Asp Lys Glu Arg Leu Tyr Arg Tyr Ile Glu Lys Asn Arg Val
        1645                1650                1655

ACC TAC TTG TCT GGC ACC CCA TCC GTG GTC TCC ATG TAC GAA TTT AGC 5285
Thr Tyr Leu Ser Gly Thr Pro Ser Val Val Ser Met Tyr Glu Phe Ser
    1660                1665                1670

CGG TTC AAG GAC CAT CTA CGC CGT GTG GAC TGC GTG GGG GAG GCG TTC 5333
Arg Phe Lys Asp His Leu Arg Arg Val Asp Cys Val Gly Glu Ala Phe
1675                1680                1685                1690

AGC GAA CCG GTC TTC GAC AAG ATC CGC GAA ACG TTC CAT GGC CTC GTT 5381
Ser Glu Pro Val Phe Asp Lys Ile Arg Glu Thr Phe His Gly Leu Val
                1695                1700                1705

ATC AAC GGC TAC GGC CCA ACT GAA GTT TCC ATC ACC ACC CAC AAG CGG 5429
Ile Asn Gly Tyr Gly Pro Thr Glu Val Ser Ile Thr Thr His Lys Arg
            1710                1715                1720

CTC TAT CCA TTC CCA GAG CGG CGA ATG GAC AAA AGT ATT GGC CAA CAG 5477
Leu Tyr Pro Phe Pro Glu Arg Arg Met Asp Lys Ser Ile Gly Gln Gln
        1725                1730                1735

GTC CAC AAT AGC ACG AGC TAT GTG CTG AAC GAG GAC ATG AAG CGC ACC 5525
Val His Asn Ser Thr Ser Tyr Val Leu Asn Glu Asp Met Lys Arg Thr
    1740                1745                1750

CCC ATA GGG GCT GTC GGC GAG CTC TAC CTG GGT GGT GAA GGA GTG GTA 5573
Pro Ile Gly Ala Val Gly Glu Leu Tyr Leu Gly Gly Glu Gly Val Val
1755                1760                1765                1770

CGG GGA TAT CAC AAT CGC GCA GAT GTG ACC GCG GAG CGT TTT ATT CCT 5621
Arg Gly Tyr His Asn Arg Ala Asp Val Thr Ala Glu Arg Phe Ile Pro
                1775                1780                1785

AAT CCA TTC CAG TCG GAA GAA GAT AAG CGA GAA GGT CGT AAC TCC CGT 5669
Asn Pro Phe Gln Ser Glu Glu Asp Lys Arg Glu Gly Arg Asn Ser Arg
            1790                1795                1800

TTG TAC AAG ACC GGT GAC CTG GTA CGC TGG ATT CCT GGA AGC AGC GGG 5717
Leu Tyr Lys Thr Gly Asp Leu Val Arg Trp Ile Pro Gly Ser Ser Gly
        1805                1810                1815

GAG GTC GAG TAT CTA GGT CGT AAT GAC TTC CAG GTC AAG ATT CGC GGA 5765
Glu Val Glu Tyr Leu Gly Arg Asn Asp Phe Gln Val Lys Ile Arg Gly
    1820                1825                1830

CTG CGC ATC GAA GTA GGC GAG ATT GAG GCC ATC CTA TCG TCT TAT CAC 5813
Leu Arg Ile Glu Val Gly Glu Ile Glu Ala Ile Leu Ser Ser Tyr His
1835                1840                1845                1850

GGA ATC AAA CAG TCT GTG GTG ATT GCC AAG GAT TGC AGA GAA GGG GCC 5861
Gly Ile Lys Gln Ser Val Val Ile Ala Lys Asp Cys Arg Glu Gly Ala
                1855                1860                1865

CAG AAA TTC CTG GTT GGT TAC TAT GTC GCC GAT GCA GCG CTG CCG TCC 5909
Gln Lys Phe Leu Val Gly Tyr Tyr Val Ala Asp Ala Ala Leu Pro Ser
            1870                1875                1880

GCT GCC ATT CGG CGC TTC ATG CAG TCT CGG CTC CCT GGC TAC ATG GTG 5957
Ala Ala Ile Arg Arg Phe Met Gln Ser Arg Leu Pro Gly Tyr Met Val
        1885                1890                1895

CCC TCT CGT CTC ATT CTC GTC AGC AAG TTC CCC GTC ACT CCT AGT GGA 6005
Pro Ser Arg Leu Ile Leu Val Ser Lys Phe Pro Val Thr Pro Ser Gly
    1900                1905                1910

AAA TTA GAC ACC AAG GCT TTG CCC CCA GCC GAG GAA GAG AGC GAG ATT 6053
Lys Leu Asp Thr Lys Ala Leu Pro Pro Ala Glu Glu Glu Ser Glu Ile
1915                1920                1925                1930

GAC GTG GTG CCG CCG CGT AGT GAA ATC GAA CGC TCC TTG TGT GAC ATC 6101
Asp Val Val Pro Pro Arg Ser Glu Ile Glu Arg Ser Leu Cys Asp Ile
                1935                1940                1945

TGG GCG GAA CTA CTC GAG ATG CAC CCA GAG GAG ATC GGC ATT TAC AGC 6149
Trp Ala Glu Leu Leu Glu Met His Pro Glu Glu Ile Gly Ile Tyr Ser
            1950                1955                1960

GAT TTC TTC AGC CTG GGA GGT GAC AGC CTA AAG AGC ACA AAG CTT TCC 6197
Asp Phe Phe Ser Leu Gly Gly Asp Ser Leu Lys Ser Thr Lys Leu Ser
        1965                1970                1975

TTC ATG ATT CAC GAG TCC TTT AAC CGC GCC GTC TCA GTC AGC GCC CTT 6245
Phe Met Ile His Glu Ser Phe Asn Arg Ala Val Ser Val Ser Ala Leu
    1980                1985                1990

TTC TGT CAC CGG ACA GTT GAA GCC CAG ACG CAC TTG ATC CTG AAC GAT 6293
Phe Cys His Arg Thr Val Glu Ala Gln Thr His Leu Ile Leu Asn Asp
1995                2000                2005                2010

GCT GCA GAT GTG CAC GAA ATT ACT CCC ATA GAT TGC AAT GAT ACG CAG 6341
Ala Ala Asp Val His Glu Ile Thr Pro Ile Asp Cys Asn Asp Thr Gln
                2015                2020                2025

ATG ATT CCC GTG TCC CGT GCC CAG GAG CGA CTC CTC TTC ATC CAC GAA 6389
Met Ile Pro Val Ser Arg Ala Gln Glu Arg Leu Leu Phe Ile His Glu
            2030                2035                2040

TTT GAG AAT GGC AGC AAT GCA TAC AAT ATC GAC GCT GCA TTT GAA CTG 6437
Phe Glu Asn Gly Ser Asn Ala Tyr Asn Ile Asp Ala Ala Phe Glu Leu
        2045                2050                2055

CCT GGC TCG GTT GAC GCG TCG CTT CTC GAG CAG GCG CTG CGT GGA AAC 6485
Pro Gly Ser Val Asp Ala Ser Leu Leu Glu Gln Ala Leu Arg Gly Asn
    2060                2065                2070

CTT GCT CGA CAT GAG GCG TTG AGA ACT TTA CTG GTC AAG GAT CAC GCA 6533
Leu Ala Arg His Glu Ala Leu Arg Thr Leu Leu Val Lys Asp His Ala
2075                2080                2085                2090

ACC GGC ATC TAT CTT CAG AAG GTA TTG AGT CCC GAT GAA GCC CAG GGC 6581
Thr Gly Ile Tyr Leu Gln Lys Val Leu Ser Pro Asp Glu Ala Gln Gly
                2095                2100                2105

ATG TTC TCC GTC AAC GTG GAC ACA GCC AAG CAG GTG GAG CGG CTG GAC 6629
Met Phe Ser Val Asn Val Asp Thr Ala Lys Gln Val Glu Arg Leu Asp
            2110                2115                2120

CAG GAG ATA GCC AGT CTA TCC CAG CAT GTT TTC CGC CTC GAT GAT GAA 6677
Gln Glu Ile Ala Ser Leu Ser Gln His Val Phe Arg Leu Asp Asp Glu
        2125                2130                2135

CTG CCT TGG GAG GCC CGC ATC CTT AAA CTC GAA TCC GGC GGC CTG TAT 6725
Leu Pro Trp Glu Ala Arg Ile Leu Lys Leu Glu Ser Gly Gly Leu Tyr
    2140                2145                2150

CTC ATT CTG GCG TTC CAC CAT ACC TGC TTC GAT GCA TGG TCA TTG AAA 6773
Leu Ile Leu Ala Phe His His Thr Cys Phe Asp Ala Trp Ser Leu Lys
2155                2160                2165                2170

GTC TTC GAG CAA GAG CTT CGG GCC TTG TAC GCA GCG CTC CAG AAA ACC     6821
Val Phe Glu Gln Glu Leu Arg Ala Leu Tyr Ala Ala Leu Gln Lys Thr
            2175                2180                2185

AAA AGT GCA GCG AAC TTA CCA GCC CTC AAA GCG CAG TAC AAG GAA TAC     6869
Lys Ser Ala Ala Asn Leu Pro Ala Leu Lys Ala Gln Tyr Lys Glu Tyr
        2190                2195                2200

GCG CTC TAC CAT CGC CGG CAG CTG TCT GGC GAT CGC ATG CGC AAC CTG     6917
Ala Leu Tyr His Arg Arg Gln Leu Ser Gly Asp Arg Met Arg Asn Leu
    2205                2210                2215

TCA GAC TTT TGG CTG CGG AAA CTC ATT GGC TTG GAA CCA TTG CAG CTG     6965
Ser Asp Phe Trp Leu Arg Lys Leu Ile Gly Leu Glu Pro Leu Gln Leu
2220                2225                2230

ATC ACG GAC CGC CCA CGT CCT GTG CAA TTC AAA TAC GAC GGT GAC GAC     7013
Ile Thr Asp Arg Pro Arg Pro Val Gln Phe Lys Tyr Asp Gly Asp Asp
2235                2240                2245                2250

CTC AGT ATC GAA CTG AGC AAG AAG GAA ACG GAG AAC CTG AGG GGG GTG     7061
Leu Ser Ile Glu Leu Ser Lys Lys Glu Thr Glu Asn Leu Arg Gly Val
            2255                2260                2265

GCC AAA CGT TGC AAG TCG AGT CTG TAC GTC GTG TTG GTT TCC GTT TAT     7109
Ala Lys Arg Cys Lys Ser Ser Leu Tyr Val Val Leu Val Ser Val Tyr
        2270                2275                2280

TGC GTT ATG CTA GCC TCG TAC GCG AAC CAG TCC GAT GTT TCC GTG GGT     7157
Cys Val Met Leu Ala Ser Tyr Ala Asn Gln Ser Asp Val Ser Val Gly
    2285                2290                2295

ATC CCA GTC AGC CAC CGA ACG CAT CCT CAG TTC CAA TCG GTC ATT GGA     7205
Ile Pro Val Ser His Arg Thr His Pro Gln Phe Gln Ser Val Ile Gly
2300                2305                2310

TTC TTC GTC AAC CTT GTG GTG CTA AGG GTG GAT ATT TCT CAG TCA GCC     7253
Phe Phe Val Asn Leu Val Val Leu Arg Val Asp Ile Ser Gln Ser Ala
2315                2320                2325                2330

ATT TGC GGG CTC ATC AGA AGG GTA ATG AAA GAG CTC GTG GAC GCC CAA     7301
Ile Cys Gly Leu Ile Arg Arg Val Met Lys Glu Leu Val Asp Ala Gln
            2335                2340                2345

CTG CAC CAA GAC ATG CCG TTC CAG GAA GTG ACG AAG CTG CTG CAG GTG     7349
Leu His Gln Asp Met Pro Phe Gln Glu Val Thr Lys Leu Leu Gln Val
        2350                2355                2360

GAT AAT GAC CCC AGC CGG CAT CCG CTG GTA CAG AAC GTG TTC AAC TTC     7397
Asp Asn Asp Pro Ser Arg His Pro Leu Val Gln Asn Val Phe Asn Phe
    2365                2370                2375

GAA TCC CGT GCG AAC GGA GAA CAC GAT GCC AGG TCG GAG GAT GAA GGA     7445
Glu Ser Arg Ala Asn Gly Glu His Asp Ala Arg Ser Glu Asp Glu Gly
2380                2385                2390

TCG CTT GCA TTC AAT CAA TAC CGG CCG GTT CAG CCC GTG GAT TCC GTT 7493
Ser Leu Ala Phe Asn Gln Tyr Arg Pro Val Gln Pro Val Asp Ser Val
2395                2400                2405                2410

GCG AAG TTC GAT CTG AAC GCA ACG GTC ACG GAA TTG GAG TCG GGA TTG 7541
Ala Lys Phe Asp Leu Asn Ala Thr Val Thr Glu Leu Glu Ser Gly Leu
                2415                2420                2425

AGA GTC AAC TTC AAC TAT GCG ACC AGC CTA TTC AAC AAA AGC ACG ATC 7589
Arg Val Asn Phe Asn Tyr Ala Thr Ser Leu Phe Asn Lys Ser Thr Ile
            2430                2435                2440

CAG GGT TTT TTG CAT ACC TAT GAG TAT CTC CTG CGC CAG CTG TCC GAA 7637
Gln Gly Phe Leu His Thr Tyr Glu Tyr Leu Leu Arg Gln Leu Ser Glu
        2445                2450                2455

CTG AGT GCA GAA GGG ATC AAT GAG GAT ACG CAG CTG TCG TTA GTT CGC 7685
Leu Ser Ala Glu Gly Ile Asn Glu Asp Thr Gln Leu Ser Leu Val Arg
    2460                2465                2470

CCG ACA GAG AAT GGC GAT CTG CAC TTG CCA TTG GCA CAG TCC CCG CTT 7733
Pro Thr Glu Asn Gly Asp Leu His Leu Pro Leu Ala Gln Ser Pro Leu
2475                2480                2485                2490

GCG ACG ACT GCT GAG GAG CAG AAA GTA GCG TCG TTG AAC CAG GCC TTT 7781
Ala Thr Thr Ala Glu Glu Gln Lys Val Ala Ser Leu Asn Gln Ala Phe
                2495                2500                2505

GAG CGC GAA GCT TTC CTT GCC GCA GAG AAG ATT GCC GTC GTG CAG GGA 7829
Glu Arg Glu Ala Phe Leu Ala Ala Glu Lys Ile Ala Val Val Gln Gly
            2510                2515                2520

GAT AGA GCA CTT AGT TAT GCT GAT CTT AAC GGG CAG GCT AAC CAG CTC 7877
Asp Arg Ala Leu Ser Tyr Ala Asp Leu Asn Gly Gln Ala Asn Gln Leu
        2525                2530                2535

GCC CGG TAC ATA CAG TCC GTG TCC TGT ATT GGG GCA GAC GAC GGA ATA 7925
Ala Arg Tyr Ile Gln Ser Val Ser Cys Ile Gly Ala Asp Asp Gly Ile
    2540                2545                2550

GCT TTG ATG CTG GAA AAG AGT ATC GAC ACG ATT ATT TGC ATT CTC GCG 7973
Ala Leu Met Leu Glu Lys Ser Ile Asp Thr Ile Ile Cys Ile Leu Ala
2555                2560                2565                2570

ATT TGG AAG GCT GGT GCA GCA TAC GTG CCC TTG GAT CCG ACT TAC CCA 8021
Ile Trp Lys Ala Gly Ala Ala Tyr Val Pro Leu Asp Pro Thr Tyr Pro
                2575                2580                2585

CCC GGA CGC GTC CAG CTG ATT CTG GAG GAG ATT AAA GCG AAG GCT GTC 8069
Pro Gly Arg Val Gln Leu Ile Leu Glu Glu Ile Lys Ala Lys Ala Val
            2590                2595                2600

CTT GTG CAC TCC AGT CAT GCT TCG AAA TGT GAA CGC CAT GGC GCG AAG 8117
Leu Val His Ser Ser His Ala Ser Lys Cys Glu Arg His Gly Ala Lys
        2605                2610                2615

GTG ATT GCA GTC GAC TCG CCC GCC ATC GAG ACG GCG GTC AGC CAA CAG 8165
Val Ile Ala Val Asp Ser Pro Ala Ile Glu Thr Ala Val Ser Gln Gln
    2620                2625                2630

TCA GCT GCT GAC CTG CCC ACA ATT GCT AGC CTC GGC AAT CTA GCG TAT 8213
Ser Ala Ala Asp Leu Pro Thr Ile Ala Ser Leu Gly Asn Leu Ala Tyr
2635                2640                2645                2650

ATA ATC TTT ACT TCA GGC ACT TCC GGT AAG CCA AAG GGA GTC CTA GTT 8261
Ile Ile Phe Thr Ser Gly Thr Ser Gly Lys Pro Lys Gly Val Leu Val
                2655                2660                2665

GAG CAA AAG GCA GTT CTT CTT CTA CGC GAT GCC CTC CGG GAG CGG TAT 8309
Glu Gln Lys Ala Val Leu Leu Leu Arg Asp Ala Leu Arg Glu Arg Tyr
            2670                2675                2680

TTC GGT CGA GAC TGT ACC AAG CAT CAT GGC GTC CTG TTC CTG TCC AAC 8357
Phe Gly Arg Asp Cys Thr Lys His His Gly Val Leu Phe Leu Ser Asn
        2685                2690                2695

TAC GTC TTC GAC TTC TCC GTC GAA CAA CTT GTG TTG TCG GTG CTC AGC 8405
Tyr Val Phe Asp Phe Ser Val Glu Gln Leu Val Leu Ser Val Leu Ser
    2700                2705                2710

GGA CAC AAG CTG ATC GTT CCC CCA GCT GAG TTC GTC GCA GAT GAT GAA 8453
Gly His Lys Leu Ile Val Pro Pro Ala Glu Phe Val Ala Asp Asp Glu
2715                2720                2725                2730

TTT TAC AGA ATG GCC AGC ACG CAC GGT CTC TCC TAT CTC AGC GGC ACA 8501
Phe Tyr Arg Met Ala Ser Thr His Gly Leu Ser Tyr Leu Ser Gly Thr
                2735                2740                2745

CCA TCC TTA CTG CAG AAG ATC GAT CTG GCA CGA CTG GAC CAT CTG CAG 8549
Pro Ser Leu Leu Gln Lys Ile Asp Leu Ala Arg Leu Asp His Leu Gln
            2750                2755                2760

GTT GTT ACC GCC GCG GGC GAA GAG CTT CAC GCC ACC CAG TAC GAG AAG 8597
Val Val Thr Ala Ala Gly Glu Glu Leu His Ala Thr Gln Tyr Glu Lys
        2765                2770                2775

ATG CGC CGC CGA TTC AAC GGT CCC ATC TAC AAT GCC TAT GGT GTC ACC 8645
Met Arg Arg Arg Phe Asn Gly Pro Ile Tyr Asn Ala Tyr Gly Val Thr
    2780                2785                2790

GAG ACC ACG GTG TAC AAC ATT ATC GCG GAA TTC ACA ACG AAT TCG ATA 8693
Glu Thr Thr Val Tyr Asn Ile Ile Ala Glu Phe Thr Thr Asn Ser Ile
2795                2800                2805                2810

TTT GAG AAT GCT CTT CGG GAA GTG CTC CCT GGT ACC CGA GCG TAT GTG 8741
Phe Glu Asn Ala Leu Arg Glu Val Leu Pro Gly Thr Arg Ala Tyr Val
                2815                2820                2825

CTG AAC GCG GCA CTT CAG CCC GTC CCC TTC GAT GCT GTC GGA GAA CTC 8789
Leu Asn Ala Ala Leu Gln Pro Val Pro Phe Asp Ala Val Gly Glu Leu
            2830                2835                2840

TAT CTT GCC GGC GAC ACG GTT ACG CGT GGT TAT CTC AAC CAA CCT CTT     8837
Tyr Leu Ala Gly Asp Thr Val Thr Arg Gly Tyr Leu Asn Gln Pro Leu
        2845                2850                2855

CTA ACG GAT CAG CGA TTC ATT CCC AAC CCT TTC TGC AAA GAG GAG GAC     8885
Leu Thr Asp Gln Arg Phe Ile Pro Asn Pro Phe Cys Lys Glu Glu Asp
    2860                2865                2870

ATC GCT ATG GGG CGC TTC GCG CGG CTC TAC AAG ACC GGC GAC CTG GTT     8933
Ile Ala Met Gly Arg Phe Ala Arg Leu Tyr Lys Thr Gly Asp Leu Val
2875                2880                2885                2890

CGA TCG CGT TTC AAC CGT CAG CAG CAG CCG CAG CTG GAA TAC CTA GGA     8981
Arg Ser Arg Phe Asn Arg Gln Gln Gln Pro Gln Leu Glu Tyr Leu Gly
                2895                2900                2905

AGA GGC GAT CTG CAG ATC AAG ATG AGG GGA TAC CGG ATC GAG ATT TCT     9029
Arg Gly Asp Leu Gln Ile Lys Met Arg Gly Tyr Arg Ile Glu Ile Ser
            2910                2915                2920

GAA GTT CAG AAC GTG CTC ACT TCA AGT CCC GGT GTC CGG GAG GGT GCA     9077
Glu Val Gln Asn Val Leu Thr Ser Ser Pro Gly Val Arg Glu Gly Ala
        2925                2930                2935

GTC GTT GCC AAG TAT GAG AAC AAC GAT ACC TAT TCC CGG ACC GCT CAC     9125
Val Val Ala Lys Tyr Glu Asn Asn Asp Thr Tyr Ser Arg Thr Ala His
    2940                2945                2950

TCT CTG GTC GGT TAC TAT ACC ACG GAC AAT GAA ACA GTA TCG GAA GCC     9173
Ser Leu Val Gly Tyr Tyr Thr Thr Asp Asn Glu Thr Val Ser Glu Ala
2955                2960                2965                2970

GAT ATT CTC ACT TTC ATG AAA GCA AGG CTT CCA ACG TAC ATG GTG CCA     9221
Asp Ile Leu Thr Phe Met Lys Ala Arg Leu Pro Thr Tyr Met Val Pro
                2975                2980                2985

AGC CAC CTC TGC TGT CTG GAA GGC GCA CTG CCT GTG ACG ATT AAC GGA     9269
Ser His Leu Cys Cys Leu Glu Gly Ala Leu Pro Val Thr Ile Asn Gly
            2990                2995                3000

AAG CTC GAC GTC CGG AGA TTG CCG GAG ATT ATC AAC GAC TCC GCG CAG     9317
Lys Leu Asp Val Arg Arg Leu Pro Glu Ile Ile Asn Asp Ser Ala Gln
        3005                3010                3015

TCC TCG TAC AGC CCA CCA AGG AAC ATA ATC GAG GCC AAG ATG TGC AGA     9365
Ser Ser Tyr Ser Pro Pro Arg Asn Ile Ile Glu Ala Lys Met Cys Arg
    3020                3025                3030

CTG TGG GAA TCC GCC TTG GGA ATG GAG CGA TGC GGT ATC GAC GAC GAC     9413
Leu Trp Glu Ser Ala Leu Gly Met Glu Arg Cys Gly Ile Asp Asp Asp
3035                3040                3045                3050

CTG TTC AAA CTG GGT GGC GAC AGC ATC ACA TCT TTG CAT CTC GTG GCC     9461
Leu Phe Lys Leu Gly Gly Asp Ser Ile Thr Ser Leu His Leu Val Ala
                3055                3060                3065

CAG ATT CAC AAC CAG GTG GGC TGC AAG ATC ACC GTT CGG GAT ATA TTT 9509
Gln Ile His Asn Gln Val Gly Cys Lys Ile Thr Val Arg Asp Ile Phe
            3070                3075                3080

GAA CAT CGT ACC GCC CGA GCC CTC CAT GAT CAC GTC TTC ATG AAG GAC 9557
Glu His Arg Thr Ala Arg Ala Leu His Asp His Val Phe Met Lys Asp
        3085                3090                3095

TCC GAC CGG AGT AAT GTG ACT CAG TTC CGA ACC GAA CAA GGG CCG GTC 9605
Ser Asp Arg Ser Asn Val Thr Gln Phe Arg Thr Glu Gln Gly Pro Val
    3100                3105                3110

ATC GGC GAG GCG CCC CTA CTG CCG ATT CAA GAC TGG TTT TTG TCA AAG 9653
Ile Gly Glu Ala Pro Leu Leu Pro Ile Gln Asp Trp Phe Leu Ser Lys
3115                3120                3125                3130

GCT CTG CAG CAT CCG ATG TAT TGG AAT CAC ACT TTC TAC GTC CGA ACG 9701
Ala Leu Gln His Pro Met Tyr Trp Asn His Thr Phe Tyr Val Arg Thr
                3135                3140                3145

CCA GAG CTG GAT GTT GAT TCC TTA AGC GCT GCT GTC AGG GAC TTG CAA 9749
Pro Glu Leu Asp Val Asp Ser Leu Ser Ala Ala Val Arg Asp Leu Gln
            3150                3155                3160

CAG TAT CAC GAT GTT TTC CGC ATG CGA CTC AAG CGC GAG GAA GTC GGA 9797
Gln Tyr His Asp Val Phe Arg Met Arg Leu Lys Arg Glu Glu Val Gly
        3165                3170                3175

TTC GTG CAG TCC TTT GCT GAG GAC TTC TCT CCT GCC CAG CTT CGG GTG 9845
Phe Val Gln Ser Phe Ala Glu Asp Phe Ser Pro Ala Gln Leu Arg Val
    3180                3185                3190

CTG AAC GTA AAA GAT GTT GAC GGG TCC GCG GCC GTC AAC GAG ATA TTG 9893
Leu Asn Val Lys Asp Val Asp Gly Ser Ala Ala Val Asn Glu Ile Leu
3195                3200                3205                3210

GAT GGG TGG CAG TCT GGC TTC AAC CTT GAG AAC GGA CCC ATT GGT TCC 9941
Asp Gly Trp Gln Ser Gly Phe Asn Leu Glu Asn Gly Pro Ile Gly Ser
                3215                3220                3225

ATT GGC TAC CTA CAT GGG TAT GAA GAC CGA TCC GCG CGA GTC TGG TTC 9989
Ile Gly Tyr Leu His Gly Tyr Glu Asp Arg Ser Ala Arg Val Trp Phe
            3230                3235                3240

TCC GTT CAC CAT ATG GCC ATT GAC ACC GTC AGC TGG CAG ATC CTT GTC 10037
Ser Val His His Met Ala Ile Asp Thr Val Ser Trp Gln Ile Leu Val
        3245                3250                3255

CGT GAC CTG CAG ACG CTG TAC CGA AAT GGA AGC CTC GGA AGC AAG GGC 10085
Arg Asp Leu Gln Thr Leu Tyr Arg Asn Gly Ser Leu Gly Ser Lys Gly
    3260                3265                3270

AGC AGT TTC CGG CAG TGG GCT GAA GCC ATC CAA AAT TAC AAG GCG TCA 10133
Ser Ser Phe Arg Gln Trp Ala Glu Ala Ile Gln Asn Tyr Lys Ala Ser
3275                3280                3285                3290

GAC TCT GAG AGG AAC CAT TGG AAT AAG CTC GTC ATG GAA ACA GCT TCC     10181
Asp Ser Glu Arg Asn His Trp Asn Lys Leu Val Met Glu Thr Ala Ser
            3295                3300                3305

AGC ATA TCC GCA TTG CCT ACG TCA ACC GGT TCG CGC GTG CGC CTG AGC     10229
Ser Ile Ser Ala Leu Pro Thr Ser Thr Gly Ser Arg Val Arg Leu Ser
        3310                3315                3320

AGA AGT TTG AGC CCT GAG AAG ACA GCC TCA CTG ATC CAA GGA GGA ATC     10277
Arg Ser Leu Ser Pro Glu Lys Thr Ala Ser Leu Ile Gln Gly Gly Ile
    3325                3330                3335

GAT CGA CAG GAT GTC TCC GTG TAC GAC TCC CTC CTG ACT TCA GTT GGA     10325
Asp Arg Gln Asp Val Ser Val Tyr Asp Ser Leu Leu Thr Ser Val Gly
3340                3345                3350

TTG GCG CTC CAA CAT ATC GCT CCA ACC GGC CCA AGT ATG GTT ACG ATC     10373
Leu Ala Leu Gln His Ile Ala Pro Thr Gly Pro Ser Met Val Thr Ile
3355                3360                3365                3370

GAG GGA CAT GGC CGT GAA GAA GTG GAT CAG ACA CTG GAT GTG AGC CGC     10421
Glu Gly His Gly Arg Glu Glu Val Asp Gln Thr Leu Asp Val Ser Arg
            3375                3380                3385

ACC ATG GGT TGG TTC ACC ACC ATG TAT CCA TTT GAA ATT CCC CGT CTC     10469
Thr Met Gly Trp Phe Thr Thr Met Tyr Pro Phe Glu Ile Pro Arg Leu
        3390                3395                3400

AGC ACC GAG AAC ATT GTT CAA GGA GTC GTC GCT GTG AGC GAA CGG TTC     10517
Ser Thr Glu Asn Ile Val Gln Gly Val Val Ala Val Ser Glu Arg Phe
    3405                3410                3415

AGA CAG GTG CCT GCC CGT GGC GTC GGG TAT GGA ACC TTG TAC GGC TAT     10565
Arg Gln Val Pro Ala Arg Gly Val Gly Tyr Gly Thr Leu Tyr Gly Tyr
3420                3425                3430

ACT CAA CAC CCG CTG CCC CAG GTG ACC GTC AAC TAC CTG GGC CAG CTC     10613
Thr Gln His Pro Leu Pro Gln Val Thr Val Asn Tyr Leu Gly Gln Leu
3435                3440                3445                3450

GCC CGC AAG CAA TCG AAG CCA AAG GAA TGG GTC CTC GCG GTG GGC GAC     10661
Ala Arg Lys Gln Ser Lys Pro Lys Glu Trp Val Leu Ala Val Gly Asp
            3455                3460                3465

AAC GAA TTT GAA TAC GGA CTC ATG ACT AGC CCA GAG GAC AAA GAC CGG     10709
Asn Glu Phe Glu Tyr Gly Leu Met Thr Ser Pro Glu Asp Lys Asp Arg
        3470                3475                3480

AGC TCT TCT GCC GTC GAC GTC ACG GCC GTG TGT ATT GAC GGC ACT ATG     10757
Ser Ser Ser Ala Val Asp Val Thr Ala Val Cys Ile Asp Gly Thr Met
    3485                3490                3495

ATC ATC GAT GTG GAC AGT GCT TGG AGC CTT GAG GAG AGC GAG CAA TTC     10805
Ile Ile Asp Val Asp Ser Ala Trp Ser Leu Glu Glu Ser Glu Gln Phe
3500                3505                3510

ATC TCG AGC ATC GAG GAA GGA CTG AAC AAG ATC CTC GAC GGC AGG GCA 10853
Ile Ser Ser Ile Glu Glu Gly Leu Asn Lys Ile Leu Asp Gly Arg Ala
3515                3520                3525                3530

AGT CAG CAA ACC TCG CGA TTC CCG GAT GTT CCT CAA CCG GCG GAG ACA 10901
Ser Gln Gln Thr Ser Arg Phe Pro Asp Val Pro Gln Pro Ala Glu Thr
                3535                3540                3545

TAT ACG CCG TAT TTC GAG TAT CTG GAA CCT CCA CGA CAG GGA CCG ACG 10949
Tyr Thr Pro Tyr Phe Glu Tyr Leu Glu Pro Pro Arg Gln Gly Pro Thr
            3550                3555                3560

CTG TTC CTG CTG CCG CCG GGC GAA GGA GGC GCC GAG AGT TAC TTC AAC 10997
Leu Phe Leu Leu Pro Pro Gly Glu Gly Gly Ala Glu Ser Tyr Phe Asn
        3565                3570                3575

AAC ATC GTC AAG CGC CTG CGT CAG ACA AAT ATG GTG GTC TTC AAC AAC 11045
Asn Ile Val Lys Arg Leu Arg Gln Thr Asn Met Val Val Phe Asn Asn
    3580                3585                3590

TAC TAC TTG CAC AGC AAA CGC CTG CGC ACG TTC GAG GAG CTG GCG GAA 11093
Tyr Tyr Leu His Ser Lys Arg Leu Arg Thr Phe Glu Glu Leu Ala Glu
3595                3600                3605                3610

ATG TAT CTC GAC CAA GTA CGC GGC ATC CAA CCA CAC GGA CCG TAC CAC 11141
Met Tyr Leu Asp Gln Val Arg Gly Ile Gln Pro His Gly Pro Tyr His
                3615                3620                3625

TTC ATC GGA TGG AGC TTC GGA GGA ATT CTC GCA ATG GAA ATG TCG CGG 11189
Phe Ile Gly Trp Ser Phe Gly Gly Ile Leu Ala Met Glu Met Ser Arg
            3630                3635                3640

CGA CTG GTA GCC TCG GAC GAG AAG ATT GGC TTC CTC GGT ATT ATC GAC 11237
Arg Leu Val Ala Ser Asp Glu Lys Ile Gly Phe Leu Gly Ile Ile Asp
        3645                3650                3655

ACC TAT TTC AAC GTG CGG GGA GCG ACA CGC ACC ATT GGC TTG GGG GAC 11285
Thr Tyr Phe Asn Val Arg Gly Ala Thr Arg Thr Ile Gly Leu Gly Asp
    3660                3665                3670

ACT GAG ATT CTG GAC CCG ATC CAT CAC ATC TAC AAT CCC GAT CCG GCC 11333
Thr Glu Ile Leu Asp Pro Ile His His Ile Tyr Asn Pro Asp Pro Ala
3675                3680                3685                3690

AAC TTC CAA CGC CTG CCC TCT GCA ACA GAT CGC ATT GTG CTG TTC AAG 11381
Asn Phe Gln Arg Leu Pro Ser Ala Thr Asp Arg Ile Val Leu Phe Lys
                3695                3700                3705

GCC ATG AGG CCG AAC AAC AAG TAC GAA TCC GAG AAC CAG CGT CGC CTG 11429
Ala Met Arg Pro Asn Asn Lys Tyr Glu Ser Glu Asn Gln Arg Arg Leu
            3710                3715                3720

TAC GAG TAC TAT GAC CGC ACT CGA CTC AAC GGA CTG GAC AGC TTG TTA 11477
Tyr Glu Tyr Tyr Asp Gly Thr Arg Leu Asn Gly Leu Asp Ser Leu Leu
        3725                3730                3735

CCA AGC GAT TCC GAC GTC CAG CTG GTC CCG CTT ACG GAC GAT ACA CAC 11525
Pro Ser Asp Ser Asp Val Gln Leu Val Pro Leu Thr Asp Asp Thr His
    3740                3745                3750

TTT TCC TGG GTC GGA AAT CCA CAA CAG GTG GAG CAG ATG TGT GCG ACT 11573
Phe Ser Trp Val Gly Asn Pro Gln Gln Val Glu Gln Met Cys Ala Thr
3755                3760                3765                3770

ATC AAG GAA CAC CTC GCT CGC TAT TGA 11600
Ile Lys Glu His Leu Ala Arg Tyr End
                3775

TCCGTCACTA GCAGCACAGT ATATCGGACG ATGGAAGTGA TGGAGTGGGG GGGATAGGAT 11660
ACGATCAAAC CAGGGTGCGG TTCTTTTTGG GGGGAACTAG TCTCTGGTTG AGGAAAGCGA 11720
GGTAGCAAAT AAACTACCAA GGTCTAGACC CACATAGTCT GTCATTGTTT TCGATCCTAA 11780
ATTGATATAT AATAGGCGAC ACCTTTAGTT AGCCAAATTT TCTATATAGA AACACCACGG 11840
TTTTTAGGAG TTAGGACCAC GTCAGACCGT GGCCCTTTCA CTAACGCTTC CGTTTACATC 11900
CATACCGGAT GTGGTTGTAG CACATTTATA TGATATCATT TAAGACTATA TACGCCTATT 11960
CCCCCCTATC GAATAGGCCC CTACGTATTT CTTTTGTTTT TTTCTTTTTC TTTTTTTTTT 12020
TTTTCTTTTC GCTCTCTCCC CTTTTATACC CAATATCGGA TCGAGTTGAT AATATCAATA 12080
TCTAAAACTC CCAATTAAAC CTACAAAGCC TATCTTAGTG TAAGTGAATT TGGGCTCTGG 12140
ACCAAATTCT CCGCCAAGGA TAATCTTTCC GATAAACGGT GGTTATCCGG TCATCATAAA 12200
AAAGGAAAAG GTACTCCGTC CTCGATAATA AAACGTAACA TAAGCATGTC GTTCACCATA 12260
GACAAGAGGA ACCAACATCA TTAAGCAGGG GATAGGTTCA TCCGGTCTAG GGCGTCGAGT 12320
GCCACCGCCC GTAGGTTGTC AATCTTGAGC TGGATTTGGA GATC 12364

### Sequence Listing No. 2

SEQ. SEQ. ID No.: 2
SEQUENCE TYPE: Nucleotide with corresponding protein
SEQUENCE LENGTH: 11601 base pairs
STRANDEDNESS: single
TOPOLOGY: linear
MOLECULE TYPE: genomic
ORGANISM: Acremonium chrysogenum
FEATURES: from 388 to 11526 bp peptide
PROPERTIES: gene encoding ACV Synthetase

GGATGCATGC ATTGGCCTGT ATCAAAGGTC CGGGATTCCC CAGGAGTATA AGACGTTCGT 60
GCTGGGAGAT CTAGCGACGT GTTGGGAAAT ATCGGCCGTA GAGTGCGAAA AAGAACTGGC 120
GGAAATATTT CTCCTTGGAC TCGGTCACAC TCAGTCAGTA GTGGACTGCC AGTCTATCAT 180
ACACCTTTGA TATCAACATG ACTATCCTTA CAGGTGCCGA CGACGCCTCG TCATACCACA 240
GGTATGTCTT CACAGCCTCT GGAAAGCGCA GTTGGGAGCT ATCTCTAACA TTACCACATC 300
AGGCGCAATG GAAGCTCTGA TATCCCAAAA GGTGCCATCC ACCGCAACGG CTTCGCAGCC 360
GCAGCCCCTG ACTGCTGGAT CCGGTCC GTG GCC CTG GAA CAG TGG AAG ACT ACG 414
Val Ala Leu Glu Gln Trp Lys Thr Thr
1               5

GTC CAG TCC GTC TCG GAG CGG TGC GAT CTG AGC GGG CTG AGC CAG CAT 462
Val Gln Ser Val Ser Glu Arg Cys Asp Leu Ser Gly Leu Ser Gln His
10                  15                  20                  25

CCC ACC GAC TAC CAG CTG GCC TCT ACG GGC GTG AAG GGC GCA GGC GGT 510
Pro Thr Asp Tyr Gln Leu Ala Ser Thr Gly Val Lys Gly Ala Gly Gly
                30                  35                  40

AGC AGC ATC GAG GAG CGC AGT GCC ATC GTC TCA GAC GAG TTG TTC TCG 558
Ser Ser Ile Glu Glu Arg Ser Ala Ile Val Ser Asp Glu Leu Phe Ser
            45                  50                  55

AGT CTG CGA GAC GTG TGC TCA CAG AGA CAG CTG GAC CCT CGG TCA CTC 606
Ser Leu Arg Asp Val Cys Ser Gln Arg Gln Leu Asp Pro Arg Ser Leu
        60                  65                  70

ATG CTG TTT TCC GTG CAC CAG ATG CTC AAG AGG TTC GGA AAC GGA TCT 654
Met Leu Phe Ser Val His Gln Met Leu Lys Arg Phe Gly Asn Gly Ser
    75                  80                  85

CAC ACC GTC GTG GCG TCA CTC GTA ACT TCA TCA GAG GGA TGC CCT TCA 702
His Thr Val Val Ala Ser Leu Val Thr Ser Ser Glu Gly Cys Pro Ser
90                  95                  100                 105

ACT TCG GCC TGG AGG GCC ATC CCC TCC GTC ATC CAT CAT ATA GAG GGC 750
Thr Ser Ala Trp Arg Ala Ile Pro Ser Val Ile His His Ile Glu Gly
                110                 115                 120

GGA GAC AAC AAC AAC ACA GTC GCC TCT GCC GTG GAA CAG GCG GCG AAT 798
Gly Asp Asn Asn Asn Thr Val Ala Ser Ala Val Glu Gln Ala Ala Asn
            125                 130                 135

CTC CTG AAC TCA GAA GGA TCG GGA CAG GAC CTT CTG ATT CCC ATC GGA 846
Leu Leu Asn Ser Glu Gly Ser Gly Gln Asp Leu Leu Ile Pro Ile Gly
        140                 145                 150

CTC ACT GAG CTC GTC AAG TCG GAG CTG ATT GAC CTC CTG GTC ATC TTC 894
Leu Thr Glu Leu Val Lys Ser Glu Leu Ile Asp Leu Leu Val Ile Phe
    155                 160                 165

GAC GAC GAG ACA AAT AAC ATA CGA CTG CCG CAG GAC TTC CCA CTT ATC 942
Asp Asp Glu Thr Asn Asn Ile Arg Leu Pro Gln Asp Phe Pro Leu Ile
170                 175                 180                 185

CTG CGG ATA CAT CAG CGG CAA GAC CAC TGG CAG CTG TCA GTC CGG TAT 990
Leu Arg Ile His Gln Arg Gln Asp His Trp Gln Leu Ser Val Arg Tyr
                190                 195                 200

CCC TCG CCC CTT TTC GAC ACC ATG GTC ATC GAC AGC TTT CTG AGC GCA 1038
Pro Ser Pro Leu Phe Asp Thr Met Val Ile Asp Ser Phe Leu Ser Ala
            205                 210                 215

CTT CAC AAC CTG TTG TCC GCG GTG ACA AAA CCC TCC CAG CTC GTG CGC 1086
Leu His Asn Leu Leu Ser Ala Val Thr Lys Pro Ser Gln Leu Val Arg
        220                 225                 230

GAC ATC GAG CTG CTC CCA GAA TAC CAG GTC GCT CAG CTG GAG AAG TGG 1134
Asp Ile Glu Leu Leu Pro Glu Tyr Gln Val Ala Gln Leu Glu Lys Trp
    235                 240                 245

AAC AAC ACA GAC GGC GAC TAC CCC ACC GAG AAG CGG CTA CAT CAT CTG 1182
Asn Asn Thr Asp Gly Asp Tyr Pro Thr Glu Lys Arg Leu His His Leu
250                 255                 260                 265

TTC GAG GAG GCA GCA GTG CGT CGT CCC CAA CAC GTT GCC CTC ATC TGC 1230
Phe Glu Glu Ala Ala Val Arg Arg Pro Gln His Val Ala Leu Ile Cys
                270                 275                 280

GGC GAC AAG CGC ATC ACC TAT GAG GAG TTG AAT GCT ATG GCG AAT CGC 1278
Gly Asp Lys Arg Ile Thr Tyr Glu Glu Leu Asn Ala Met Ala Asn Arg
            285                 290                 295

CTG GCC CAC CAT CTG GTA TCC TCG GGT ATC CAG ACT GAG CAG CTC GTC 1326
Leu Ala His His Leu Val Ser Ser Gly Ile Gln Thr Glu Gln Leu Val
        300                 305                 310

GGT CTC TTC CTC GAC AAG ACC GAG CTC ATG ATC GCT ACT ATT CTG GGC 1374
Gly Leu Phe Leu Asp Lys Thr Glu Leu Met Ile Ala Thr Ile Leu Gly
    315                 320                 325

ATC TGG AAA TCT GGT GCC GCG CAT GTA CCT ATC GAC CCT GGG TAC CCG 1422
Ile Trp Lys Ser Gly Ala Ala His Val Pro Ile Asp Pro Gly Tyr Pro
330                 335                 340                 345

GAC GAG CGT GTC AAG TTC GTC CTG AAT GAT ACG AAG GCG CAA GTG GTC 1470
Asp Glu Arg Val Lys Phe Val Leu Asn Asp Thr Lys Als Gln Val Val
350 355 360

ATT GCT AGT CAG AGG CAC GTC GAT CGA CTG CGG GCT GAG GCT GTT GGC 1518
Ile Ala Ser Gln Arg His Val Asp Arg Leu Arg Ala Glu Ala Val Gly
365 370 375

GGC CAG CAT CTT CGG ATC ATC GGT CTC GAA TCT CTG TTC GAC AAC CTT 1566
Gly Gln His Leu Arg Ile Ile Gly Leu Glu Ser Leu Phe Asp Asn Leu
380 385 390

GCT CAA CAG ACA CAA CAC TCA CCA GAG ACG TCG GGC AAT TTG ACC CAT 1614
Ala Gln GLn Thr Gln His Ser Pro Glu Thr Ser Gly Asn Leu Thr His
395 400 405

CTG CCC CTG AAC AGC AAA CAG CTT GCG TAC GTG ACA TAC ACC TCG GGC 1662
Leu Pro Leu Asn Ser Lys Gln Leu Ala Tyr Val Thr Tyr Thr Ser Gly
410 415 420 425

ACC ACG GGC TTC CCG AAA GGC ATC TAC AAG GAG CAC ACA AGC GTC GTT 1710
Thr Thr Gly Phe Pro Lys Gly Ile Tyr Lys Glu His Thr Ser Val Val
430 435 440

AAC AGC ATC ACC GAT CTG TCT GCT CGG TAC GGT GTG GCC GGG GAG GAC 1758
Asn Ser Ile Thr Asp Leu Ser Ala Arg Tyr Gly Val Ala Gly Glu Asp
445 450 455

GAC GAG GTG ATA CTC GTC TTC TCC GCC TAC GTC TTC GAG CCA TTC GTG 1806
Asp Glu Val Ile Leu Val Phe Ser Ala Tyr Val Phe Glu Pro Phe Val
460 465 470

CGC CAG ATG CTC ATG GCC CTG ACC ACG GGC AAC TCT CTC GCC ATC ATC 1854
Arg Pro Met Leu Met Ala Leu Thr Thr Gly Asn Ser Leu Ala Ile Ile
475 480 485

AGC GAC GAG GAC AAG TTC GAC CCT GAC ACC CTT ATT CCC TTC ATC CAA 1902
Ser Asp Glu Asp Lys Phe Asp Pro Asp Thr Leu Ile Pro Phe Ile Gln
490 495 500 505

AAA CAC AAA GTC ACT TAC ATC CAC GCC ACC TCG TCA GTG TTG CAG GAG 1950
Lys His Lys Val Thr Tyr Ile His Ala Thr Ser Ser Val Leu Gln Glu
510 515 520

TAC GAC TTC GGG TCC TGC CCC TCG TTG AAA CGC ATG ATT CTG GTG GGA 1998
Tyr Asp Phe Gly Ser Cys Pro Ser Leu Lys Arg Met Ile Leu Val Gly
525 530 535

GAG AAC TTG ACA GAG CCG CGC TAC GAG GCC CTG AGG CAG CGC TTC AAG 2046
Glu Asn Leu Thr Glu Pro Arg Tyr Glu Ala Leu Arg Gln Arg Phe Lys
540 545 550

TCG CGC ATC CTG AAT GAA TAT GGC TTC ACC GAG TCT GCG TTT GTG ACG 2094
Ser Arg Ile Leu Asn Glu Tyr Gly Phe Thr Glu Ser Ala Phe Val Thr
555 560 565

GCG CTC AAC ATA TTC GAG CCT ACC TCA CAG AGG AAG GAC ATG AGT CTG 2142
Ala Leu Asn Ile Phe Glu Pro Thr Ser Gln Arg Lys Asp Met Ser Leu
570 575 580 585

GGA AGG CCG GTG CGC AAC GTC AAG TGC TAT ATC TTG GAT GCC AAC CTC 2190
Gly Arg Pro Val Arg Asn Val Lys Cys Tyr Ile Leu Asp Ala Asn Leu
590 595 600

AAG AGA GTC CCC ATC GGT GTT ACA GGG GAG CTG CAC ATC GGT GGC TTG 2238
Lys Arg Val Pro Ile Gly Val Thr Gly Glu Leu His Ile Gly Gly Leu
605 610 615

GGT ATA TCC CGG GGG TAC ATG AAT AGG GAG GAG CTC ACA AGG CAG AAG 2286
Gly Ile Ser Arg Gly Tyr Met Asn Arg Glu Glu Leu Thr Arg Gln Lys
620 625 630

TTC CTC CCG AAC CCC TAC CAG ACC GAT AAG GAG CGC CAA CGG GGT GTC 2334
Phe Leu Pro Asn Pro Tyr Gln Thr Asp Lys Glu Arg Gln Arg Gly Val
635 640 645

AAC TCA ACC ATG TAC AAG ACA GGA GAT CTG GCC CGC TGG CTA CCC AGT 2382
Asn Ser Thr Met Tyr Lys Thr Gly Asp Leu Ala Arg Trp Leu Pro Ser
650 655 660 665

GGC GAA GTC GAG TAT CTC GGC CGT GCC GAC TTC CAG ATC AAG CTG CGC 2430
Gly Glu Val Glu Tyr Leu Gly Arg Ala Asp Phe Gln Ile Lys Leu Arg
670 675 680

GGC ATT CGA ATT GAG CCC GGC GAG ATC GAG TCC ACT CTC GCC ATG TAT 2478
Gly Ile Arg Ile Glu Pro Gly Glu Ile Glu Ser Thr Leu Ala Met Tyr
685 690 695

CCC GGA ATC AGG GCC AGC ATC GTC GTG TCA AAG AAG CTT CTC AGT CAG 2526
Pro Gly Ile Arg Ala Ser Ile Val Val Ser Lys Lys Leu Leu Ser Gln
700 705 710

GGG CAG GAG ACG ATC CAA GAC CAC CTT GTG GGG TAC TAT GTT TGC GAT 2574
Gly Gln Glu Thr Ile Gln Asp His Leu Val Gly Tyr Tyr Val Cys Asp
715 720 725

GAG GGC CAC ATC CCC GAG GGT GAC CTG CTG AGC TTC CTG GAG AAG AAG 2622
Glu Gly His Ile Pro Glu Gly Asp Leu Leu Ser Phe Leu Glu Lys Lys
730 735 740 745

CTA CCT CGG TAC ATG GTC CCG ACG CGC CTT GTC CAA CTG GCT CAG ATT 2670
Leu Pro Arg Tyr Met Val Pro Thr Arg Leu Val Gln Leu Ala Gln Ile
750 755 760

CCA ACC AAT ATC AAC GGC AAG GCG GAT CTG CGT GCT CTT CCT GCC GTC 2718
Pro Thr Asn Ile Asn Gly Lys Ala Asp Leu Arg Ala Leu Pro Ala Val
765 770 775

GAA GTC GCC GTA GCT CCC ACC CAC AAG CAG GAT GGC GAG CGA GGA AAC 2766
Glu Val Ala Val Ala Pro Thr His Lys Gln Asp Gly Glu Arg Gly Asn
780 785 790

CAG CTG GAG AGC GAC CTG GCT GCC ATA TGG GGC AAC ATT TTG AGT GTT 2814
Gln Leu Glu Ser Asp Leu Ala Ala Ile Trp Gly Asn Ile Leu Ser Val
795 800 805

CCC GCT CAA GAC ATT GGG TCT GAA TCC AAC TTC TTC CGC CTG GGT GGC 2862
Pro Ala Gln Asp Ile Gly Ser Glu Ser Asn Phe Phe Arg Leu Gly Gly
810 815 820 825

CAC AGT ATT GCA TGC ATC CAG CTC ATT GCT CGT GTG CGA CAG CAG CTA 2910
His Ser Ile Ala Cys Ile Gln Leu Ile Ala Arg Val Arg Gln Gln Leu
830 835 840

GGC CAG GGG ATT ACC CTC GAG GAG GTC TTC CAG ACC AAG ACG TTG CGA 2958
Gly Gln Gly Ile Thr Leu Glu Glu Val Phe Gln Thr Lys Thr Leu Arg
845 850 855

GCT ATG GCT GCC CTC TTG TCG GAA AAG TAC ACG AAG GCG TCG AAT GGG 3006
Ala Met Ala Ala Leu Leu Ser Glu Lys Tyr Thr Lys Ala Ser Asn Gly
860 865 870

ACG AAC GGA GTG ACC AAC GGC ACT GCT CAC GTC AAC GGC CAC GCA GCG 3054
Thr Asn Gly Val Thr Asn Gly Thr Ala His Val Asn Gly His Ala Ala
875 880 885

AAC GGC CAT GTC AGC GAC AGC TAC GTG GCC AGC AGT TTG CAG CAA GGC 3102
Asn Gly His Val Ser Asp Ser Tyr Val Ala Ser Ser Leu Gln Gln Gly
890 895 900 905

TTT GTT TAC CAT TCA CTC AAG AAC GAA CTG TCC GAG GCG TAC ACC ATG 3150
Phe Val Tyr His Ser Leu Lys Asn Glu Leu Ser Glu Ala Tyr Thr Met
910 915 920

CAA TCC ATG ATC CAC TAT GGT GTG CCC CTG AAA CGG GAT ATT TAC CAA 3198
Gln Ser Met Ile His Tyr Gly Val Pro Leu Lys Arg Asp Ile Tyr Gln
925 930 935

GCG GCA TGG CAG AGG GTA CAG GGG GAG CAC CCT GCA CTG CGG CTT CGG 3246
Ala ALa Trp Gln Arg Val Gln Gly Glu His Pro Ala Leu Arg Leu Arg
940 945 950

TTC ACA TGG GAG GCC GAA GTG ATG CAG ATC GTG GAC CCG AAA TCT GAA 3294
Phe Thr Trp Glu Ala Glu Val Met Gln Ile Val Asp Pro Lys Ser Glu
955 960 965

CTC GAC TGG CGT GTT GTT GAC TGG ACC GAT GTT TCG AGC CGG GAG AAG 3342
Leu Asp Trp Arg Val Val Asp Trp Thr Asp Val Ser Ser Arg Glu Lys
970 975 980 985

CAG CTG GTT GCG CTG GAG CAA CTC CAA ACG GAG GAC CTT GCT AAG GTC 3390
Gln Leu Val Ala Leu Glu Gln Leu Gln Thr Glu Asp Leu Ala Lys Val
990 995 1000

TAC CAT CTC GAT AAG GGG CCC CTT ATG CGA CTA TAC CTC ATC CTG CTT 3438
Tyr His Leu Asp Lys Gly Pro Leu Met Arg Leu Tyr Leu Ile Leu Leu
1005 1010 1015

CCG GAC TCA AAG TAC TCC TGT CTG TTC AGC TGC CAC CAT GCC ATT CTC 3486
Pro Asp Ser Lys Tyr Ser Cys Leu Phe Ser Cys His His Ala Ile Leu
1020 1025 1030

GAT GGG TGG AGT CTG CCC CTG CTC TTC AAC AAT GTC CAC CAG GCC TAC 3534
Asp Gly Trp Ser Leu Pro Leu Leu Phe Asn Asn Val His Gln Ala Tyr
1035 1040 1045

CTC GAT CTC GTC GAA GGC ACT GCT TCG CCC GTC GAG CAG GAC GCT ACC 3582
Leu Asp Leu Val Glu Gly Thr Ala Ser Pro Val Glu Gln Asp Ala Thr
1050 1055 1060 1065

TAC CTA CTC GGC CAG CAG TAC CTG CAG AGC CAC AGG GAC GAC CAT CTC 3630
Tyr Leu Leu Gly Gln Gln Tyr Leu Gln Ser His Arg Asp Asp His Leu
1070 1075 1080

GAC TTC TGG GCC GAG CAG ATC GGC AGG ATC GAA GAG CGC TGC GAC ATG 3678
Asp Phe Trp Ala Glu Gln Ile Gly Arg Ile Glu Glu Arg Cys Asp Met
1085 1090 1095

AAT GCG CTG CTG AAT GAG GCC AGC CGA TAC AAG GTG CCC CTG GCC GAC 3726
Asn Ala Leu Leu Asn Glu Ala Ser Arg Tyr Lys Val Pro Leu Ala Asp
1100 1105 1110

TAT GAC CAA GTC CGC GAG CAG AGG CAG CAG ACC ATC AGT CTG CCC TGG 3774
Tyr Asp Gln Val Arg Glu Gln Arg Gln Gln Thr Ile Ser Leu Pro Trp
1115 1120 1125

AAC AAC TCC ATG GAC GCT GGT GTG CGG GAA GAA CTC TCC AGT CGT GGC 3822
Asn Asn Ser Met Asp Ala Gly Val Arg Glu Glu Leu Ser Ser Arg Gly
1130 1135 1140 1145

ATC ACC CTT CAT TCC ATT CTA CAG ACG GTC TGG CAC CTG GTC CTC CAC 3870
Ile Thr Leu His Ser Ile Leu Gln Thr Val Trp His Leu Val Leu His
1150 1155 1160

TCT TAT GGA GGA GGC ACC CAC ACG ATC ACC GGC ACC ACC ATC TCC GGC 3918
Ser Tyr Gly Gly Gly Thr His Thr Ile Thr Gly Thr Thr Ile Ser Gly
1165 1170 1175

CGT CAC CTG CCC GTC CCC GGA ATT GAG CGC TCT GTT GGT CTC TTC ATC 3966
Arg His Leu Pro Val Pro Gly Ile Glu Arg Ser Val Gly Leu Phe Ile
1180 1185 1190

AAC ACA CTC CCT ATG ATC TTT GAT CAC ACC GTC TGC CAG GAT ATG ACA 4014
Asn Thr Leu Pro Met Ile Phe Asp His Thr Val Cys Gln Asp Met Thr
1195 1200 1205

GCG CTC GAG GCC ATT GAG CAT GTC CAA GGC CAA GTC AAC GCC ATG AAC 4062
Ala Leu Glu Ala Ile Glu His Val Gln Gly Gln Val Asn Ala Met Asn
1210 1215 1220 1225

TCC CGG GGC AAC GTC GAG CTC GGA CGC ATG AGC AAG AAC GAC CTC AAG 4110
Ser Arg Gly Asn Val Glu Leu Gly Arg Met Ser Lys Asn Asp Leu Lys
1230 1235 1240

CAC GGG CTC TTC GAC ACC CTC TTC GTC CTC GAG AAC TAC CCA AAC CTC 4158
His Gly Leu Phe Asp Thr Leu Phe Val Leu Glu Asn Tyr Pro Asn Leu
1245 1250 1255

GAC ACG GAG CAG CGG GAG AAG CAC GAG GAG AAG CTC AAG TTC ACC ATC 4206
Asp Thr Glu Gln Arg Glu Lys His Glu Glu Lys Leu Lys Phe Thr Ile
1260 1265 1270

AAG GGT GGC ACG GAG AAG CTC AGT TAC CCG CTG GCC GTG ATT GCC CAA 4254
Lys Gly GLy Thr Glu Lys Leu Ser Tyr Pro Leu Ala Val Ile Ala Gln
1275 1280 1285

GAG GAC GGC GAC AGC GGA TGC TCG TTT ACG CTC TGC TAT GCG GGC GAG 4302
Glu Asp Gly Asp Ser Gly Cys Ser Phe Thr Leu Cys Tyr Ala Gly Glu
1290 1295 1300 1305

CTC TTC ACG GAT GAG TCC ATC CAG GCG CTC CTG GAC ACT GTC CGG GAC 4350
Leu Phe Thr Asp Glu Ser Ile Gln Ala Leu Leu Asp Thr Val Arg Asp
1310 1315 1320

ACC CTG AGT GAT ATT CTC GGG AAC ATC CAT GCC CCT ATC CGC AAC ATG 4398
Thr Leu Ser Asp Ile Leu Gly Asn Ile His Ala Pro Ile Arg Asn Met
1325 1330 1335

GAG TAC CTC TCC TCG AAC CAG ACG GCG CAG CTC GAC AAG TGG AAT GCC 4446
Glu Tyr Leu Ser Ser Asn Gln Thr Ala Gln Leu Asp Lys Trp Asn Ala
1340 1345 1350

ACC GCC TTC GAG TAC CCC AAC ACC ACA CTG CAC GCC ATG TTC GAG TCC 4494
Thr Ala Phe Glu Tyr Pro Asn Thr Thr Leu His Ala Met Phe Glu Ser
1355 1360 1365

GAG GCG CAG CAG AAG CCG GAC AAG GTG GCC GTG GTG TAC GAG GAT ATC 4542
Glu Ala Gln Gln Lys Pro Asp Lys Val Ala Val Tyr Tyr Glu Asp Ile
1370 1375 1380 1385

AGG CTG ACC TAC CGC GAG CTC AAC AGC CGT GCC AAT GCC CTG GCG TTC 4590
Arg Leu Thr Tyr Arg Glu Leu Asn Ser Arg Ala Asn Ala Leu Ala Phe
1390 1395 1400

TAC CTC CTC TCC CAG GCG GCT ATC CAA CCG AAC AAG CTG GTC GGG CTG 4638
Tyr Leu Leu Ser Gln Ala ALa Ile Gln Pro Asn Lys Leu Val Gly Leu
1405 1410 1415

ATC ATG GAC AAG AGC GAG CAC ATG ATC ACG AGC ATC CTC GCG GTC TGG 4686
Ile Met Asp Lys Ser Glu His Met Ile Thr Ser Ile Leu Ala Val Trp
1420 1425 1430

AAA ACG GGT GGA GCC TAC GTC CCG ATC GAC CCT CGA TAC CCT GAC CAG 4734
Lys Thr Gly Gly Ala Tyr Val Pro Ile Asp Pro Arg Tyr Pro Asp Gln
1435 1440 1445

CGT ATC CAG TAT ATC CTG GAG GAT ACG GCG GCT CTC GCA GTC ATC ACG 4782
Arg Ile Gln Tyr Ile Leu Glu Asp Thr Ala Ala Leu Ala Val Ile Thr
1450 1455 1460 1465

GAC AGT CCT CAT ATT GAC CGT CTG CGC AGC ATC ACC AAC AAC CGC CTT 4830
Asp Ser Pro His Ile Asp Arg Leu Arg Ser Ile Thr Asn Asn Arg Leu
1470 1475 1480

CCT GTT ATC CAG TCG GAC TTT GCT CTC CAA CTC CCG CCC AGC CCA GTT 4878
Pro Val Ile Gln Ser Asp Phe Ala Leu Gln Leu Pro Pro Ser Pro Val
1485 1490 1495

CAT CCC GTC TCA AAC TGC AAG CCA AGC GAC CTC GCC TAC ATC ATG TAC 4926
His Pro Val Ser Asn Cys Lys Pro Ser Asp Leu Als Tyr Ile Met Tyr
1500 1505 1510

ACA TCC GGC ACC ACT GGC AAC CCC AAG GGT GTC ATG GTG GAG CAC CAC 4974
Thr Ser Gly Thr Thr Gly Asn Pro Lys Gly Val Met Val Glu His His
1515 1520 1525

GGT GTA GTG AAT CTG TGC GTT TCA CTC TGC CGG CTC TTC GGC CTT CGG 5022
Gly Val Val Asn Leu Cys Val Ser Leu Cys Arg Leu Phe Gly Leu Arg
1530 1535 1540 1545

AAC ACA GAT GAC GAG GTC ATC CTC TCG TTC TCG AAC TAC GTC TTC GAC 5070
Asn Thr Asp Asp Glu Val Ile Leu Ser Phe Ser Asn Tyr Val Phe Asp
1550 1555 1560

CAC TTT GTC GAG CAG ATG ACG GAT GCC CTT CTC AAC GGT CAG ACT CTT 5118
His Phe Val Glu Gln Met Thr Asp Ala Leu Leu Asn Gly Gln Thr Leu
1565 1570 1575

GTG GTC CTC AAC GAC GAG ATG CGT GGC GAC AAG GAG AGG CTT TAC AGA 5166
Val Val Leu Asn Asp Glu Met Arg Gly Asp Lys Glu Arg Leu Tyr Arg
1580 1585 1590

TAC ATC GAG ACC AAC CGC GTC ACG TAC CTC TCG GGG ACA CCT TCC GTC 5214
Tyr Ile Glu Thr Asn Arg Val Thr Tyr Leu Ser Gly Thr Pro Ser Val
1595 1600 1605

ATC TCC ATG TAC GAG TTC GAC CGG TTC CGC GAC CAC CTG CGG CGC GTG 5262
Ile Ser Met Tyr Glu Phe Asp Arg Phe Arg Asp His Leu Arg Arg Val
1610 1615 1620 1625

GAT TGC GTC GGC GAG GCC TTC AGC GAG CCG GTA TTC GAC AAG ATC CGC 5310
Asp Cys Val Gly Glu Ala Phe Ser Glu Pro Val Phe Asp Lys Ile Arg
1630 1635 1640

GAG ACG TTC CCG GGT CTC ATC ATC AAC GGT TAT GGC CCG ACT GAG GTG 5358
Glu Thr Phe Pro Gly Leu Ile Ile Asn Gly Tyr Gly Pro Thr Glu Val
1645 1650 1655

TCT ATC ACT ACC CAC AAG CGG CCC TAC CCG TTC CCG GAG CGC CGC ACA 5406
Ser Ile Thr Thr His Lys Arg Pro Tyr Pro Phe Pro Glu Arg Arg Thr
1660 1665 1670

GAC AAG AGC ATC GGT TGC CAG CTG GAC AAC AGC ACG AGC TAC GTC CTC 5454
Asp Lys Ser Ile Gly Cys Gln Leu Asp Asn Ser Thr Ser Tyr Val Leu
1675 1680 1685

AAC GAT GAC ATG AAG CGC GTG CCC ATC GGG GCC GTG GGA GAG CTG TAC 5502
Asn Asp Asp Met Lys Arg Val Pro Ile Gly Ala Val Gly Glu Leu Tyr
1690 1695 1700 1705

CTT GGT GGC GAT GGC GTC GCT CGC GGA TAC CAC AAC CGG CCA GAC CTG 5550
Leu Gly Gly Asp Gly Val Ala Arg Gly Tyr His Asn Arg Pro Asp Leu
1710 1715 1720

ACG GCT GAC CGG TTC CCT GCC AAC CCC TTC CAG ACG GAG CAG GAG AGA 5598
Thr Ala Asp Arg Phe Pro Ala Asn Pro Phe Gln Thr Glu Gln Glu Arg
1725 1730 1735

CTT GAG GGC CGA AAT GCG CGT CTG TAT AAG ACT GGT GAC TTG GTT CGC 5646
Leu Glu Gly Arg Asn Ala Arg Leu Tyr Lys Thr Gly Asp Leu Val Arg
1740 1745 1750

TGG ATC CAC AAT GCA AAC GGC GAT GGT GAG ATC GAG TAC CTC GGC CGC 5694
Trp Ile His Asn Ala Asn Gly Asp Gly Glu Ile Glu Tyr Leu Gly Arg
1755 1760 1765

AAC GAC TTC CAG GTC AAG ATT CGA GGC CAG AGA ATC GAG CTG GGA GAG 5742
Asn Asp Phe Gln Val Lys Ile Arg Gly Gln Arg Ile Glu Leu Gly Glu
1770 1775 1780 1785

ATC GAG GCC GTG CTT TCA TCC TAT CCG GGC ATC AAA CAA TCC GTC GTC 5790
Ile Glu Ala Val Leu Ser Ser Tyr Pro Gly Ile Lys Gln Ser Val Val
1790 1795 1800

CTG GCC AAG GAC CGC AAG AAT GAC GGG CAG AAG TAC CTC GTC GGC TAC 5838
Leu Ala Lys Asp Arg Lys Asn Asp Gly Gln Lys Tyr Leu Val Gly Tyr
1805 1810 1815

TTC GTC TCC TCA GCA GGG TCC CTG TCC GCC CAG GCC ATC CGC CGC TTC 5886
Phe Val Ser Ser Ala Gly Ser Leu Ser Ala Gln Ala Ile Arg Arg Phe
1820 1825 1830

ATG CTC ACG AGC CTG CCC GAT TAC ATG GTT CCT GCG CAG CTG GTG CCC 5934
Met Leu Thr Ser Leu Pro Asn Tyr Met Val Pro Ala Gln Leu Val Pro
1835 1840 1845

ATC GCC AAG TTC CCC GTC ACC GTG AGC GGG AAG CTC GAT GCC AAG GCC 5982
Ile Ala Lys Phe Pro Val Thr Val Ser Gly Lys Leu Asp Ala Lys Ala
1850 1855 1860 1865

TTG CCC GTG CCA GAC GAT ACA GTC GAG GAT GAC ATT GTG CCA CCG CGT 6030
Leu Pro Val Pro Asp Asp Thr Val Glu Asp Asp Ile Val Pro Pro Arg
1870 1875 1880

ACC GAG GTT GAG CGC ATC CTA GCT GGG ATC TGG TCT GAG CTG TTG GAG 6078
Thr Glu Val Glu Arg Ile Leu Ala Gly Ile Trp Ser Glu Leu Leu Glu
1885 1890 1895

ATA CCG GTC GAC AGG ATC AGC ATC TAC AGT GAC TTC TTC AGT CTG GGC 6126
Ile Pro Val Asp Arg Ile Ser Ile Tyr Ser Asp Phe Phe Ser Leu Gly
1900 1905 1910

GGC GAC AGT CTC AAG AGT ACC AAG CTG TCC TTT GCT GCC ACT CGG GCT 6174
Gly Asp Ser Leu Lys Ser Thr Lys Leu Ser Phe Ala Ala Thr Arg Ala
1915 1920 1925

CTC GGT GTG GCC GTC AGT GTC CGC AAC TTG TTC AGC CAT CCG ACT ATC 6222
Leu Gly Val Ala Val Ser Val Arg Asn Leu Phe Ser His Pro Thr Ile
1930 1935 1940 1945

GAA GCC TTG TCT CAG TGG ATT ATC AGG GGT TCG AAC GAG GTC AAG GAT 6270
Glu Ala Leu Ser Gln Trp Ile Ile Arg Gly Ser Asn Glu Val Lys Asp
1950 1955 1960

GTG GCT GTG GTG AAG GGC GGT GCC AGT CTT GAT ATC CCC CTA TCC CCT 6318
Val Ala Val Val Lys Gly Gly Ala Ser Leu Asp Phe Pro Leu Ser Pro
1965 1970 1975

GCC CAG GAA AGA CTC ATG TTC ATC CAC GAG TTC GGC CAT AGC GGC GAG 6366
Ala Gln Glu Arg Leu Met Phe Ile His Glu Phe Gly His Ser Gly Glu
1980 1985 1990

GAT ACT GGT GCT TAC AAT GTG CCT TTG CAG CTG CAG CTT CAC CAT GAT 6414
Asp Thr Gly Ala Tyr Asn Val Pro Leu Gln Leu Gln Leu His His Asp
1995 2000 2005

GTC TGT CTC GAG TCG CTT GAG AAG GCT CTG CGG GAT GTC GTC TCG AGA 6462
Val Cys Leu Glu Ser Leu Glu Lys Ala Leu Arg Asp Val Val Ser Arg
2010 2015 2020 2025

CAC GAG GCT CTC CGG ACC TTG ATC ACC AGG ACC CAG AAG TCC TCC GTG 6510
His Glu Ala Leu Arg Thr Leu Ile Thr Arg Thr Gln Lys Ser Ser Val
2030 2035 2040

CAC TGC CAG AAG ATC CTC GAC GCC GAA GAA GCG CAA AAG CTC TTC TCT 6558
His Cys Gln Lys Ile Leu Asp Ala Glu Glu Ala Gln Lys Leu Phe Ser
2045 2050 2055

GTT GAT GTT CTG CGC CTG ACC TCG GAG ACG GAG ATG CAG GGC AGG ATG 6606
Val Asp Val Leu Arg Leu Thr Ser Glu Thr Glu Met Gln Gly Arg Met
2060 2065 2070

GCC GAG AGT ACC GCC CAC GCC TTC AAG CTC GAC GAG GAA CTC CCG ATT 6654
Ala Glu Ser Thr Ala His Ala Phe Lys Leu Asp Glu Glu Leu Pro Ile
2075 2080 2085

CAT GTA CGC CTG TAC CAG GTT GTA CGT GAT GGC CGC ACG CTC AGC TTT 6702
His Val Arg Leu Tyr Gln Val Val Arg Asp Gly Arg Thr Leu Ser Phe
2090 2095 2100 2105

GCC AGC ATC GTC TGC CAC CAT CTG GCG TTT GAC GCG TGG TCA TGG GAT 6750
Ala Ser Ile Val Cys His His Leu Ala Phe Asp Ala Trp Ser Trp Asp
2110 2115 2120

GTG TTC CAG AGG GAC TTG GAC GCC TTC TAT GCC GTC CAT ACG AAG CAC 6798
Val Phe Gln Arg Asp Leu Asp Ala Phe Tyr Ala Val His Thr Lys His
2125 2130 2135

AAG GCT GCC GCC AAC CTG CCA ACC CTC CGC GTG CAA TAT AAG GAG TAT 6846
Lys Ala Ala Ala Asn Leu Pro Thr Leu Arg Val Gln Tyr Lys Glu Tyr
2140 2145 2150

GCG ATA GAG CAC CGC CGG GCT CTC CGC GCT GAG CAA CAC CGT GTT CTC 6894
Ala Ile Glu His Arg Arg Ala Leu Arg Ala Glu Gln His Arg Val Leu
2155 2160 2165

GCG GAC TAC TGG CTG CGC AAG CTC AGT GAC ATG GAG GCG TCT TAT CTG 6942
Ala Asp Tyr Trp Leu Arg Lys Leu Ser Asp Met Glu Ala Ser Tyr Leu
2170 2175 2180 2185

GTC CCC GAT CGC CCT CGA CCG GCG CAG TTT GAC TAT ACC GGG AAC GAT 6990
Val Pro Asp Arg Pro Arg Pro Ala Gln Phe Asp Tyr Thr Gly Asn Asp
2190 2195 2200

CTC CAG TTC TCA ACT ACT CCC GAG ACC ACC GCG CAG TTG AAG GAG CTG 7038
Leu Gln Phe Ser Thr Thr Pro Glu Thr Thr Ala Gln Leu Lys Glu Leu
2205 2210 2215

GCC AAG CGC GAG GGT TCA AGC CTC TAC ACC GTT GTG GCG GCG GCG TAC 7086
Ala Lys Arg Glu Gly Ser Ser Leu Tyr Thr Val Val Ala Ala Ala Tyr
2220 2225 2230

TTT CTG CTT CTC TAC GTG TAC ACC AAC CAG CGG GAT ATC ACG ATT GGT 7134
Phe Leu Leu Leu Tyr Val Tyr Thr Asn Gln Arg Asp Ile Thr Ile Gly
2235 2240 2245

ATT CCC GTT GCG CAC CGT AAC CAT CCG GAC TTT GAG TCG GTT GTC GGC 7182
Ile Pro Val Ala His Arg Asn His Pro Asp Phe Glu Ser Val Val Gly
2250 2255 2260 2265

TTC TTT GTC AAC TTG CTC CCT CTG CGG GTC AAC GTG TCT CAG TCG GAC 7230
Phe Phe Val Asn Leu Leu Pro Leu Arg Val Asn Val Ser Gln Ser Asp
2270 2275 2280

ATT CAT GGA CTT ATC CAG GCA GTG CAG AAA GAG CTT GTC GAT GCC CAG 7278
Ile His Gly Leu Ile Gln Ala Val Gln Lys Glu Leu Val Asp Ala Gln
2285 2290 2295

ATC CAT CAG GAC TTG CCA TTC CAG GAG ATC ACC AAG CTT CTT CAT GTG 7326
Ile His Gln Asp Leu Pro Phe Gln Glu Ile Thr Lys Leu Leu His Val
2300 2305 2310

CAG CAC GAT CCA AGC CGC CAT CCC CTT CTC CAG GCC GTG TTC AAC TGG 7374
Gln His Asp Pro Ser Arg His Pro Leu Leu Gln Ala Val Phe Asn Trp
2315 2320 2325

GAA AAC GTA CCC GCC AAT GTC CAC GAG GAG CAG CTG CTT CAG GAG TAC 7422
Glu Asn Val Pro Ala Asn Val His Glu Glu Gln Leu Leu Gln Glu Tyr
2330 2335 2340 2345

AAG CCG CCC TCG CCT CTG CCT TCG GCG GCC AAG TTT GAT CTC AAC GTC 7470
Lys Pro Pro Ser Pro Leu Pro Ser Ala Ala Lys Phe Asp Leu Asn Val
2350 2355 2360

ACG GTG AAA GAG AGC GTC AAT TCG CTC AAC GTC AAC TTC AAC TAT CCT 7518
Thr Val Lys Glu Ser Val Asn Ser Leu Asn Val Asn Phe Asn Tyr Pro
2365 2370 2375

ACC AGC CTC TTC GAG GAG GAG ACC GTT CAG GGG TTC ATG GAA ACC TTC 7566
Thr Ser Leu Phe Glu Glu Glu Thr Val Gln Gly Phe Met Glu Thr Phe
2380 2385 2390

CAT CTC CTT CTT CGA CAA CTG GCC CAC AAC AAG GCT AGC ACA AGC CTC 7614
His Leu Leu Leu Arg Gln Leu Ala His Asn Lys Ala Ser Thr Ser Leu
2395 2400 2405

TCG AAG CTG TCG GTT GAA GAT GGA GTG TTG AAT CCA GAG CCG ACT AAC 7662
Ser Lys Leu Ser Val Glu Asp Gly Val Leu Asn Pro Glu Pro Thr Asn
2410 2415 2420 2425

CTT CAG CCC TCA AGC CGG GAC AGC GGA AAT TCA CTC CAT GGG CTC TTC 7710
Leu Gln Pro Ser Ser Arg Asp Ser Gly Asn Ser Leu His Gly Leu Phe
2430 2435 2440

GAG GAC ATC GTG GCC TCG ACC CCG GAC CGC ATC GCA ATT GCT GAC GGC 7758
Glu Asp Ile Val Ala Ser Thr Pro Asp Arg Ile Ala Ile Ala Asp Gly
2445 2450 2455

ACC AGG AGT CTC TCG TAC TCC GAA CTC AAC GAG CGG GCA AAC CAG CTG 7806
Thr Arg Ser Leu Ser Tyr Ser Glu Leu Asn Glu Arg Ala Asn Gln Leu
2460 2465 2470

GTA CAT TTG ATC ATC TCT TCT GCC AGT ATT GTA GCA GAC GAC CGC ATC 7854
Val His Leu Ile Ile Ser Ser Ala Ser Ile Val Ala Asp Asp Arg Ile
2475 2480 2485

GCT CTT CTT TTG GAC AAG AGC ATC GAT ATG GTG ATT GCT CTC CTG GCA 7902
Ala Leu Leu Leu Asp Lys Ser Ile Asp Met Val Ile Ala Leu Leu Ala
2490 2495 2500 2505

GTT TGG AAG GCC GGT GCC GCA TAT GTG CCC CTT GAC CCG ACA TAT CCG 7950
Val Trp Lys Ala Gly Ala Ala Tyr Val Pro Leu Asp Pro Thr Tyr Pro
2510 2515 2520

TCG CAG AGG ACT GAG CTC ATC TTG GAG GAA TCT AGT GCC AGG ACG CTC 7998
Ser Gln Arg Thr Glu Leu Ile Leu Glu Glu Ser Ser Ala Arg Thr Leu
2525 2530 2535

ATC ACC ACT AGA AAG CAC ACG CCG AGG GGA GGA ACA GTC GCA AAT GTT 8046
Ile Thr Thr Arg Lys His Thr Pro Arg Gly Gly Thr Val Ala Asn Val
2540 2545 2550

CCA TCC GTG GTC CTT GAC AGC CCC GAG ACC CTA GCC TGC CTC AAC CAG 8094
Pro Ser Val Val Leu Asp Ser Pro Glu Thr Leu Ala Cys Leu Asn Gln
2555 2560 2565

CAG TCA AAG GAA AAC CCG ACA ACG TCA ACG CAG AAA CCG TCC GAC CTC 8142
Gln Ser Lys Glu Asn Pro Thr Thr Ser Thr Gln Lys Pro Ser Asp Leu
2570 2575 2580 2585

GCA TAT GTC ATC TTC ACC TCG GGA ACC ACA GGC AAG CCC AAG GGG GTT 8190
Ala Tyr Val Ile Phe Thr Ser Gly Thr Thr Gly Lys Pro Lys Gly Val
2590 2595 2600

CTG GTG GAG CAC CAG AGC GTA GTC CAG CTG CGC AAT TCC CTC ATC GAG 8238
Leu Val Glu His Gln Ser Val Val Gln Leu Arg Asn Ser Leu Ile Glu
2605 2610 2615

CGA TAC TTC GGC GAG ACC AAC GGG TCT CAC GCC GTG CTC TTC CTG TCC 8286
Arg Tyr Phe Gly Glu Thr Asn Gly Ser His Ala Val Leu Phe Leu Ser
2620 2625 2630

AAC TAC GTC TTC GAC TTC TCT CTT GAA CAG CTC TGT CTC TCA GTC TTG 8334
Asn Tyr Val Phe Asp Phe Ser Leu Glu Gln Leu Cys Leu Ser Val Leu
2635 2640 2645

GGT GGA AAC AAG CTC ATC ATT CCA CCA GAG GAG GGT CTC ACG CAC GAG 8382
Gly Gly Asn Lys Leu Ile Ile Pro Pro Glu Glu Gly Leu Thr His Glu
2650 2655 2660 2665

GCA TTC TAC GAC ATC GGC CGC AGG GAG AAG CTA TCC TAT CTC AGC GGG 8430
Ala Phe Tyr Asp Ile Gly Arg Arg Glu Lys Leu Ser Tyr Leu Ser Gly
2670 2675 2680

ACG CCC TCG GTG CTG CAG CAG ATT GAG CTC TCC CGT CTG CCG CAT CTT 8478
Thr Pro Ser Val Leu Gln Gln Ile Glu Leu Ser Arg Leu Pro His Leu
2685 2690 2695

CAC ATG GTC ACC GCT GCG GGC GAG GAG TTC CAC GCT AGT CAG TTT GAG 8526
His Met Val Thr Ala Ala Gly Glu Glu Phe His Ala Ser Gln Phe Glu
2700 2705 2710

AAG ATG CGC TCC CAG TTC GCG GGC CAG ATC AAC AAC GCC TAT GGT ATC 8574
Lys Met Arg Ser Gln Phe Ala Gly Gln Ile Asn Asn Ala Tyr Gly Ile
2715 2720 2725

ACT GAG ACG ACC GTG TAC AAC ATC ATC ACC ACG TTC AAG GGC GAT GCC 8622
Thr Glu Thr Thr Val Tyr Asn Ile Ile Thr Thr Phe Lys Gly Asp Ala
2730 2735 2740 2745

CCC TTT ACC AAG GCA CTC TGC CAC GGG ATC CCC GGA AGT CAC GTC TAC 8670
Pro Phe Tyr Lys Ala Leu Cys His Gly Ile Pro Gly Ser His Val Tyr
2750 2755 2760

GTC CTG AAC GAC CGA CTT CAG CGT GTT CCT TTC AAC GCT GTT GGC GAG 8718
Val Leu Asn Asp Arg Leu Gln Arg Val Pro Phe Asn Ala Val Gly Glu
2765 2770 2775

CTC TAC TTG GGC GGT GAC TGC CTT GCT CGC GGG TAC CTC AAC CAG GAT 8766
Leu Tyr Leu Gly Gly Asp Cys Leu Ala Arg Gly Tyr Leu Asn Gln Asp
2780 2785 2790

GCC CTG ACC AAC GAG CGA TTC ATC CCC AAC CCT TTC TAC GAG CCG AAA 8814
Ala Leu Thr Asn Glu Arg Phe Ile Pro Asn Pro Phe Tyr Glu Pro Lys
2795 2800 2805

CAG GCA AGT GAC AGT CGT CCC CAG AGA CTC TAC AAG ACT GGA GAT CTG 8862
Gln Ala Ser Asp Ser Arg Pro Gln Arg Leu Tyr Lys Thr Gly Asp Leu
2810 2815 2820 2825

GTG CGC TTC CGT GGA CCC CAC CAT CTC GAG TAT CTC GGC CGC AAG GAC 8910
Val Arg Phe Arg Gly Pro His His Leu Glu Tyr Leu Gly Arg Lys Asp
2830 2835 2840

CAG CAG GTC AAG CTG AGG GGC TTC CGC ATC GAG CTC TCC GAG GTG CGG 8958
Gln Gln Val Lys Leu Arg Gly Phe Arg Ile Glu Leu Ser Glu Val Arg
2845 2850 2855

GAT GCC GTC CTA GCC ATC TCT GCT GTT AAG GAG GCT GCC GTC ATC CCC 9006
Asp Ala Val Leu Ala Ile Ser Ala Val Lys Glu Ala Ala Val Ile Pro
2860 2865 2870

AAG TAT GAC GAG GAT GGC TCC GAT TCA CGA AGG GTC AGC GCC ATC GTC 9054
Lys Tyr Asp Glu Asp Gly Ser Asp Ser Arg Arg Val Ser Ala Ile Val
2875 2880 2885

TGC TAC TAC ACG CTC AAC GCC GGA ACT GTG TGC GAA GCA TCG AGT ATC 9102
Cys Tyr Tyr Thr Leu Asn Ala Gly Thr Val Cys Glu Ala Ser Ser Ile
2890 2895 2900 2905

CGT GAC CAC CTG CAC GCC AAC CTT CCC CCG TAC ATG GTC CCA AGT CAG 9150
Arg Asp His Leu His Ala Asn Leu Pro Pro Tyr Met Val Pro Ser Gln
2910 2915 2920

ATC CAC CAG TTG GAG GGA TCT CTC CCC GTG ACC GTC AAT GGG AAG CTC 9198
Ile His Gln Leu Glu Gly Ser Leu Pro Val Thr Val Asn Gly Lys Leu
2925 2930 2935

GAC CTG AAC AGG CTC TCC ACA ACT CAA GTC TCG CAG CCA GAG CTT TAC 9246
Asp Leu Asn Arg Leu Ser Thr Thr Gln Val Ser Gln Pro Glu Leu Tyr
2940 2945 2950

ACC GCT CCA CGA AAT TCG ACA GAG GAA ACC TTG TGC CAG CTT TGG GCA 9294
Thr Ala Pro Arg Asn Ser Thr Glu Glu Thr Leu Cys Gln Leu Trp Ala
2955 2960 2965

TCT CTC CTA GGC GTC GAC CAC TGC GGC ATT GAC GAC GAC CTG TTT GCC 9342
Ser Leu Leu Gly Val Asp His Cys Gly Ile Asp Asp Asp Leu Phe Ala
2970 2975 2980 2985

CGA GGC GGC GAC AGC ATC TCC TCT CTC CGA CTA GTG GGT GAC ATC TAC 9390
Arg Gly Gly Asp Ser Ile Ser Ser Leu Arg Leu Val Gly Asp Ile Tyr
2990 2995 3000

CGC GCG CTA GGA CGC AAG GTC ACC GTC AAG GAC ATC TAC CTC CAC CGC 9438
Arg Ala Leu Gly Arg Lys Val Thr Val Lys Asp Ile Tyr Leu His Arg
3005 3010 3015

AGC GTC CGA GCC CTA AGC GAA AAT GTC CTG ACC GAC CAG AAG GAT AAG 9486
Ser Val Arg Ala Leu Ser Glu Asn Val Leu Thr Asp Gln Lys Asp Lys
3020 3025 3030

GGT ACT CTG CCA GCG TCT CCT CCC CTC CAG CGA GCG GAG CAG GGC CAG 9534
Gly Thr Leu Pro Ala Ser Pro Pro Leu Gln Arg Ala Glu Gln Gly Gln
3035 3040 3045

GTT GAG GGC GAC GCA CCG CTT CTC CCC ATC CAG GAC TGG TTC CTT TCC 9582
Val Glu Gly Asp Ala Pro Leu Leu Pro Ile Gln Asp Trp Phe Leu Ser
3050 3055 3060 3065

AAG CCC CTG GAT AAC CCC GCT TAC TGG AAC CAC TGC TTC ACC ATT CGA 9630
Lys Pro Leu Asp Asn Pro Ala Tyr Trp Asn His Cys Phe Thr Ile Arg
3070 3075 3080

ACC GGG GCA CTC TCC GTC GAA GGG CTC CGG GGT GCT CTG AAG CTG CTG 9678
Thr Gly Ala Leu Ser Val Glu Gly Leu Arg Gly Ala Leu Lys Leu Leu
3085 3090 3095

CAG GAG CGC CAC GAC GTG CTG CGT CTG AGA CTG CAA CGC CGG GAC GAA 9726
Gln Glu Arg His Asp Val Leu Arg Leu Arg Leu Gln Arg Arg Asp Glu
3100 3105 3110

GGT CGC CAT GTT CAG ACC TTT GCG CGT GAC TGC GCG CAA CCT CGC TTG 9774
Gly Arg His Val Gln Thr Phe Ala Arg Asp Cys Ala Gln Pro Arg Leu
3115 3120 3125

ACT GTG CTA GAC CGA CGA AGC TTC GAG GAC GCA GAG GAT GTA CAG GAG 9822
Thr Val Leu Asp Arg Arg Ser Phe Glu Asp Ala Glu Asp Val Gln Glu
3130 3135 3140 3145

GCT CTC TGC GAG ATC CAA TCT CAT TTC GAC CTC GAG AAT GGA CCC CTC 9870
Ala Leu Cys Glu Ile Gln Ser His Phe Asp Leu Glu Asn Gly Pro Leu
3150 3155 3160

TAC ACA GTG GCG TAC ATC CAC GGT TAC GAG GAC GGC TCC GCC CGA GTG 9918
Tyr Thr Val Ala Tyr Ile His Gly Tyr Glu Asp Gly Ser Ala Arg Val
3165 3170 3175

TGG TTT GCC TGC CAT CAC GTC ATG GTC GAC ACT GTG AGC TGG AAC ATT 9966
Trp Phe Ala Cys His His Val Met Val Asp Thr Val Ser Trp Asn Ile
3180 3185 3190

ATA CTG CAA GAC CTG CAG GCT CTC TAT CAT GGA GAC AGC CTT GGT CCC 10014
Ile Leu Gln Asp Leu Gln Ala Leu Tyr His Gly Asp Ser Leu Gly Pro
3195 3200 3205

AAG AGC AGC AGC GTG CAG CAG TGG TCG CTA GCT GTC AGC GAC TAC AAA 10062
Lys Ser Ser Ser Val Gln Gln Trp Ser Leu Ala Val Ser Asp Tyr Lys
3210 3215 3220 3225

ATG CCA CTG TCG GAG AGG GCG CAT TGG AAT GTG CTC AGG AAG ACA GTC 10110
Met Pro Leu Ser Glu Arg Ala His Trp Asn Val Leu Arg Lys Thr Val
3230 3235 2340

GCC CAG AGC TTC GAG ACC CTG CCT ATC TGC ATG GGC GGC GTG CTC CAG 10158
Ala Gln Ser Phe Glu Thr Leu Pro Ile Cys Met Gly Gly Val Leu Gln
3245 3250 3255

TGC CAG GAG AAG TTC TCG AGG GAA ACG ACA ACA GCT CTG CTC TCC AAG 10206
Cys Gln Glu Lys Phe Ser Arg Glu Thr Thr Thr Ala Leu Leu Ser Lys
3260 3265 3270

GCC TGC CCT GCC TTG GAC TCC GGT ATG CAT GAG ATC CTT CTC ATG GCC 10254
Ala Cys Pro Ala Leu Asp Ser Gly Met His Glu Ile Leu Leu Met Ala
3275 3280 3285

GTG GGC TCC GCG CTG CAG AAG GCG GCA GGG GAT GTC CCT CAG GTC GTC     10302
Val Gly Ser Ala Leu Gln Lys Ala Ala Gly Asp Val Pro Gln Val Val
3290                3295                3300                3305

ACG ATA GAG GGT CAC GGG CGC GAA GAT ACT ATC GAC GCA ACT CTG GAC     10350
Thr Ile Glu Gly His Gly Arg Glu Asp Thr Ile Asp Ala Thr Leu Asp
                3310                3315                3320

GTC AGC CGG ACA GTC GGC TGG TTC ACG AGC ATG TAC CCC TTC GAG ATC     10398
Val Ser Arg Thr Val Gly Trp Phe Thr Ser Met Tyr Pro Phe Glu Ile
            3325                3330                3335

CCC AAA GTG ACC GAC CCC GCT CAG GGC GTC GTC GAT GTC AAG GAG GCG     10446
Pro Lys Val Thr Asp Pro Ala Gln Gly Val Val Asp Val Lys Glu Ala
        3340                3345                3350

ATG CGT CGC GTG CCG AAT AGG GGT GTC GGT TAC GGT CCA GCC TAC GGA     10494
Met Arg Arg Val Pro Asn Arg Gly Val Gly Tyr Gly Pro Ala Tyr Gly
    3355                3360                3365

TAC GGC GGA TCG TGC CTG CCC GCG GTG AGC TTC AAC TAC CTT GGT CGC     10542
Tyr Gly Gly Ser Cys Leu Pro Ala Val Ser Phe Asn Tyr Leu Gly Arg
3370                3375                3380                3385

CTG GAC CAG GCT TCC TCG GGG GCT CAA AGG GAC TGG ACG CTG GTC ATG     10590
Leu Asp Gln Ala Ser Ser Gly Ala Gln Arg Asp Trp Thr Leu Val Met
                3390                3395                3400

GAT GAA GAC GAG TAT CCG GTC GGA CTG TGC ACC AGC GCT GAG GAC TCG     10638
Asp Glu Asp Glu Tyr Pro Val Gly Leu Cys Thr Ser Ala Glu Asp Ser
            3405                3410                3415

GGA CGA AGC TCC TCC ATG GTG GAT TTC ACC TTC TCT ATC TCT GGC GGC     10686
Gly Arg Ser Ser Ser Met Val Asp Phe Thr Phe Ser Ile Ser Gly Gly
        3420                2325                3430

CAG CTT GTC ATG GAT ATG AGT AGC AGC TGG GGC CAC GGC GCA CGA AAT     10734
Gln Leu Val Met Asp Met Ser Ser Ser Trp Gly His Gly Ala Arg Asn
    3435                3440                3445

GAA TTC GTT CGC ACA GTT CGT AAC ACA CTA GAT GAC TTG ATC AAA ACA     10782
Glu Phe Val Arg Thr Val Arg Asn Thr Leu Asp Asp Leu Ile Lys Thr
3450                3455                3460                3465

ACG AGC AGC AGG GAC TTC AGC GCA CCT CTG CCT CCG TCG GAT CAG GAG     10830
Thr Ser Ser Arg Asp Phe Ser Ala Pro Leu Pro Pro Ser Asp Gln Glu
                3470                3475                3480

TCC AGC TTC ACC CCT TAT TTT GTC TTC GAA GAG GGC GAG CGA CAC GGC     10878
Ser Ser Phe Thr Pro Tyr Phe Val Phe Glu Glu Gly Glu Arg His Gly
            3485                3490                3495

GCT CCG CTC TTC CTG CTC CCA CCT GGC GAA GGC GGA GCG GAG AGC TAC     10926
Ala Pro Leu Phe Leu Leu Pro Pro Gly Glu Gly Gly Ala Glu Ser Tyr
        3500                3505                3510

TTC CAC AAC ATT GTC AAG GGT CTC CCG AAC CGC AAT CTT GTC GTG TTC 10974
Phe His Asn Ile Val Lys Gly Leu Pro Asn Arg Asn Leu Val Val Phe
3515 3520 3525

AAC AAT CAT TAC CGC GAG GAG AAG ACG CTC CGG ACC ATC GAG GCG CTG 11022
Asn Asn His Tyr Arg Glu Glu Lys Thr Leu Arg Thr Ile Glu Ala Leu
3530 3535 3540 3545

GCC GAG TAC TAC CTG TCG CAC ATC CGA TCC ATC CAG CCG GAG GGG CCA 11070
Ala Glu Tyr Tyr Leu Ser His Ile Arg Ser Ile Gln Pro Glu Gly Pro
3550 3555 3560

TAC CAC ATC CTC GGC TGG AGT TTC GGA GGC ATC CTC GGT CTC GAG GCG 11118
Tyr His Ile Leu Gly Trp Ser Phe Gly Gly Ile Leu Gly Leu Glu Ala
3565 3570 3575

GCA AAG CGA TTG ACT GGC GAG GGT CAC AAG ATT GCC ACG CTG GCA CTT 11166
Ala Lys Arg Leu Thr Gly Glu Gly His Lys Ile Ala Thr Leu Ala Leu
3580 3585 3590

ATC GAT CCG TAC TTT GAC ATC CCG TCC GCG TCC AAG GCC ATC GGC CAA 11214
Ile Asp Pro Tyr Phe Asp Ile Pro Ser Ala Ser Lys Ala Ile Gly Gln
3595 3600 3605

CCT GAC GAT GCC TGC GTC TTG GAC CCC ATA TAC CAC GTC TAC CAC CCG 11262
Pro Asp Asp Ala Cys Val Leu Asp Pro Ile Tyr His Val Tyr His Pro
3610 3615 3620 3625

TCG CCG GAG AGC TTC AGG ACG GTG TCA TCT CTC ACT AAT CAC ATA GCC 11310
Ser Pro Glu Ser Phe Arg Thr Val Ser Ser Leu Thr Asn His Ile Ala
3630 3635 3640

CTG TTC AAG GCT ACC GAG ACG AAT GAC CAG CAT GGC AAT GCC ACG CAG 11358
Leu Phe Lys Ala Thr Glu Thr Asn Asp Gln His Gly Asn Ala Thr Gln
3645 3650 3655

CAG GCC CTG TAT GAG TGG TTT GCC ACG TGC CCT TTG AAC AAC CTG GAC 11406
Gln Ala Leu Tyr Glu Trp Phe Ala Thr Cys Pro Leu Asn Asn Leu Asp
3660 3665 3670

AAG TTT TTG GCG GCC GAC ACG ATC AAG GTG GTT CCT CTG GAG GGT ACA 11454
Lys Phe Leu Ala Ala Asp Thr Ile Lys Val Val Pro Leu Glu Gly Thr
3675 3680 3685

CAT TTT ACC TGG GTG CAC CAC CCG GAG CAG GTG CGC TCA ATG TGC ACT 11502
His Phe Thr Trp Val His His Pro Glu Gln Val Arg Ser Met Cys Thr
3690 3695 3700 3705

ATG CTG GAT GAA TGG CTT GGG TGA 11526
Met Leu Asp Glu Trp Leu Gly End
3710

ACGAGGCAGT TGCTGTGAGA GAATGAGAAT GAGACACAAA ACGCGGGCGG AAGAGAGACT 11586
TCCTCGGACG GCGGG 11601

## Claims

1. A DNA fragment of fewer than about 15 kbp comprising:
an open reading frame encoding δ-(L-α-aminoadipyl)-L-cysteinyl-D-valine synthetase, wherein the sequence of said open reading frame is included in the complete sequence shown in sequence listing 1 or in sequence listing 2 or wherein the sequence of said open reading frame includes conservative and/or non-conservative mutations of the sequence shown in sequence listing 1 or in sequence listing 2, said mutations producing a biologically active expression product.

2. An expression cassette comprising:
as components, in the direction of transcription, a promoter functional in a host cell, an open reading frame encoding δ-(L-α-aminoadipyl)-L-cysteinyl-D-valine synthetase, as defined in claim 1, and a terminator functional in a host cell, wherein expression of said synthetase is under regulatory control of said promoter and terminator.

3. The expression cassette of claim 2, wherein said promoter is functional in a filamentous fungus, and is preferably a Penicillium chrysogenum pgk promoter.

4. The expression cassette of claim 2, wherein said promoter is active in the presence of glucose.

5. The expression cassette of claim 2, wherein said promoter is functional in a prokaryotic cell, and is preferably an E.coli trp promoter, a Streptomyces aph promoter or a tyrosinase promoter.

6. Plasmid pPCV02, having a restriction site and functional map of Figure 7;
plasmid pPCV03, having a restriction site and functional map of Figure 8;
plasmid pMA-ACVS, having a restriction site and functional map of Figure 10;
plasmid pSLACV-01, having a restriction site and functional map of Figure 11;
plasmid pSLACV-03A, having a restriction site and functional map of Figure 12A, or
plasmid pSLACV-03B, having a restriction site and functional map of Figure 12B.

7. A transformed host cell comprising:
an expression cassette as defined in any one of the claims 2-5.

8. The cell of claim 7, wherein said cell is a eukaryotic cell, preferably a filamentous fungus, and more preferably Penicillium chrysogenum, Acremonium chrysogenum or Asperqillus nidulans.

9. A transformed Penicillium chrysogenum, Acremonium chrysogenum or Asperqillus nidulans host cell comprising plasmid pPCV02 or pPCV03, as defined in claim 6.

10. A transformed E.coli cell comprising plasmid pMA-ACVS, as defined in claim 6.

11. A transformed Streptomyces cell comprising plasmid pSL-ACV01, pSL-ACV03A or pSL-ACV03B, as defined in claim 6.

12. Use of a transformed host cell as defined in claims 7-9 in an industrial β-lactam production process.

13. A method for obtaining or enhancing ACVS production in a microbial host cell, said method comprising:
growing the host cell of claims 7-9 under conditions whereby said ACVS is produced and optionally isolated.

14. A method for enhancing production of β-lactam antibiotics in a microbial host cell, said method comprising:
transforming a host cell capable of producing β-lactam antibiotics with the expression cassette of claim 2-5 whereby transformed cells are obtained;
cloning said transformed cells by means of a selection marker on said expression cassette;
identifying transformed cells producing increased amounts of β-lactam antibiotics as compared to untransformed cells (hereinafter "enhanced expressers") and isolating said enhanced expressers;
growing said enhanced expressers whereby enhanced production of β-lactam antibiotics in a microbial host is obtained;
and optionally isolating said β-lactam antibiotics.

15. The method according to claim 14, wherein said host cell is a filamentous fungus, and preferably a Penicillium chrysogenum, Acremonium chrysogenum or Aspergillus nidulans cell, or a Streptomyces cell.

## Patentansprüche

1. DNA-Fragment mit weniger als etwa 15 kbp, umfassend: einen offenen Leseraster, der für δ-(L-α-Aminoadipyl)-L-cysteinyl-D-valin-synthetase kodiert, wobei die Sequenz des offenen Leserasters in der in der Sequenzliste 1 oder in der Sequenzliste 2 dargestellten vollständigen Sequenz enthalten ist oder wobei die Sequenz des offenen Leserasters konservative und/oder nicht-konservative Mutationen der in der Sequenzliste 1 oder in der Sequenzliste 2 dargestellten Sequenz umfaßt, wobei die Mutationen ein biologisch aktives Expressionsprodukt erzeugen.

2. Expressionskassette, umfassend:
als Bestandteile in Transkriptionsrichtung einen in einer Wirtszelle funktionellen Promotor, einen für δ-(L-α-Aminoadipyl) -L-cysteinyl-D-valin-synthetase kodierenden offenen Leseraster gemäß der Definition in Anspruch 1 und einen in einer Wirtszelle funktionellen Terminator, wobei die Expression der Synthetase unter regulatorischer Kontrolle des Promotors und des Terminators steht.

3. Expressionskassette nach Anspruch 2, wobei der Promotor in einem filamentösen Pilz funktionell ist und es sich vorzugsweise um einen *Penicillium chrysogenum-pgk*-Promotor handelt.

4. Expressionskassette nach Anspruch 2, wobei der Promotor in Gegenwart von Glucose aktiv ist.

5. Expressionskassette nach Anspruch 2, wobei der Promotor in einer prokaryontischen Zelle funktionell ist und es sich vorzugsweise um einen *E. coli-trp*-Promotor, einen *Streptomyces-aph-*Promotor oder einen Tyrosinase-Promotor handelt.

6. Plasmid pPCV02 mit einer Restriktionsstelle und der funktionellen Karte von Fig. 7;
Plasmid pPCV03 mit einer Restriktionsstelle und der funktionellen Karte von Fig. 8;
Plasmid pMA-ACVS mit einer Restriktionsstelle und der funktionellen Karte von Fig. 10;
Plasmid pSLACV-01 mit einer Restriktionsstelle und der funktionellen Karte von Fig. 11;
Plasmid pSLACV-03A mit einer Restriktionsstelle und der funktionellen Karte von Fig. 12A; oder
Plasmid pSLACV-03B mit einer Restriktionsstelle und der funktionellen Karte von Fig. 12B.

7. Transformierte Wirtszelle, umfassend:
eine Expressionskassette gemäß der Definition in einem der Ansprüche 2 bis 5.

8. Zelle nach Anspruch 7, wobei es sich bei der Zelle um eine eukaryontische Zelle handelt, vorzugsweise um einen filamentösen Pilz und insbesondere um *Penicillium chrysogenum, Acremonium chrysogenum* oder *Aspergillus nidulans.*

9. Transformierte *Penicillium chrysogenum-, Acremonium chrysogenum-* oder *Aspergillus nidulans-*Wirtszelle, enthaltend das Plasmid pPCV02 oder pPCV03 gemäß der Definition in Anspruch 6.

10. Transformierte E. coli-Zelle enthaltend das Plasmid pMA-ACVS gemäß der Definition in Anspruch 6.

11. Transformierte Streptomyces-Zelle, enthaltend das Plasmid pSL-ACV01, pSL-ACV03A oder pSL-ACV03B gemäß der Definition in Anspruch 6.

12. Verwendung einer transformierten Wirtszelle gemäß der Definition in den Ansprüchen 7 bis 9 in einem großtechnischen β-Lactam-Antibiotika-Herstellungsverfahren.

13. Verfahren zur Bildung von ACVS oder zur Verstärkung der ACVS-Bildung in einer mikrobiellen Wirtszelle, wobei das Verfahren folgendes umfaßt:
das Züchten der Wirtszelle nach den Ansprüchen 7 bis 9 unter Bedingungen, bei denen ACVS gebildet und ggf.isoliert wird.

14. Verfahren zur Verstärkung der Bildung von β-Lactam-Antibiotika in einer mikrobiellen Wirtszelle, wobei das Verfahren folgendes umfaßt:
das Transformieren einer Wirtszelle, die zur Bildung von β-Lactam-Antibiotika befähigt ist, mit einer Expressionskassette nach den Ansprüchen 2 bis 5, wobei transformierte Zellen erhalten werden;
das Klonen der transformierten Zellen mittels eines Selektionsmarkers an der Expressionskassette;
das Identifizieren von transformierten Zellen, die im Vergleich zu untransformierten Zellen erhöhte Mengen an β-Lactam-Antibiotika bilden (nachstehend kurz "verstärkte Expressoren") und das Isolieren der verstärkten Expressoren;
das Züchten der verstärkten Expressoren, wobei eine verstärkte Bildung von β-Lactam-Antibiotika in einem mikrobiellen Wirt erreicht wird;
und ggf. das Isolieren der β-Lactam-Antibiotika.

15. Verfahren nach Anspruch 14, wobei es sich bei der Wirtszelle um einen filamentösen Pilz und vorzugsweise um eine *Penicillium chrysogenum-, Acremonium chrysogenum-* oder *Aspergillus nidulans*-Zelle; oder um eine *Streptomyces-*Zelle handelt.

## Revendications

1. Fragment d'ADN ayant moins d'environ 15 kpdb, comprenant :
un cadre de l'ecture ouvert codant la δ-(L-α-aminoadipyl)-L-cystéinyl-D-valine synthétase, où la séquence dudit cadre de lecture ouvert est incluse dans la séquence complète représentée dans la liste de séquences 1 ou dans la liste de séquences 2 ou où la séquence dudit cadre de lecture ouvert comporte des mutations conservatives ou non conservatives de la séquence représentée dans la liste de séquences 1 ou dans la liste de séquences 2, lesdites mutations produisant un produit d'expression biologiquement actif.

2. Cassette d'expression comprenant :
comme composants, dans le sens de la transcription, un promoteur fonctionnel dans une cellule hôte, un cadre de lecture ouvert codant la δ-(L-α-aminoadipyl)-L-cystéinyl-D-valine synthétase selon la revendication 1 et un terminateur fonctionnel dans une cellule hôte, où l'expression de ladite ynthétase est sous le contrôle régulateur dudit promoteur et dudit terminateur.

3. Cassette d'expression selon la revendication 2, où ledit promoteur est fonctionnel dans un champignon filamenteux et est de préférence un promoteur pgk de Penicillium chrysogenum.

4. Cassette d'expression selon la revendication 2, où ledit promoteur est actif en présence de glucose.

5. Cassette d'expression selon la revendication 2, où ledit promoteur est fonctionnel dans une cellule procaryote et est de préférence un promoteur trp de E. coli, un promoteur aph de Streptomyces ou un promoteur tyrosinase.

6. Plasmide pPCV02, ayant un site de restriction et une carte fonctionnelle selon la figure 7 :
plasmide pPCV03, ayant un site de restriction et une carte fonctionnelle selon la figure 8 :
plasmide pMA-ACVS. ayant un site de restriction et une carte fonctionnelle selon la figure 10 :
plasmide pSLACV-01, ayant un site de restriction et une carte fonctionnelle selon la figure 11;
plasmide pSLACV-03A, ayant un site de restriction et une carte fonctionnelle selon la figure 12A, ou
plasmide pSLACV-03B, ayant un site de restriction et une carte fonctionnelle selon la figure 12B.

7. Cellule hôte transformée, comprenant :
une cassette d'expression selon l'une quelconque des revendication 2 à 5.

8. Cellule selon la revendication 7, où ladite cellule est une cellule eucaryote, de préférence un champignon filamenteux, et de façon particulièrement préférée Penicillium chrysogenum, Acremonium chrysogenum ou Aspergillus nidulans.

9. Cellule hôte de Penicillium chrysogenum, Acremonium chrysogenum ou Aspergillus nidulans transformée, comprenant le plasmide pPCV02 ou pPCV03 selon la revendication 6.

10. Cellule de E. coli transformée, comprenant le plasmide pMA-ACVS selon la revendication 6.

11. Cellule de Streptomyces transformée, comprenant le plasmide pSL-ACV01, pSL-ACV03A ou pSL-ACV03B selon la revendication 6.

12. Utilisation d'une cellule hôte transformée selon les revendications 7 à 9 dans un procédé de production industrielle de β-lactames.

13. Procédé d'obtention ou d'amplification de la production de ACVS dans une cellule hôte microbienne, ledit procédé comprenant :
la culture de la cellule hôte selon les revendications 7 à 9 dans des conditions où ladite ACVS est produite et facultativement isolée.

14. Procédé de stimulation de la production d'antibiotiques β-lactamiques dans une cellule hôte microbienne, ledit procédé comprenant :
la transformation d'une cellule hôte capable de produire des antibiotiques β-lactamiques au moyen de la cassette d'expression selon les revendications 2 à 5, des cellules transformées étant ainsi obtenues ;
le clonage desdites cellules transformées au moyen d'un marqueur de sélection sur ladite cassette d'expression ;
l'identification des cellules transfonnées produisant des quantités accrues d'antibiotiques β-lactamiques par comparaison à des cellules non transformées (appelées ci-après "exprimeurs stimulés") et l'isolement desdits exprimeurs stimulés :
la culture desdits exprimeurs stimulés. une production stimulée d'antibiotiques β-lactamiques dans un hôte microbien étant ainsi obtenue ;
et l'isolement facultatif desdits antibiotiques β-lactamiques.

15. Procédé selon la revendication 14, où ladite cellule hôte est un champignon filamenteux et de préférence une cellule de Penicillium chrysogenum, Acremonium chrysogenum ou Aspergillus nidulans ou une cellule de Streptomyces.
